(19) Europäisches Patentamt
European Patent Office
Office européen des brevets

(11) EP 2 105 443 A1

(12) **EUROPEAN PATENT APPLICATION**

(43) Date of publication:
**30.09.2009 Bulletin 2009/40**

(21) Application number: **09154902.2**

(22) Date of filing: **11.03.2009**

(51) Int Cl.:
***C07D 471/16*** *(2006.01)* ***C07D 215/42*** *(2006.01)*
***G03F 7/031*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **24.03.2008 JP 2008076553**

(71) Applicant: **FUJIFILM Corporation**
**Minato-ku**
**Tokyo (JP)**

(72) Inventors:
- **Tsuchimura, Tomotaka**
  **Haibara-gun Shizuoka (JP)**
- **Makino, Masaomi**
  **Haibara-gun Shizuoka (JP)**

(74) Representative: **Brookes Batchellor LLP**
**102-108 Clerkenwell Road**
**London**
**EC1M 5SA (GB)**

(54) **Oxime derivatives and their use in photopolymerizable compositions for colour filters**

(57) The present invention provides a compound represented by the following Formula (1):

Formula (1)

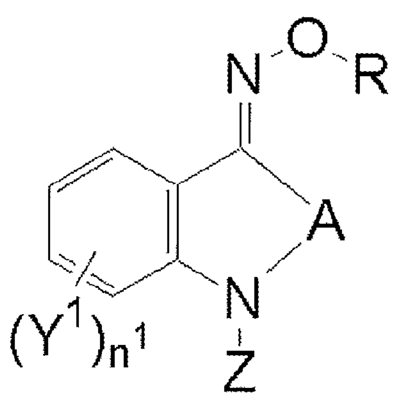

wherein R, $Y^1$ and Z each independently represent a monovalent substituent, $n^1$ represents an integer of 0 to 4, $Y^1$ and Z may be bound to each other to form a ring, and A represents a divalent linking group.

EP 2 105 443 A1

## Description

BACKGROUND

Field of the Invention

The present invention relates to novel compounds, photopolymerizable compositions, photopolymerizable compositions for color filter, color filters, and method for production thereof, solid-state imaging devices and planographic printing plate precursors.

Related Art

Photopolymerizable compositions may include, for example, an ethylenic unsaturated bond-containing polymerizable compound and a photopolymerization initiator. Such photopolymerizable compositions polymerize and cure when they are irradiated with light, and are therefore used for, for example, photosetting inks, photosensitive printing plates, color filters, and a variety of photoresists.
In other aspect of photopolymerization initiator, for example, an acid is generated upon application of light, and the acid serves as a catalyst. Specifically, such compositions may be used in materials for image formation, anti-counterfeiting or detection of energy-ray dose, in which a color reaction of a pigment precursor in the presence of the generated acid serving as a catalyst is used, or may be used for positive resists for use in manufacture of semiconductors, TFTs, color filters, micromachine components, and the like, in which a decomposition reaction in the presence of the generated acid serving as a catalyst is used.
In recent years, photopolymerizable compositions sensitive to shorter wavelength (365 nm or 405 nm) light sources have been demanded in various applications, and demands for such compounds (e.g. photopolymerizable initiators) capable of exhibiting high sensitivity to such short wavelength light sources have been increasing. However, highly sensitive photopolymerization initiators are generally poor in stability. Therefore, there is a demand for photopolymerization initiators having both of improved sensitivity and stability over time.
Under the circumstances, it is proposed to use oxime ester derivatives as photopolymerization initiators for photopolymerizable compositions, for example in U.S. Patent Nos. 4,255,513 and 4,590,145 and Japanese Patent Application Laid-Open (JP-A) Nos. 2000-80068, 2001-233842, 2006-342166 and 2007-231000. However, these known oxime ester compounds have low absorbance at a wavelength of 365 nm or 405 nm and are not satisfactory in terms of sensitivity. At present, there have been a demand for photopolymerizable compositions having not only high stability over time but also high sensitivity to light with a short wavelength such as 365 nm or 405 nm.
For example, JP-A No. 2005-202252 discloses a colored radiation-sensitive composition for color filters that contains an oxime compound. However, the stability over time and short-wavelength sensitivity of such a composition is still insufficient. There have also been a demand for colored radiation-sensitive compositions having improved reproducibility of hue after pattern formation, and suppression of change in coloring property over time has been strongly demanded.
On the other hand, there have been a demand for color filters for image sensors having high color concentrations and small thicknesses, in order to enhance the light-gathering power of solid-state imaging devices such as CCDs and in order to improve the image quality by improving color-separation properties. If a large amount of a coloring material is added to achieve a high color concentration, the sensitivity may be insufficient for faithful reproduction of the shape of a fine image pattern of 2.5 μm or less, so that pattern defects can frequently occur over the product. In order to avoid such defects, photo-irradiation has to be performed with higher energy, which requires a long exposure time and leads to a significant reduction in manufacturing yield. Under the circumstances, there have been a demand for colored radiation-sensitive compositions for color filters having a high content of a coloring material (colorant) and also having high sensitivity in order to achieve excelleng pattern forming property.

SUMMARY

The present invention has been made in view of the above circumstances and provides a novel compound, a photopolymerizable composition, a photopolymerizable composition for color filter, a color filter, a method for production thereof, a solid-state imaging device and a planographic printing plate precursor.
A first aspect of the invention provides a compound represented by the following Formula (1):

Formula (1)

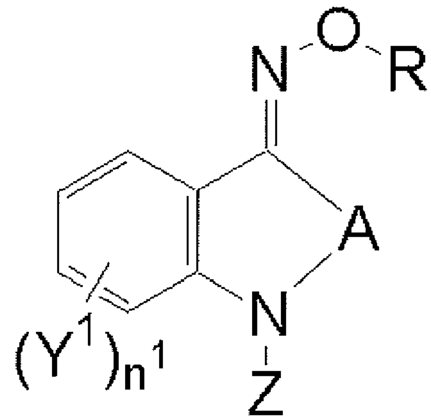

wherein R, $Y^1$ and Z each independently represent a monovalent substituent, $n^1$ represents an integer of 0 to 4, $Y^1$ and Z may be bound to each other to form a ring, and A represents a divalent linking group.

DETAILED DESCRIPTION

< Specific Oxime Compound >

Novel compounds of the present invention can be represented by Formula (I) (hereinafter referred to as "the specific oxime compound" in some cases).

Formula (1)

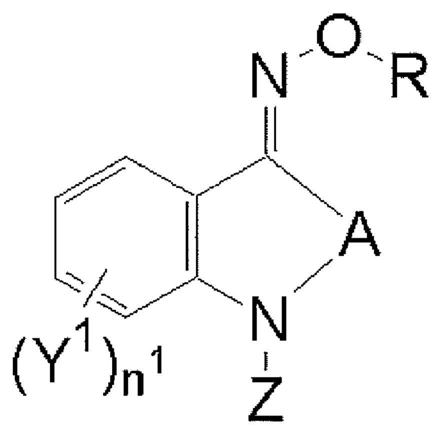

wherein R, $Y^1$ and Z independently represent a monovalent substituent, $n^1$ represents an integer of 0 to 4, and A represents a divalent linking group.

The monovalent substituent represented by R in Formula (1) is preferably a monovalent nonmetallic atomic group shown below.

The monovalent nonmetallic atomic group represented by R in Formula (1) includes an optionally substituted alkyl group, an optionally substituted aryl group, an optionally substituted alkenyl group, an optionally substituted alkynyl group, an optionally substituted alkylsulfinyl group, an optionally substituted arylsulfinyl group, an optionally substituted alkylsulfonyl group, an optionally substituted arylsulfonyl group, an optionally substituted acyl group, an optionally substituted alkoxycarbonyl group, an optionally substituted aryloxycarbonyl group, an optionally substituted phosphinoyl group, an optionally substituted heterocyclic group, an optionally substituted alkylthiocarbonyl group, an optionally substituted arylthiocarbonyl group, an optionally substituted dialkylaminocarbonyl group, and an optionally substituted dialkylaminothiocarbonyl group.

The optionally substituted alkyl group is preferably an alkyl group having 1 to 30 carbon atoms, and examples include a methyl group, an ethyl group, a propyl group, a butyl group, a hexyl group, an octyl group, a decyl group, a dodecyl group, an octadecyl grouop, an isopropyl group, an isobutyl group, a sec-butyl group, a tert-butyl group, a 1-ethylpentyl group, a cyclopentyl group, a cyclohexyl group, a trifluoromethyl grouop, a 2-ethylhexyl group, a phenacyl group, a 1-naphthoylmethyl group, a 2-naphthoylmethyl group, a 4-methylsulfanylphenacyl group, a 4-phenylsulfanylphenacyl group, a 4-dimethylaminophenacyl group, a 4-cyanophenacyl group, a 4-methylphenacyl group, a 2-methylphenacyl group, a 3-fluorophenacyl group, a 3-trifluoromethylphenacyl group, and a 3-nitrophenacyl group.

The optionally substituted aryl group is preferably an aryl group having 6 to 30 carbon atoms, and examples include a phenyl group, a biphenyl group, a 1-naphthyl group, a 2-naphthyl group, a 9-anthryl group, a 9-phenanthryl group, a 1-pyrenyl group, a 5-naphthacenyl group, a 1-indenyl group, a 2-azulenyl group, a 9-fluorenyl group, a terphenyl group, a quarterphenyl group, an o-tolyl group, a m-tolyl group, a p-tolyl group, a xylyl group, an o-cumenyl group, a m-cumenyl group, a p-cumenyl group, a mesityl group, a pentalenyl group, a binaphthalenyl group, a ternaphthalenyl group, a quarternaphthalenyl group, a heptalenyl group, a biphenylenyl group, an indacenyl group, a fluoranthenyl group, an acenaphthylenyl group, an aceanthrylenyl group, a phenalenyl group, a fluorenyl group, an anthryl group, a bianthracenyl group, a teranthracenyl group, a quarteranthracenyl group, an anthraquinolyl group, a phenanthryl group, a triphenylenyl group, a pyrenyl group, a chrysenyl group, a naphthacenyl group, a pleiadenyl group, a picenyl group, a perylenyl group, a pentaphenyl group, a pentacenyl group, a tetraphenylenyl group, a hexaphenyl group, a hexacenyl group, a rubicenyl group, a coronenyl group, a trinaphthylenyl group, a heptaphenyl group, a heptacenyl group, a pyranthrenyl group, and an ovalenyl group.

The optionally substituted alkenyl group is preferably an alkenyl group having 2 to 10 carbon atoms, and examples include a vinyl group, an allyl group and a styryl group.

The optionally substituted alkynyl group is preferably an alkynyl group having 2 to 10 carbon atoms, and examples include an ethynyl group, a propynyl group and a propargyl group.

The optionally substituted alkylsulfinyl group is preferably an alkylsulfinyl group having 1 to 20 carbon atoms, and examples include a methylsulfinyl group, an ethylsulfinyl group, a propylsulfinyl group, an isopropylsulfinyl group, a butylsulfinyl group, a hexylsulfinyl group, a cyclohexylsulfinyl group, an octylsulfinyl group, a 2-ethylhexylsulfinyl group, a decanoylsulfinyl group, a dodecanoylsulfinyl group, an octadecanoylsulfinyl group, a cyanomethylsulfinyl group, and a methoxymethylsulfinyl group.

The optionally substituted arylsulfinyl group is preferably an arylsulfinyl group having 6 to 30 carbon atoms, and examples include a phenylsulfinyl group, a 1-naphthylsulfinyl group, a 2-naphthylsulfinyl group, a 2-chlorophenylsulfinyl group, a 2-methylphenylsulfinyl group, a 2-methoxyphenylsulfinyl group, a 2-butoxyphenylsulfinyl group, a 3-chlorophenylsulfinyl group, a 3-trifluoromethylphenylsulfinyl group, a 3-cyanophenylsulfinyl group, a 3-nitrophenylsulfinyl group, a 4-fluorophenylsulfinyl group, a 4-cyanophenylsulfinyl group, a 4-methoxyphenylsulfinyl group, a 4-methylsulfanylphenylsulfinyl group, a 4-phenylsulfanylphenylsulfinyl group, and a 4-dimethylaminophenylsulfinyl group.

The optionally substituted alkylsulfonyl group is preferably an alkylsulfonyl group having 1 to 20 carbon atoms, and examples include a methylsulfonyl group, an ethylsulfonyl group, a propylsulfonyl group, an isopropylsulfonyl group, a butylsulfonyl group, a hexylsulfonyl group, a cyclohexylsulfonyl group, an octylsulfonyl group, a 2-ethylhexylsulfonyl group, a decanoylsulfonyl group, a dodecanoylsulfonyl group, an octadecanoylsulfonyl group, a cyanomethylsulfonyl group, a methoxymethylsulfonyl group, and a perfluoroalkylsulfonyl group.

The optionally substituted arylsulfonyl group is preferably an arylsulfonyl group having 6 to 30 carbon atoms, and examples include a phenylsulfonyl group, a 1-naphthylsulfonyl group, a 2-naphthylsulfonyl group, a 2-chlorophenylsulfonyl group, a 2-methylphenylsulfonyl group, a 2-methoxyphenylsulfonyl group, a 2-butoxyphenylsulfonyl group, a 3-chlorophenylsulfonyl group, a 3-trifluoromethylphenylsulfonyl group, a 3-cyanophenylsulfonyl group, a 3-nitrophenylsulfonyl group, a 4-fluorophenylsulfonyl group, a 4-cyanophenylsulfonyl group, a 4-methoxyphenylsulfonyl group, a 4-methylsulfanylphenylsulfonyl group, a 4-phenylsulfanylphenylsulfonyl group, and a 4-dimethylaminophenylsulfonyl group.

The optionally substituted acyl group is preferably an acyl group having 2 to 20 carbon atoms, and examples include an acetyl group, a propanoyl group, a butanoyl group, a trifluoromethylcarbonyl group, a pentanoyl group, a benzoyl group, a toluyl group, a 1-naphthyl group, a 2-naphthoyl group, a 4-methylsulfanylbenzoyl group, a 4-phenylsulfanylbenzoyl group, a 4-dimethylaminobenzoyl group, a 4-diethylaminobenzoyl group, a 2-chlorobenzoyl group, a 2-methylbenzoyl group, a 2-methoxybenzoyl group, a 2-butoxybenzoyl group, a 3-chlorobenzoyl group, a 3-trifluoromethylbenzoyl group, a 3-cyanobenzoyl group, a 3-nitrobenzoyl group, a 4-fluorobenzoyl group, a 4-cyanobenzoyl group, and a 4-methoxybenzoyl group.

The optionally substituted alkoxycarbonyl group is preferably an alkoxycarbonyl group having 2 to 20 carbon atoms, and examples include a methoxycarbonyl group, an ethoxycarbonyl group, a propoxycarbonyl group, a butoxycarbonyl group, a hexyloxycarbonyl group, an octyloxycarbonyl group, a decyloxycarbonyl group, an octadecyloxycarbonyl group, and a trifluoromethyloxycarbonyl group.

Examples of the optionally substituted aryloxycarbonyl group include a phenoxycarbonyl group, a 1-naphthyloxycarbonyl group, a 2-naphthyloxycarbonyl group, a 4-methylsulfanylphenyloxycarbonyl group, a 4-phenylsulfanylphenyloxycarbonyl group, a 4-dimethylaminophenyloxycarbonyl group, a 4-diethylaminophenyloxycarbonyl group, a 2-chlorophenyloxycarbonyl group, a 2-methylphenyloxycarbonyl group, a 2-methoxyphenyloxycarbonyl group, a 2-butoxyphenyloxycarbonyl group, a 3-chlorophenyloxycarbonyl group, a 3-trifluoromethylphenyloxycarbanyl group, a 3-cyanophenyloxycarbonyl group, a 3-nitrophenyloxycarbonyl group, a 4-fluorophenyloxycarbonyl group, a 4-cyanophenyloxycarbonyl group, and a 4-methoxyphenyloxycarbonyl group.

The optionally substituted phosphinoyl group is preferably a phosphinoyl group having 2 to 50 carbon atoms in total, and examples include a dimethylphosphinoyl group, a diethylphosphinoyl group, a dipropylphosphinoyl group, a diphenylphosphinoyl group, a dimethoxyphosphinoyl group, a diethoxyphosphinoyl group, a dibenzoylphosphinoyl group, and a bis(2,4,6-trimethylphenyl)phosphinoyl group.

The optionally substituted heterocyclic group is preferably an aromatic or aliphatic heterocyclic group containing at least one atom selected from nitrogen, oxygen, sulfur, and phosphorus atoms. Examples include a thienyl group, a benzo[b]thienyl group, a naphtho[2,3-b]thienyl group, a thianthrenyl group, a furyl group, a pyranyl group, an isobenzofuranyl group, a chromenyl group, a xanthenyl group, a phenoxathiinyl group, a 2H-pyrrolyl group, a pyrrolyl group, an imidazolyl group, a pyrazolyl group, a pyridyl group, a pyrazinyl group, a pyrimidinyl group, a pyridazinyl group, an indolizinyl group, an isoindolyl group, a 3H-indolyl group, an indolyl group, a 1H-indazolyl group, a purinyl group, a 4H-quinolizinyl group, an isoquinolyl group, a quinolyl group, a phthalazinyl group, a naphthyridinyl group, a quinoxalinyl group, a quinazolinyl group, a cinnolinyl group, a pteridinyl group, a 4aH-carbazolyl group, a carbazolyl group, a β-carbolinyl group, a phenanthridinyl group, an acridinyl group, a perimidinyl group, a phenanthrolinyl group, a phenazinyl group, a phenarsazinyl group, an isothiazolyl group, a phenothiazinyl group, an isoxazolyl group, a furazanyl group, a phenoxazinyl group, an isochromanyl group, a chromanyl group, a pyrrolidinyl group, a pyrrolinyl group, an imidazolidinyl group, an imidazolinyl group, a pyrazolidinyl group, a pyrazolinyl group, a piperidyl group, a piperazinyl group, an indolinyl group, an isoindolinyl group, a quinuclidinyl group, a morpholinyl group, and a thioxantholyl group.

Examples of the optionally substituted alkylthiocarbonyl group include a methylthiocarbonyl group, a propylthiocarbonyl group, a butylthiocarbonyl group, a hexylthiocarbonyl group, an octylthiocarbonyl group, a decylthiocarbonyl group, an octadecylthiocarbonyl group, and a trifluoromethylthiocarbonyl group.

Examples of the optionally substituted arylthiocarbonyl group include a 1-naphthylthiocarbonyl group, a 2-naphthylthiocarbonyl group, a 4-methylsulfanylphenylthiocarbonyl group, a 4-phenylsulfanylphenylthiocarbonyl group, a 4-dimethylaminophenylthiocarbonyl group, a 4-diethylaminophenylthiocarbonyl group, a 2-chlorophenylthiocarbonyl group, a 2-methylphenylthiocarbonyl group, a 2-methoxyphenylthiocarbonyl group, a 2-butoxyphenylthiocarbonyl group, a 3-chlorophenylthiocarbonyl group, a 3-trifluoromethylphenylthiocarbonyl group, a 3-cyanophenylthiocarbonyl group, a 3-nitrophenylthiocarbonyl group, a 4-fluorophenylthiocarbonyl group, a 4-cyanophenylthiocarbonyl group, and a 4-methoxyphenylthiocarbonyl group.

Examples of the optionally substituted dialkylaminocarbonyl group include a dimethylaminocarbonyl group, a diethylaminocarbonyl group, a dipropylaminocarbonyl group, and a dibutylaminocarbonyl group.

Examples of the optionally substituted dialkylaminothiocarbonyl group include a dimethylaminothiocarbonyl group, a dipropylaminothiocarbonyl group and a dibutylaminothiocarbonyl group.

In particular, R is more preferably an acyl group from the viewpoint of higher sensitivity, and specifically, an acetyl group, an ethyloyl group, a propioyl group, a benzoyl group and a toluyl group are preferable.

The monovalent substituent represented by $Y^1$ in Formula (1) includes an optionally substituted alkyl group, an optionally substituted aryl group, an optionally substituted alkenyl group, an optionally substituted alkynyl group, an optionally substituted alkoxy group, an optionally substituted aryloxy group, an optionally substituted alkylthioxy group, an optionally substituted arylthioxy group, an optionally substituted acyloxy group, an optionally substituted alkylsulfanyl group, an optionally substituted arylsulfanyl group, an optionally substituted alkylsulfinyl group, an optionally substituted arylsulfinyl group, an optionally substituted alkylsulfonyl group, an optionally substituted arylsulfonyl group, an optionally substituted acyl group, an optionally substituted alkoxycarbonyl group, an optionally substituted aryloxycarbonyl group, an optionally substituted carbamoyl group, an optionally substituted sulfamoyl group, an optionally substituted amino group, an optionally substituted phosphinoyl group, an optionally substituted heterocyclic group, a halogen group, a hydrogen atom, a cyano group, a nitro group, a carboxylic acid group, a sulfonic acid group, and a sulfinic acid group.

Among the monovalent substituents represented by $Y^1$ in Formula (1), the optionally substituted alkyl group, optionally substituted aryl group, optionally substituted alkenyl group, optionally substituted alkynyl group, optionally substituted alkylsulfinyl group, optionally substituted arylsulfinyl group, optionally substituted alkylsulfonyl group, optionally substituted arylsulfonyl group, optionally substituted acyl group, optionally substituted alkoxycarbonyl group, optionally substituted aryloxycarbonyl group, optionally substituted phosphinoyl group, or optionally substituted heterocyclic group has the same meaning as that of the monovalent nonmetallic atomic group represented by R in Formula (1), and

the number of carbon atoms is also the same as defined therein.

The optionally substituted alkoxy group is preferably an alkoxy group having 1 to 30 carbon atoms, and examples include a methoxy group, an ethoxy group, a propyloxy group, an isopropyloxy group, a butoxy group, an isobutoxy group, a sec-butoxy group, a tert-butoxy group, a pentyloxy group, an isopentyloxy group, a hexyloxy group, a heptyloxy group, an octyloxy group, a 2-ethylhexyloxy group, a decyloxy group, a dodecyloxy group, an octadecyloxy group, an ethoxycarbonylmethyl group, a 2-ethylhexyloxycarbonylmethyloxy group, an aminocarbonylmethyloxy group, an N,N-dibutylaminocarbonylmethyloxy group, an N-methylaminocarbonylmethyloxy group, an N-ethylaminocarbonylmethyloxy group, an N-octylaminocarbonylmethyloxy group, an N-methyl-N-benzylaminocarbonylmethyloxy group, a benzyloxy group, and a cyanomethyloxy group.

The optionally substituted aryloxy group is preferably an aryloxy group having 6 to 30 carbon atoms, and examples include a phenyloxy group, a 1-naphthyloxy group, a 2-naphthyloxy group, a 2-chlorophenyloxy group, a 2-methylphenyloxy group, a 2-methoxyphenyloxy group, a 2-butoxyphenyloxy group, a 3-chlorophenyloxy group, a 3-trifluoromethylphenyloxy group, a 3-cyanophenyloxy group, a 3-nitrophenyloxy group, a 4-fluorophenyloxy group, a 4-cyanophenyloxy group, a 4-methoxyphenyloxy group, a 4-dimethylaminophenyloxy group, a 4-methylsulfanylphenyloxy group, and a 4-phenylsulfanylphenyloxy group.

The optionally substituted alkylthioxy group is preferably a thioalkoxy group having 1 to 30 carbon atoms, and examples include a methylthioxy group, an ethylthioxy group, a propylthioxy group, an isopropylthioxy group, a butylthioxy group, an isobutylthioxy group, a sec-butylthioxy group, a tert-butylthioxy group, a pentylthioxy group, an isopentylthioxy group, a hexylthioxy group, a heptylthioxy group, an octylthioxy group, a 2-ethylhexylthioxy group, a decylthioxy group, a dodecylthioxy group, an octadecylthioxy group, and a benzylthioxy group.

The optionally substituted arylthioxy group is preferably an arylthioxy group having 6 to 30 carbon atoms, and examples include a phenylthioxy group, a 1-naphthylthioxy group, a 2-naphthylthioxy group, a 2-chlorophenylthioxy group, a 2-methylphenylthioxy group, a 2-methoxyphenylthioxy group, a 2-butoxyphenylthioxy group, a 3-chlorophenylthioxy group, a 3-trifluoromethylphenylthioxy group, a 3-cyanophenylthioxy group, a 3-nitrophenylthioxy group, a 4-fluorophenylthioxy group, a 4-cyanophenylthioxy group, a 4-methoxyphenylthioxy group, a 4-dimethylaminophenylthioxy group, a 4-methylsulfanylphenylthioxy group, and a 4-phenylsulfanylphenylthioxy group.

The optionally substituted acyloxy group is preferably an acyloxy group having 2 to 20 carbon atoms, and examples include an acetyloxy group, a propanoyloxy group, a butanoyloxy group, a pentanoyloxy group, a trifluoromethylcarbonyloxy group, a benzoyloxy group, a 1-naphthylcarbonyloxy group, and a 2-naphthylcarbonyloxy group.

The optionally substituted alkylsulfanyl group is preferably an alkylsulfanyl group having 1 to 20 carbon atoms, and examples include a methylsulfanyl group, an ethylsulfanyl group, a propylsulfanyl group, an isopropylsulfanyl group, a butylsulfanyl group, a hexylsulfanyl group, a cyclohexylsulfanyl group, an octylsulfanyl group, a 2-ethylhexylsulfanyl group, a decanoylsulfanyl group, a dodecanoylsulfanyl group, an octadecanoylsulfanyl group, a cyanomethylsulfanyl group, and a methoxymethylsulfanyl group.

The optionally substituted arylsulfanyl group is preferably an arylsulfanyl group having 6 to 30 carbon atoms, and examples include a phenylsulfanyl group, a 1-naphthylsulfanyl group, a 2-naphthylsulfanyl group, a 2-chlorophenylsulfanyl group, a 2-methylphenylsulfanyl group, a 2-methoxyphenylsulfanyl group, a 2-butoxyphenylsulfanyl group, a 3-chlorophenylsulfanyl group, a 3-trifluoromethylphenylsulfanyl group, a 3-cyanophenylsulfanyl group, a 3-nitrophenylsulfanyl group, a 4-fluorophenylsulfanyl group, a 4-cyanophenylsulfanyl group, a 4-methoxyphenylsulfanyl group, a 4-methylsulfanylphenylsulfanyl group, a 4-phenylsulfanylphenylsulfanyl group, and a 4-dimethylaminophenylsulfanyl group.

The optionally substituted carbamoyl group is preferably a carbamoyl group having 1 to 30 carbon atoms in total, and examples include a N-methylcarbamoyl group, an N-ethylcarbamoyl group, an N-propylcarbamoyl group, an N-butylcarbamoyl group, an N-hexylcarbamoyl group, an N-cyclohexylcarbamoyl group, an N-octylcarbamoyl group, an N-decylcarbamoyl group, an N-octadecylcarbamoyl group, an N-phenylcarbamoyl group, an N-2-methylphenylcarbamoyl group, an N-2-chlorophenylcarbamoyl group, an N-2-isopropoxyphenylearbamoyl group, an N-2-(2-ethylhexyl)phenylcarbamoyl group, an N-3-chlorophenylcarbamoyl group, an N-3-nitrophenylcarbamoyl group, an N-3-cyanophenylcarbamoyl group, an N-4-methoxyphenylcarbamoyl group, an N-4-cyanophenylcarbamoyl group, an N-4-methylsulfanylphenylcarbamoyl group, an N-4-phenylsulfanylphenylcarbamoyl group, an N-methyl-N-phenylcarbamoyl group, an N,N-dimethylcarbamoyl group, an N,N-dibutylcarbamoyl group, and an N,N-diphenylcarbamoyl group.

The optionally substituted sulfamoyl group is preferably a sulfamoyl group having 0 to 30 carbon atoms in total, and examples include a sulfamoyl group, an N-alkylsulfamoyl group, an N-arylsulfamoyl group, an N,N-dialkylsulfamoyl group, an N,N-diarylsulfamoyl group, and an N-alkyl-N-arylsulfamoyl group. More specifically, examples include an N-methylsulfamoyl group, an N-ethylsulfamoyl group, an N-propylsulfamoyl group, an N-butylsulfamoyl group, an N-hexylsulfamoyl group, an N-cyclohexylsulfamoyl group, an N-octylsulfamoyl group, an N-2-ethylhexylsulfamoyl group, an N-decylsulfamoyl group, an N-octadecylsulfamoyl group, an N-phenylsulfamoyl group, an N-2-methylphenylsulfamoyl group, an N-2-chlorophenylsulfamoyl group, an N-2-methoxyphenylsulfamoyl group, an N-2-isopropoxyphenylsulfamoyl group, an N-3-chlorophenylsulfamoyl group, an N-3-nitrophenylsulfamoyl group, an N-3-cyanophenylsulfamoyl group,

an N-4-methoxyphenylsulfamoyl group, an N-4-cyanophenylsulfamoyl group, an N-4-dimethylaminophenylsulfamoyl group, an N-4-methylsulfanylphenylsulfamoyl group, an N-4-phenylsulfanylphenylsulfamoyl group, an N-methyl-N-phenylsulfamoyl group, an N,N-dimethylsulfamoyl group, an N,N-dibutylsulfamoyl group, and an N,N-diphenylsulfamoyl group.

The optionally substituted amino group is preferably an amino group having 0 to 50 carbon atoms in total, and examples include $-NH_2$, an N-alkylamino group, an N-arylamino group, an N-acylamino group, an N-sulfonylamino group, an N,N-dialkylamino group, an N,N-diarylamino group, an N-alkyl-N-arylamino group, and an N,N-disulfonylamino group. More specifically, examples include an N-methylamino group, an N-ethylamino group, an N-propylamino group, an N-isopropylamino group, an N-butylamino group, an N-tert-butylamino group, an N-hexylamino group, an N-cyclohexylamino group, an N-octylamino group, an N-2-ethylhexylamino group, an N-decylamino group, an N-octadecylamino group, an N-benzylamino group, an N-phenylamino group, an N-2-methylphenylamino group, an N-2-chlorophenylamino group, an N-2-methoxyphenylamino group, an N-2-isopropoxyphenylamino group, an N-2-(2-ethylhexyl)phenylamino group, an N-3-chlorophenylamino group, an N-3-nitrophenylamino group, an N-3-cyanophenylamino group, an N-3-trifluoromethylphenylamino group, an N-4-methoxyphenylamino group, an N-4-cyanophenylamino group, an N-4-trifluoromethylphenylamino group, an N-4-methylsulfanylphenylamino group, an N-4-phenylsulfanylphenylamino group, an N-4-dimethylaminophenylamino group, an N-methyl-N-phenylamino group, an N,N-dimethylamino group, an N,N-diethylamino group, an N,N-dibutylamino group, an N,N-diphenylamino group, an N,N-diacetylamino group, an N,N-dibenzoylamino group, an N,N-(dibutylcarbonyl)amino group, an N,N-(dimethylsulfonyl)amino group, an N,N-(diethylsulfonyl)amino group, an N,N-(dibutylsulfonyl)amino group, an N,N-(diphenylsulfonyl)amino group, a morpholino group, a 3,5-dimethylmorpholino group, and a carbazole group.

The halogen group may be a fluorine atom, a chlorine atom, a bromine atom, an iodine atom, or the like.

The optionally substituted alkyl group, optionally substituted aryl group, optionally substituted alkenyl group, optionally substituted alkynyl group, optionally substituted alkoxy group, optionally substituted aryloxy group, optionally substituted alkylthioxy group, optionally substituted arylthioxy group, optionally substituted acyloxy group, optionally substituted alkylsulfanyl group, optionally substituted arylsulfanyl group, optionally substituted alkylsulfinyl group, optionally substituted arylsulfinyl group, optionally substituted alkylsulfonyl group, optionally substituted arylsulfonyl group, optionally substituted acyl group, optionally substituted alkoxycarbonyl group, optionally substituted carbamoyl group, optionally substituted sulfamoyl group, optionally substituted amino group, or optionally substituted heterocyclic group may themselves be further substituted by any other substituent.

Examples of such a further substituent include a halogen group such as a fluorine, chlorine, bromine, or iodine atom; an alkoxy group such as a methoxy group, an ethoxy group, or a tert-butoxy group; an aryloxy group such as a phenoxy group or a p-tolyloxy group; an alkoxycarbonyl group such as a methoxycarbonyl group, a butoxycarbonyl group or a phenoxycarbonyl group; an acyloxy group such as an acetoxy group, a propionyloxy group or a benzoyloxy group; an acyl group such as an acetyl group, a benzoyl group, an isobutyryl group, an acryloyl group, a methacryloyl group, or a methoxalyl group; an alkylsulfanyl group such as a methylsulfanyl group or a tert-butylsulfanyl group; an arylsulfanyl group such as a phenylsulfanyl group or a p-tolylsulfanyl group; an alkylamino group such as a methylamino group or a cyclohexylamino group; a dialkylamino group such as a dimethylamino group, a diethylamino group, a morpholino group, or a piperidino group; an arylamino group such as a phenylamino group or a p-tolylamino group; an alkyl group such as a methyl group, an ethyl group, a tert-butyl group or a dodecyl group; an aryl group such as a phenyl group, a p-tolyl group, a xylyl group, a cumenyl group, a naphthyl group, an anthryl group, or a phenanthryl group; a hydroxyl group, a carboxyl group, a formyl group, a mercapto group, a sulfo group, a mesyl group, a p-toluenesulfonyl group, an amino group, a nitro group, a cyano group, a trifluoromethyl group, a trichloromethyl group, a trimethylsilyl group, a phosphinico group, a phosphono group, a trimethylammoniumyl group, a dimethylsulfoniumyl group, or a triphenylphenacylphosphoniumyl group.

$n^1$ in Formula (1) represents an integer of 0 to 4.
$n^1$ is preferably 0 to 2, more preferably 2. When $n^1$ is an integer of 2 or more, the respective Y's may be bound to one another to form an aliphatic or aromatic ring. The ring includes aliphatic or aromatic hydrocarbon rings or heteroatom-containing hetero rings. These rings may further be combined to form a polycyclic condensed ring.

The aliphatic or aromatic hydrocarbon ring includes 6-, 5- and 7-membered hydrocarbon rings, preferably 6- and 5-membered rings.
The hetero ring includes hetero rings having a sulfur, oxygen or nitrogen atom as a heteroatom, among which a hetero ring having a sulfur atom is more preferable.

When the aliphatic or aromatic hydrocarbon rings are further combined to form a polycyclic condensed ring, the polycyclic condensed ring includes a condensed ring formed by 1 to 4 benzene rings, a condensed ring formed by a benzene ring and a 5-membered unsaturated ring, etc. The heteroatom-containing hetero ring includes a condensed ring formed by a benzene ring and a 5-membered ring containing a sulfur, oxygen or nitrogen atom, a condensed ring formed by a benzene ring and a 6-membered ring containing a sulfur, oxygen or nitrogen atom, etc.

Specific examples of rings, which may be formed by binding $Y^1$'s to one another, include a benzene ring, a

naphthalene ring, an anthracene ring, a phenanthrene ring, a fluorene ring, a triphenylene ring, a naphthacene ring, a biphenyl ring, a pyrrole ring, a furan ring, a thiophene ring, a dithiolane ring, an oxirane ring, an dioxirane ring, a thirane ring, a pyrrolidine ring, a piperidine ring, an imidazole ring, an isoxazole ring, a benzodithiol ring, an oxazole ring, a thiazole ring, a benzothiazole ring, a benzimidazole ring, a benzoxazole ring, a pyridine ring, a pyrazine ring, a pyrimidine ring, a pyridazine ring, an indolizine ring, an indole ring, a benzofuran ring, a benzothiophene ring, a benzodithiol ring, an isobenzofuran ring, a quinolizine ring, a quinoline ring, a phthalazine ring, a naphthyridine ring, a quinoxaline ring, a quinazoline ring, an isoquinoline ring, a carbazole ring, a phenanthridine ring, an acridine ring, a phenanthroline ring, a thianthrene ring, a chromene ring, a xanthene ring, a phenoxathiine ring, a phenothiazine ring, and a phenazine ring.

The monovalent substituent represented by Z in Formula (1) includes an optionally substituted alkyl group, an optionally substituted aryl group, an optionally substituted alkenyl group, an optionally substituted alkynyl group, an optionally substituted alkylsulfanyl group, an optionally substituted arylsulfanyl group, an optionally substituted alkylsulfinyl group, an optionally substituted arylsulfinyl group, an optionally substituted alkylsulfonyl group, an optionally substituted arylsulfonyl group, an optionally substituted acyl group, an optionally substituted alkoxycarbonyl group, an optionally substituted carbamoyl group, and a hydrogen atom.

Specific examples of the monovalent substituent represented by Z in Formula (1) and the range of preferable number of carbon atoms is the same as exemplified by the monovalent substituent represented by $Y^1$ in Formula (1).

$Y^1$ in Formula (1) and Z in Formula (1) may be bound to each other to form an aliphatic or aromatic ring. The ring includes aliphatic or aromatic hydrocarbon rings or heteroatom-containing hetero rings. These rings may further be combined to form a polycyclic condensed ring.

The aliphatic or aromatic hydrocarbon ring includes 6-, 5- and 7-membered hydrocarbon rings and are preferably 6- and 5-membered rings, more preferably a 5-membered ring.

The hetero ring includes hetero rings having a sulfur, oxygen or nitrogen atom as a heteroatom, among which a hetero ring having a sulfur atom is more preferable.

When the aliphatic or aromatic hydrocarbon rings are further combined to form a polycyclic condensed ring, the polycyclic condensed ring includes a condensed ring formed by 1 to 4 benzene rings, a condensed ring formed by a benzene ring and a 5-membered unsaturated ring, etc. The heteroatom-containing hetero ring includes a condensed ring formed by a benzene ring and a 5-membered ring containing a sulfur, oxygen or nitrogen atom, a condensed ring formed by a benzene ring and a 6-membered ring containing a sulfur, oxygen or nitrogen atom, etc.

Specific examples of the ring, which may be formed by binding $Y^1$ in Formula (1) and Z in Formula (1) to each other, include a benzene ring, a naphthalene ring, an anthracene ring, a phenanthrene ring, a fluorene ring, a triphenylene ring, a naphthacene ring, a biphenyl ring, a pyrrole ring, a furan ring, a thiophene ring, a dithiolane ring, an oxirane ring, an dioxirane ring, a thirane ring, a pyrrolidine ring, a piperidine ring, an imidazole ring, an isoxazole ring, a benzodithiol ring, an oxazole ring, a thiazole ring, a benzothiazole ring, a benzimidazole ring, a benzoxazole ring, a pyridine ring, a pyrazine ring, a pyrimidine ring, a pyridazine ring, an indolizine ring, an indole ring, a benzofuran ring, a benzothiophene ring, a benzodithiol ring, an isobenzofuran ring, a quinolizine ring, a quinoline ring, a phthalazine ring, a naphthyridine ring, a quinoxaline ring, a quinoxazoline ring, an isoquinoline ring, a carbazole ring, a phenanthridine ring, an acridine ring, a phenanthroline ring, a thianthrene ring, a chromene ring, a xanthene ring, a phenoxathiine ring, a phenothiazine ring, and a phenazine ring.

A in Formula (1) represents a divalent linking group.

Specific examples of A in Formula (1) include an optionally substituted alkylene group having 1 to 6 carbon atoms, an optionally substituted alkynylene group having 1 to 6 carbon atoms, etc.

The substituent includes, for example, a halogen atom such as a fluorine atom, a chlorine atom, a bromine atom, or an iodine atom, an alkoxy group such as a methoxy group, an ethoxy group, a tert-butoxy group, or an alkyleneoxide group, an aryloxy group such as a phenoxy group or a p-tolyloxy group, an alkoxycarbonyl group such as a methoxycarbonyl group, a butoxycarbonyl group, or a phenoxycarbonyl group, an acyloxy group such as an acetoxy group, a propionyloxy group, or a benzoyloxy group, an acyl group such as an acetyl group, a benzoyl group, an isobutyryl group, an acryloyl group, a methacryloyl group, a methoxalyl group, a benzoyl group, or a tolyl group, an alkylsulfanyl group such as a methylsulfanyl group or a tert-butylsulfanyl group, an arylsulfanyl group such as a phenylsulfanyl group or a p-tolylsulfanyl group, an alkylamino group such as a methylamino group or a cyclohexylamino group, a dialkylamino group such as a dimethylamino group, a diethylamino group, a morpholino group, or a piperidino group, an arylamino group such as a phenylamino group or a p-tolylamino group, an alkyl group such as a methyl group, an ethyl group, a tert-butyl group, or a dodecyl group, an alkenyl group such as an allyl group, a vinyl group, or a propenyl group, an aryl group such as a phenyl group, a p-tolyl group, a xylyl group, a cumenyl group, a naphthyl group, an anthryl group, or a phenanthryl group, a hydroxyl group, a carboxyl group, a formyl group, a mercapto group, a sulfo group, a mesyl group, a p-toluenesulfonyl group, an amino group, a nitro group, a cyano group, a trifluhaloalkyl group, a trialkylsilyl group, a phosphono group, an ammonium group, a sulfonium group, or a phosphonium group.

Preferable examples of A in Formula (1) include divalent linking groups represented by a partial structure shown below. In the following specific examples, one of two * symbols binds to the carbon atom (C) in a partial structure ">C=N-O-R" in Formula (1), and the other binds to the nitrogen atom (N) in a partial structure ">N-Z" in Formula (1).

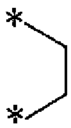
(1)
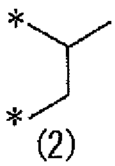
(2)
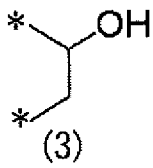
(3)
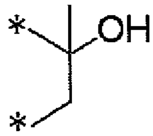
(4)
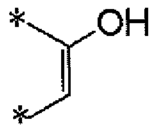
(5)
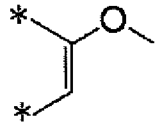
(6)
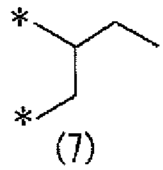
(7)

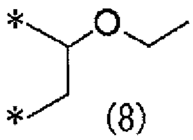
(8)
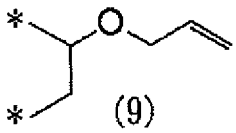
(9)
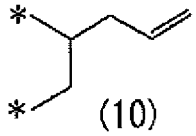
(10)
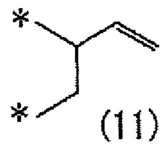
(11)
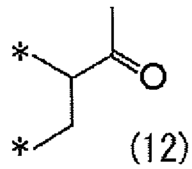
(12)

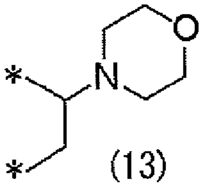
(13)
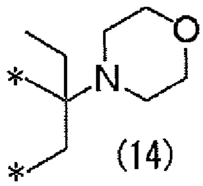
(14)
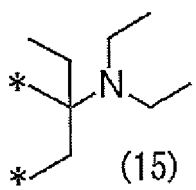
(15)
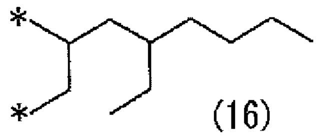
(16)

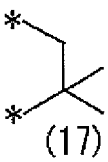
(17)
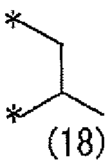
(18)
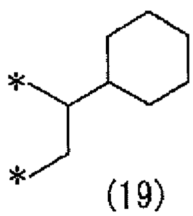
(19)
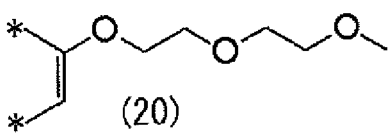
(20)
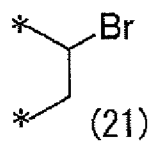
(21)

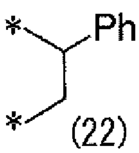
(22)
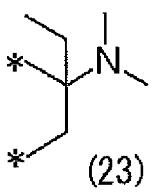
(23)
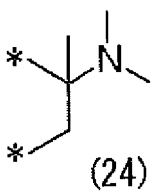
(24)
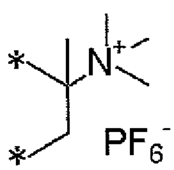
(25)
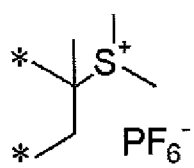
(26)

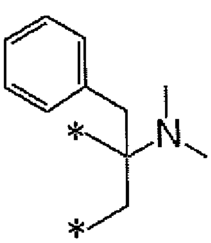
(27)
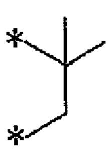
(28)
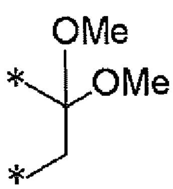
(29)
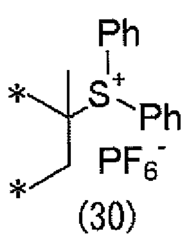
(30)
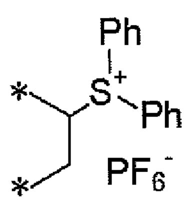
(31)

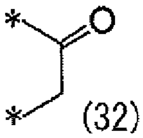
(32)
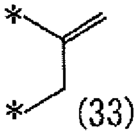
(33)
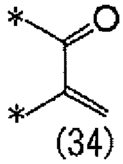
(34)
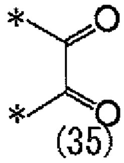
(35)
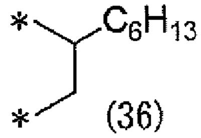
(36)

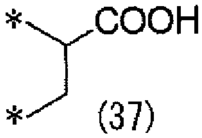
(37)
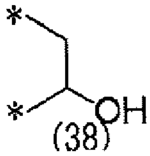
(38)
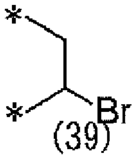
(39)
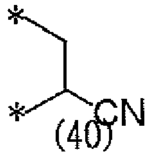
(40)
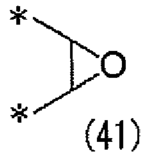
(41)

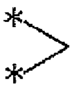
(42)
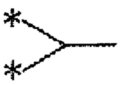
(43)
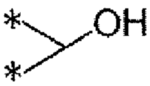
(44)
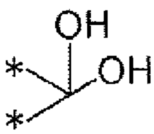
(45)
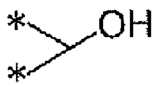
(46)
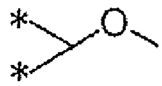
(47)
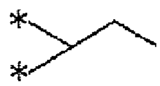
(48)

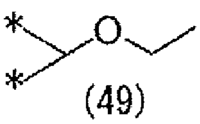
(49)
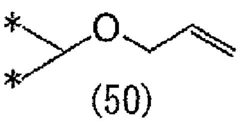
(50)
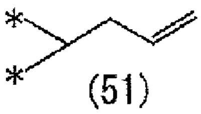
(51)
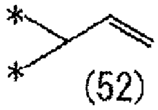
(52)
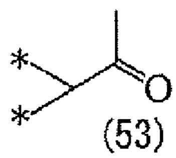
(53)

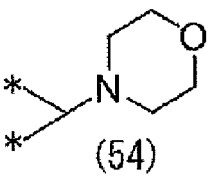
(54)
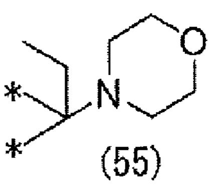
(55)
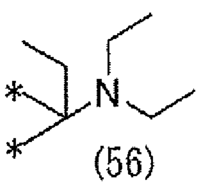
(56)
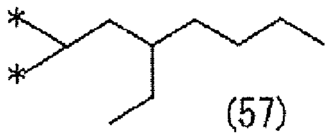
(57)

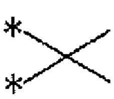
(58)
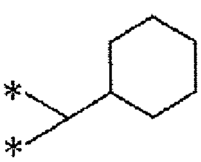
(59)
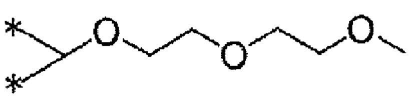
(60)
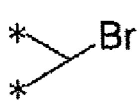
(61)

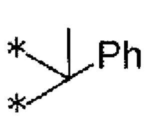
(62)
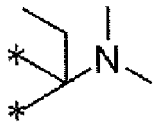
(63)
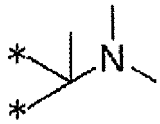
(64)
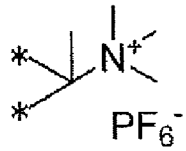
(65)
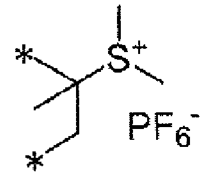
(66)

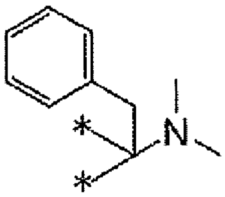
(67)
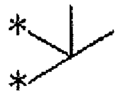
(68)
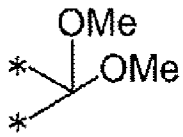
(69)
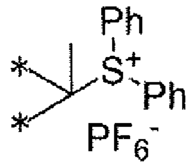
(70)
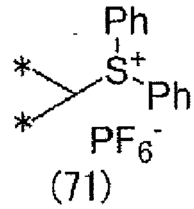
(71)

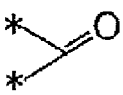
(72)
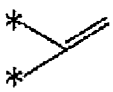
(73)
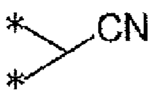
(74)
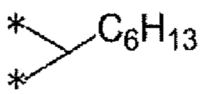
(75)
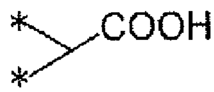
(76)

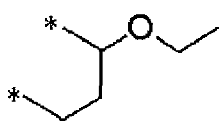
(77)
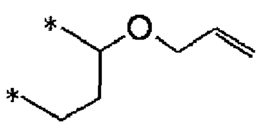
(78)
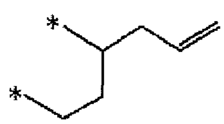
(79)
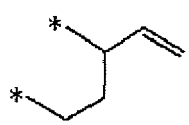
(80)

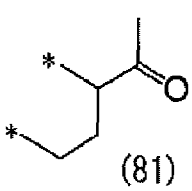
(81)
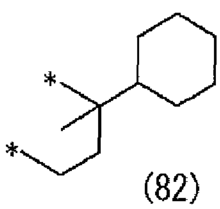
(82)
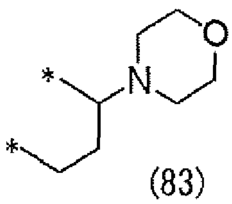
(83)
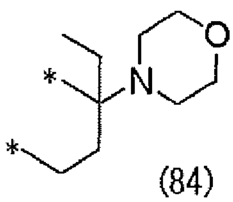
(84)

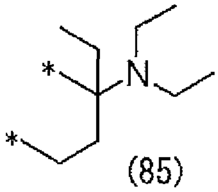
(85)
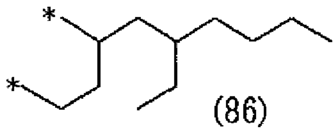
(86)
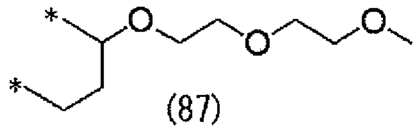
(87)

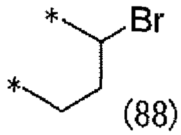
(88)
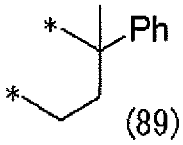
(89)
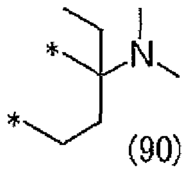
(90)
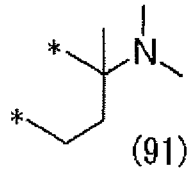
(91)
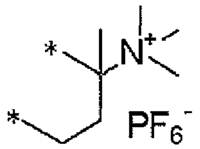
(92)

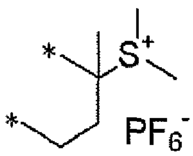
(93)
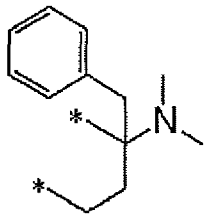
(94)
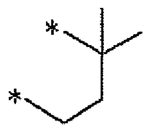
(95)
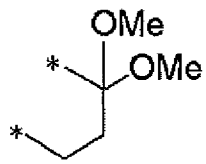
(96)
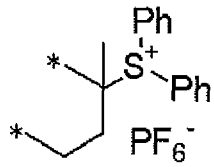
(97)

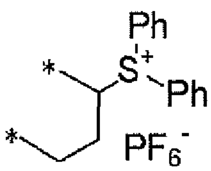
(98)
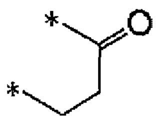
(99)
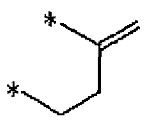
(100)
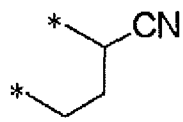
(101)

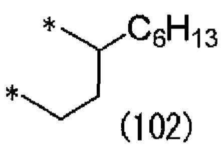
(102)
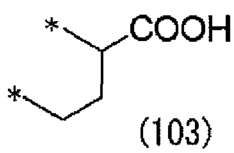
(103)
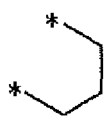
(104)
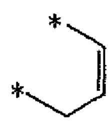
(105)
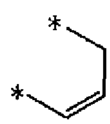
(106)

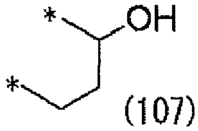
(107)
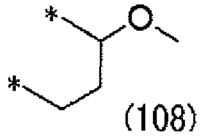
(108)
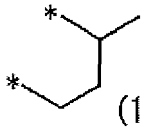
(109)

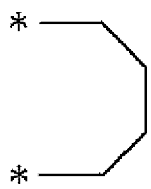
(110)

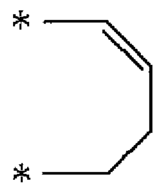
(111)

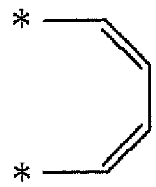
(112)

Among the specific examples (1) to (112), the examples (1) and (2) are preferable, and (1) is most preferable, from the viewpoint of attaining higher sensitivity.
In Formula (1), the combination of R, $Y^1$, $n^1$, Z and A is preferably a combination of preferable range of each R, $Y^1$, $n^1$, Z and A.
The specific oxime compound of the invention is preferably a compound represented by the following Formula (2):
Formula (2)

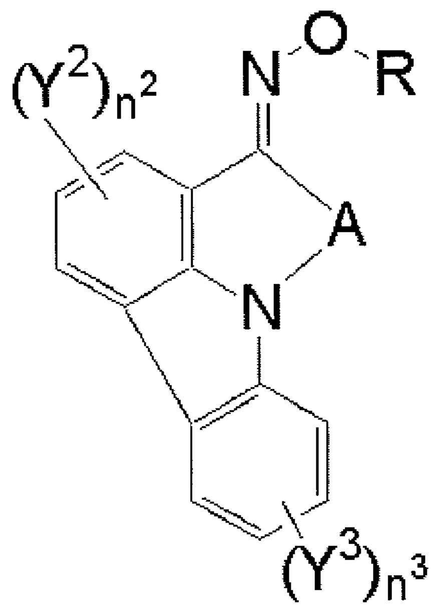

wherein R, $Y^2$ and $Y^3$ each independently represent a monovalent substituent, $n^2$ and $n^3$ independently represent an integer of 0 to 4, and A represents a divalent linking group.
R in Formula (2) has the same meaning as defined in R in Formula (1), and preferable examples thereof are also the same as defined therein.
A in Formula (2) has the same meaning as defined in A in Formula (1), and preferable examples thereof are also the same as defined therein.
The monovalent substituent represented by $Y^2$ or $Y^3$ in Formula (2) has the same meaning as that of the monovalent substituent represented by $Y^1$ in Formula (1), and preferable number of carbon atoms is also the same as defined therein.
In particular, $Y^2$ in Formula (2) is preferably an alkyl group, an alkenyl group, an alkenyl group, an alkyloxy group, or a halogen atom and is preferably unsubstituted.
$Y^3$ in Formula (2) is preferably an alkyl group, an alkenyl group, an alkynyl group, an alkyloxy group, a halogen atom, or an acyl group and is particularly preferably a substituent capable of representing the compound of Formula (3) below.
$n^2$ and $n^3$ independently represent 0 or 1, and more preferably $n^2$ is 0 and $n^3$ is 1.

In Formula (2), the combination ofR, $Y^2$, $n^2$, $Y^3$, $n^3$, Z and A is preferably a combination of preferable range of each R, $Y^2$, $n^2$, $Y^3$, $n^3$, Z and A.

The specific oxime compound of the invention is preferably a compound represented by the following Formula (3):

Formula (3)

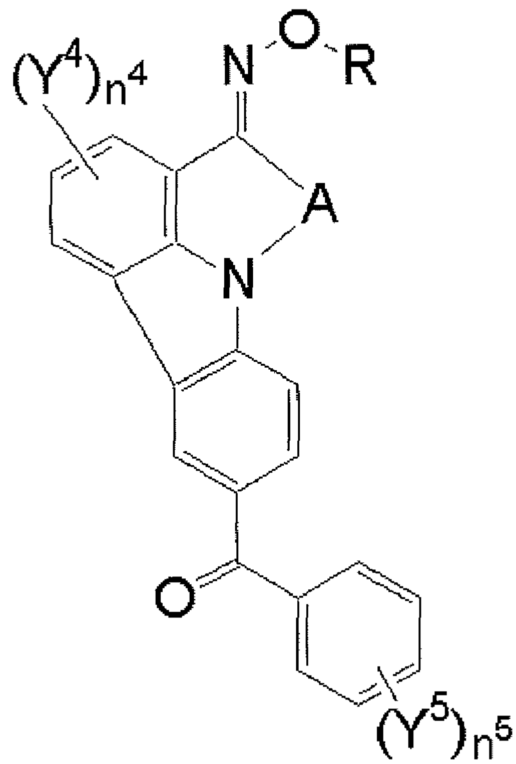

wherein R, $Y^4$ and $Y^5$ each independently represent a monovalent substituent, $n^4$ and $n^5$ independently represent an integer of 0 to 4, and A represents a divalent linking group.

R in Formula (3) has the same meaning as defined in R in Formula (1), and preferable examples thereof are also the same as defined therein.

A in Formula (3) has the same meaning as defined in A in Formula (1), and preferable examples thereof are also the same as defined therein.

The monovalent substituent represented by $Y^4$ or $Y^5$ in Formula (3) has the same meaning as that of the monovalent substituent represented by $Y^1$ in Formula (1), and the preferable number of carbon atoms is also the same as defined therein.

In particular, $Y^4$ in Formula (3) is preferably an alkyl group, an alkenyl group, an alkynyl group, an alkyloxy group, or a halogen atom, and is more preferably unsubstituted.

$Y^5$ in Formula (3) is preferably an alkyl group, an alkenyl group, an alkynyl group, an alkyloxy group, a halogen atom, an alkylthioxy group, an aryloxy group, or an arylthioxy group, more preferably an alkyl group.

$n^4$ and $n^5$ independently represent 0 or 1, and more preferably $n^4$ is 0 and $n^5$ is 1.

In Formula (3), the combination ofR, $Y^4$, $n^4$, $Y^5$, $n^5$, Z and A is preferably a combination of preferable range of each R, $Y^4$, $n^4$, $Y^5$, $n^5$, Z and A.

Examples of the specific oxime compound of the invention are shown hereinafter, however, the invention is not limited thereto.

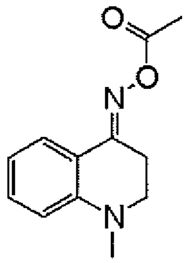
(201)

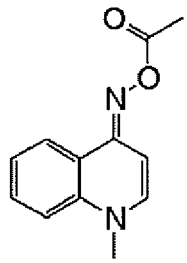
(202)

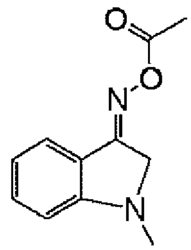
(203)

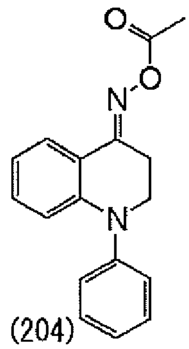
(204)

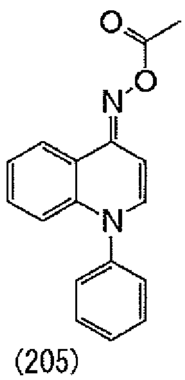
(205)

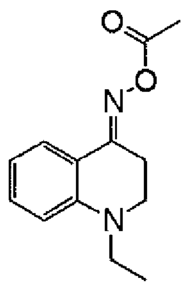
(206)

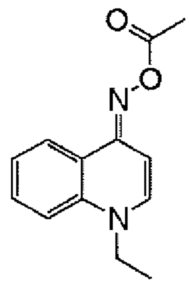
(207)

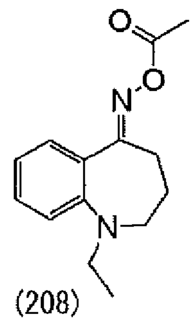
(208)

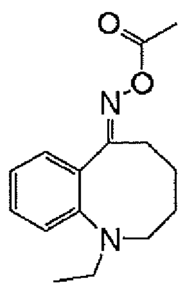
(209)

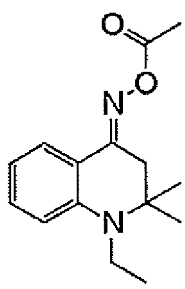
(210)

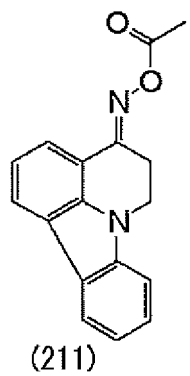
(211)

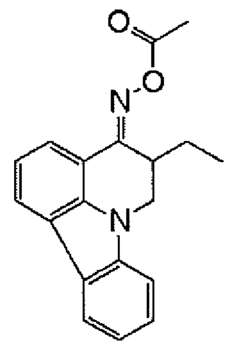
(212)

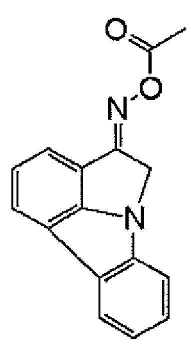
(213)

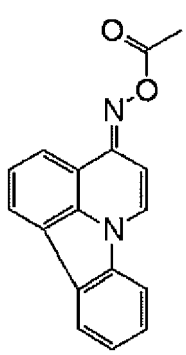
(214)

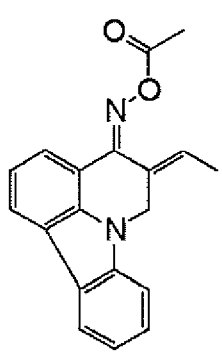
(215)

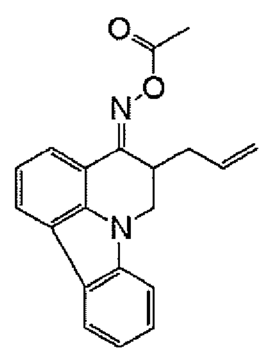
(216)

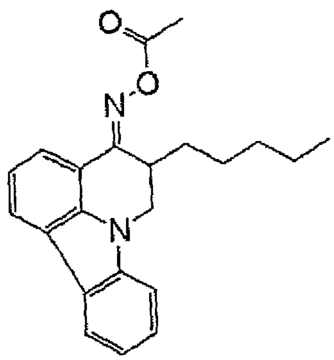
(217)

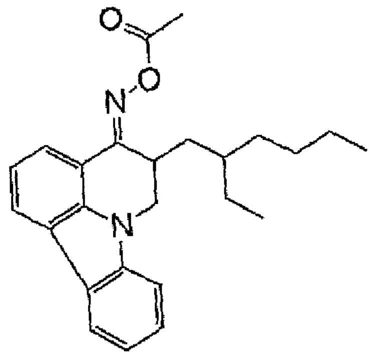
(218)

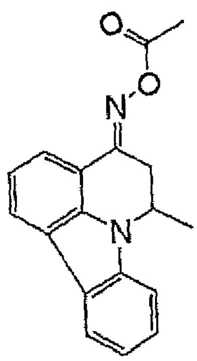
(219)

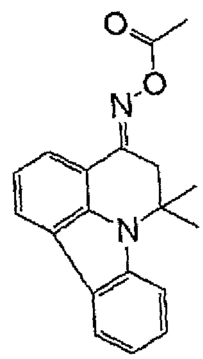
(220)

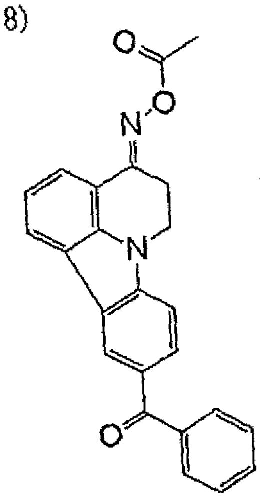
(221)

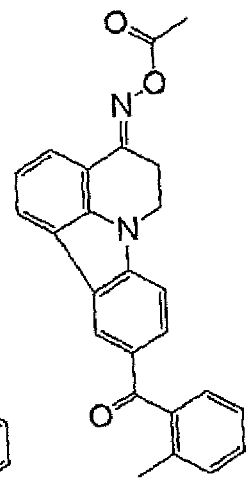
(222)

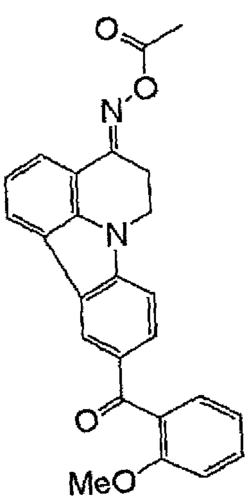
(223)

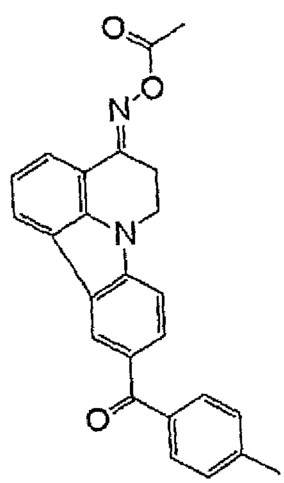
(224)

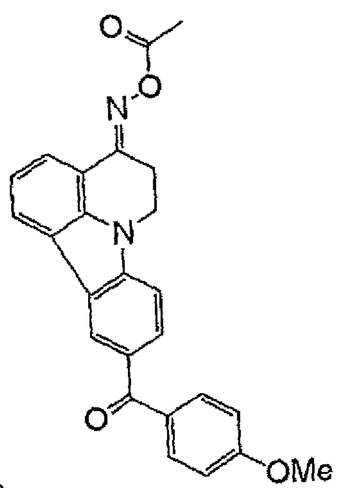
(225)

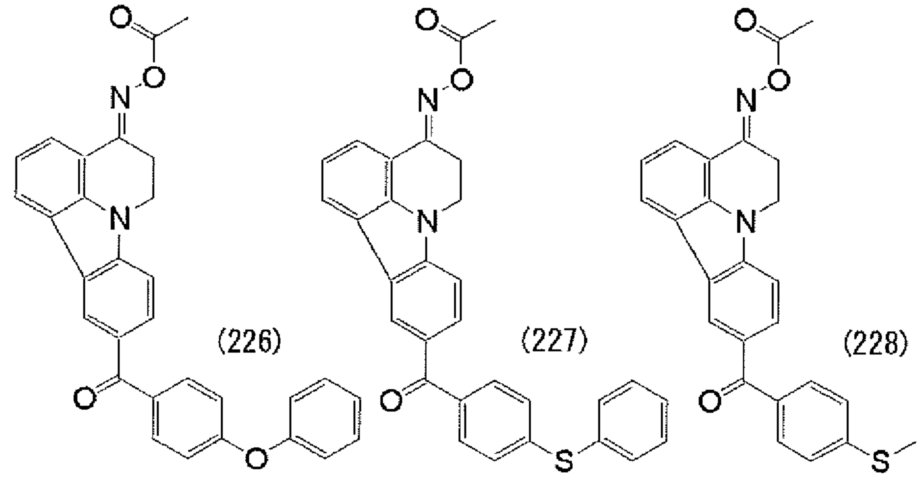

(226) (227) (228)

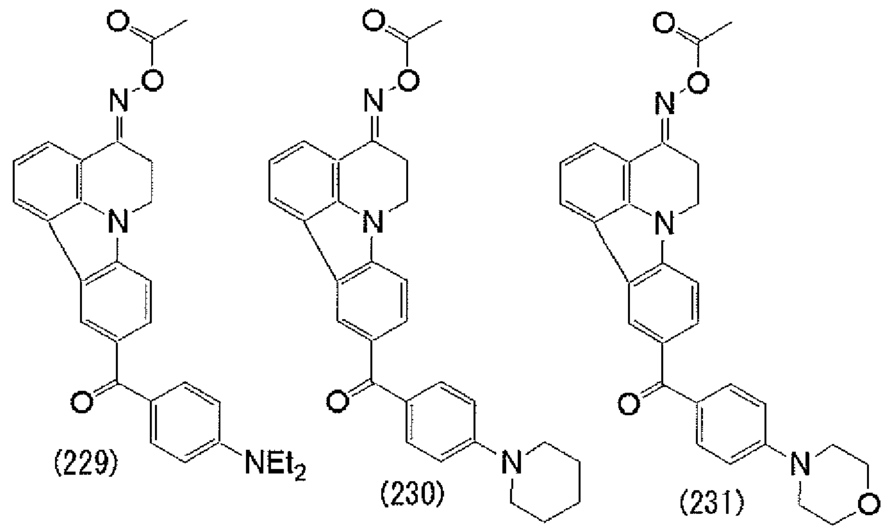

(229) (230) (231)

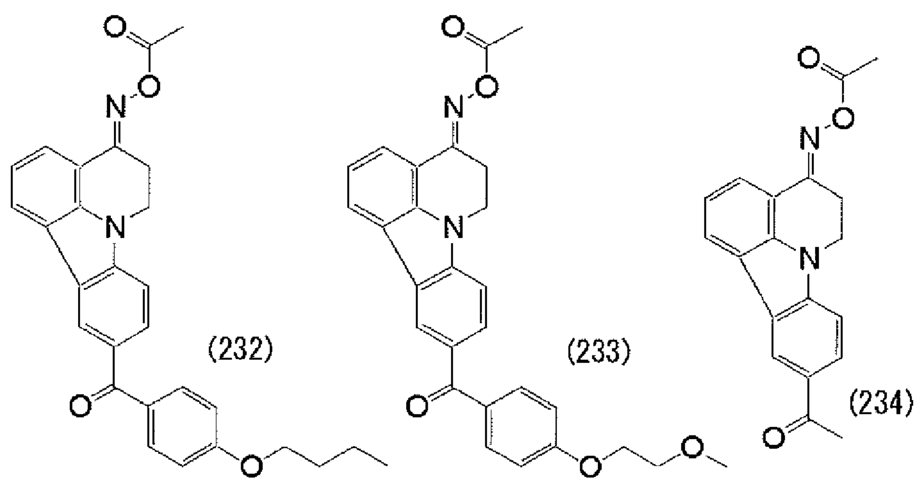

(232) (233) (234)

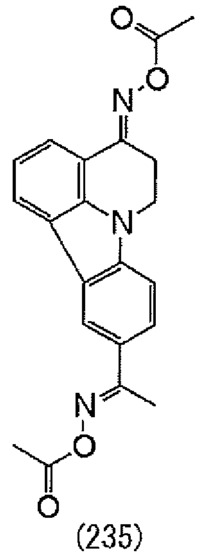
(235)

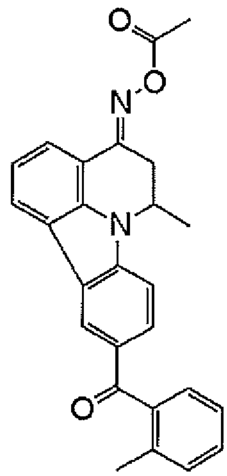
(236)

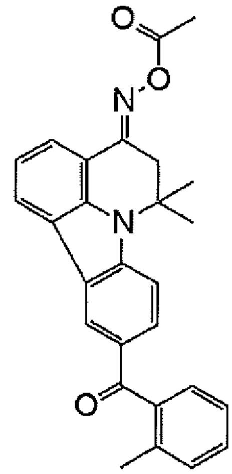
(237)

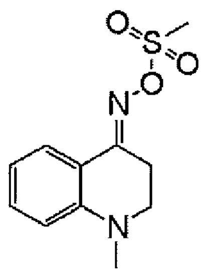
(238)

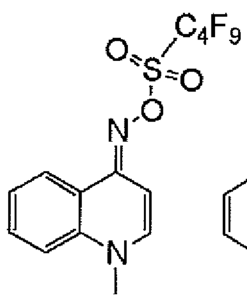
(239)

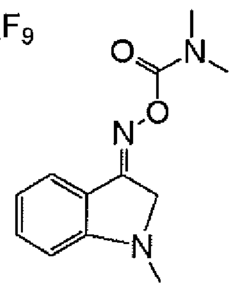
(240)

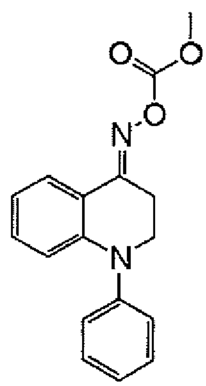
(241)

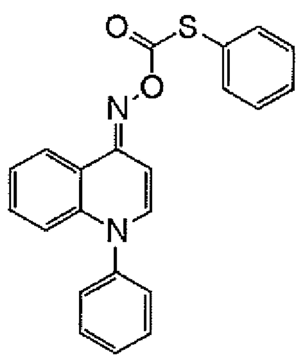
(242)

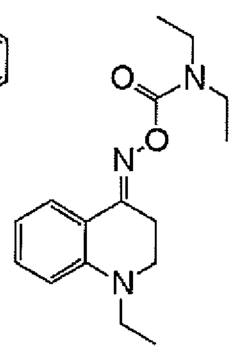
(243)

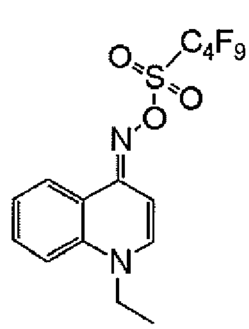
(244)

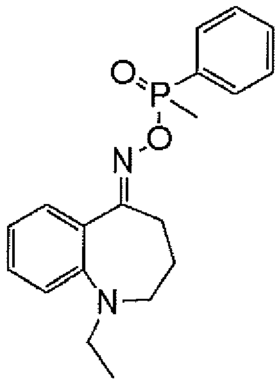

(245)

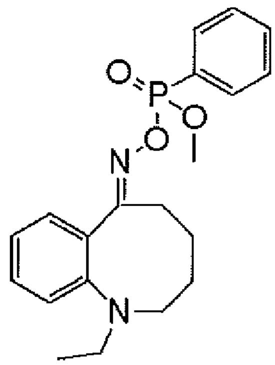

(246)

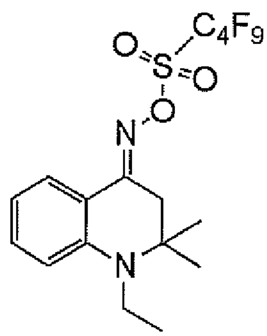

(247)

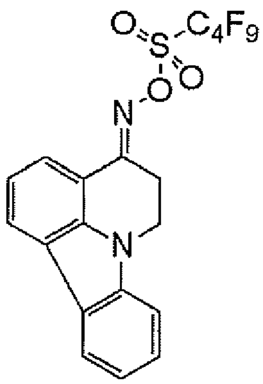

(248)

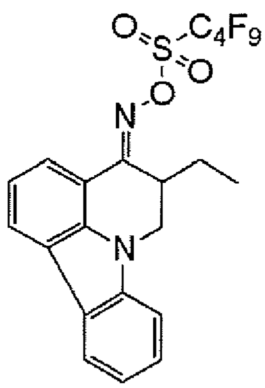

(249)

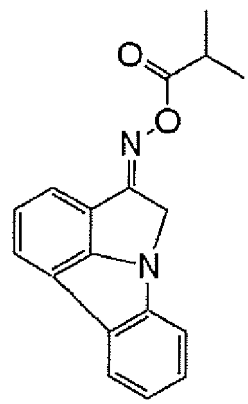

(250)

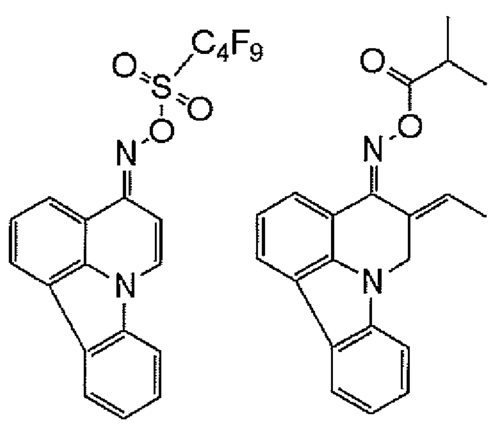

(251)

(252)

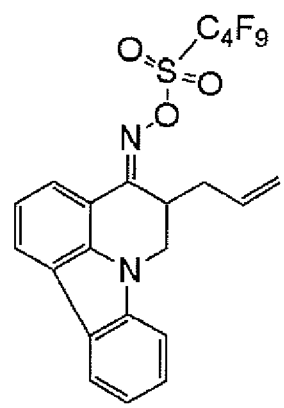

(253)

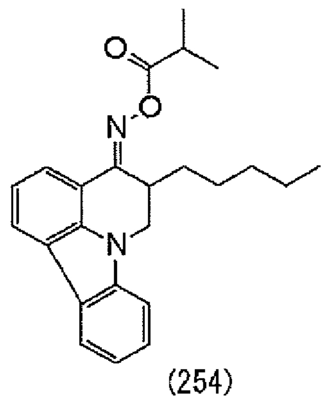
(254)

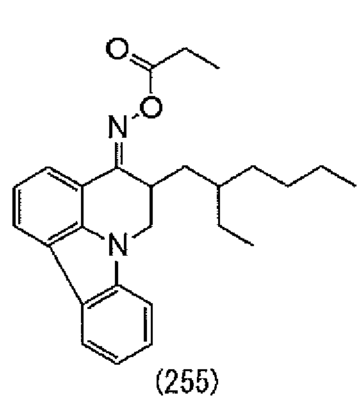
(255)

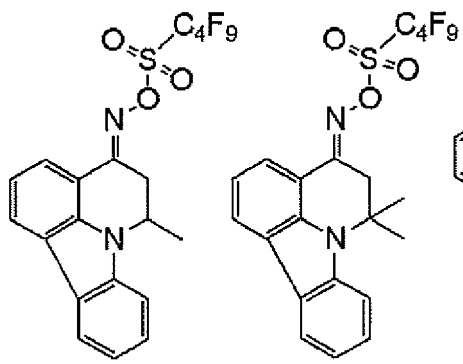
(256) (257)

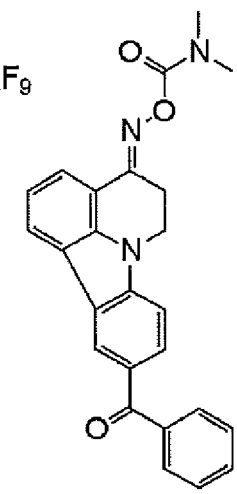
(258)

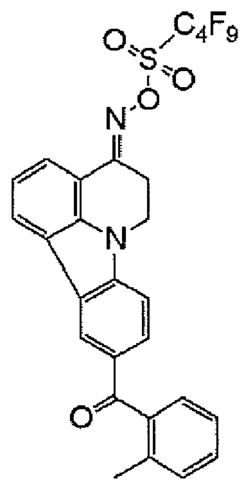
(259)

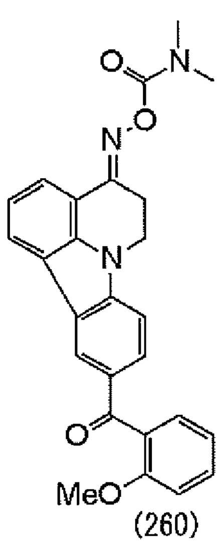
(260)

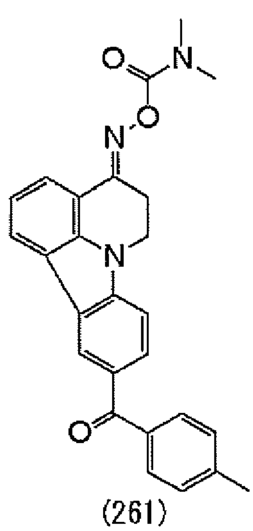
(261)

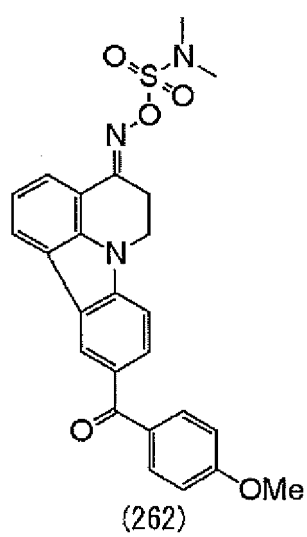
(262)

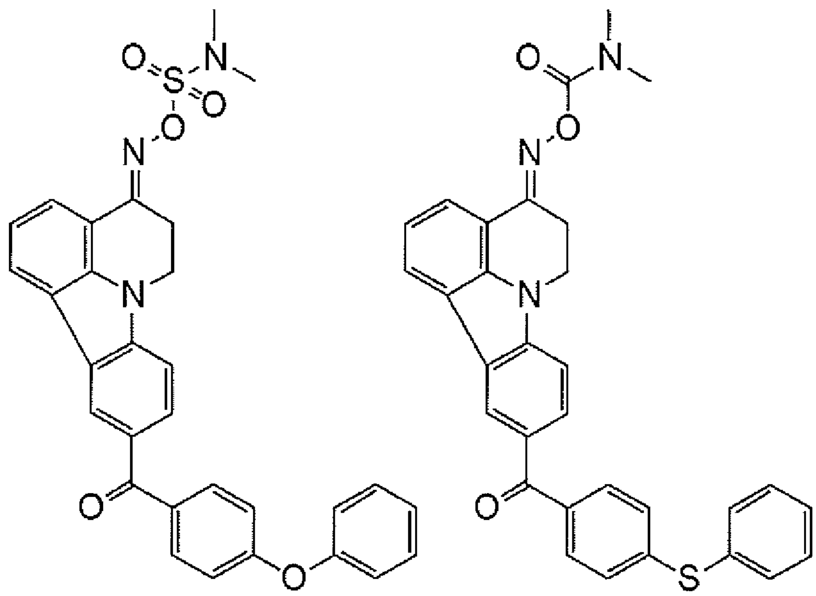

(263) (264)

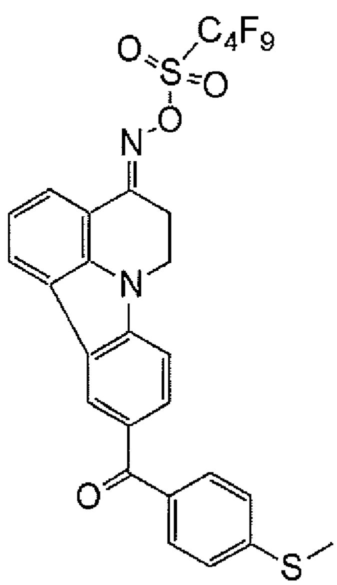

(265)

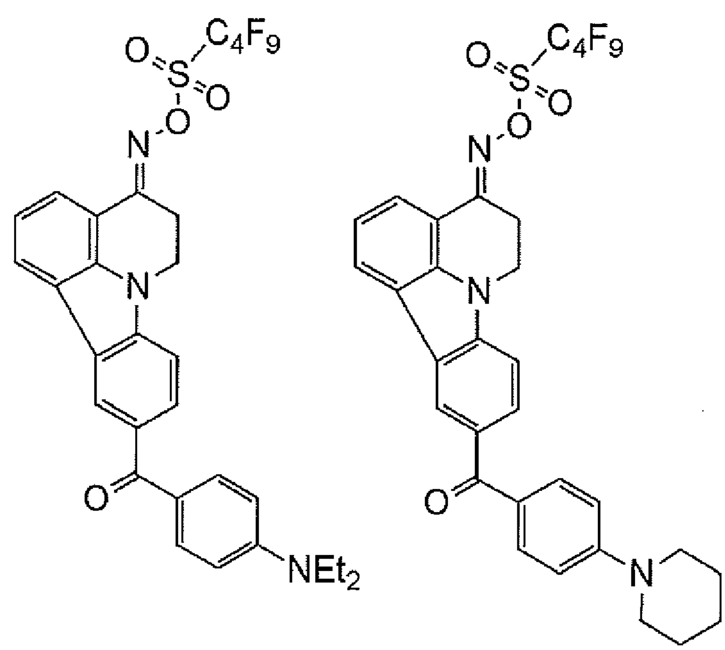

(266) (267)

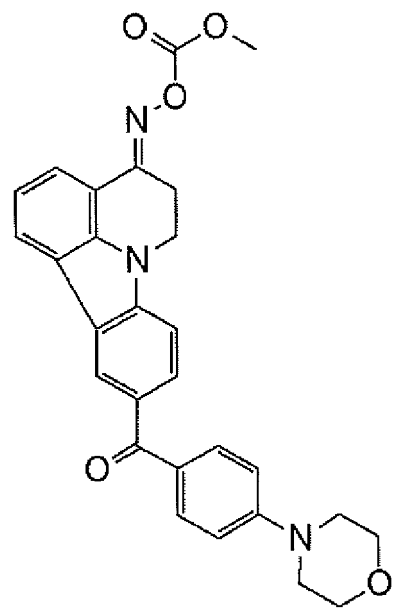

(268)

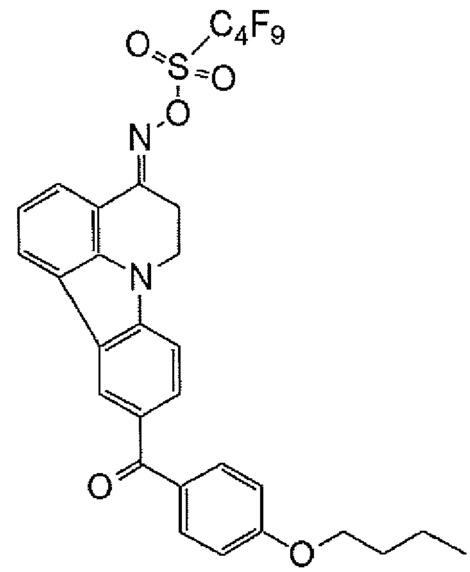

(269)

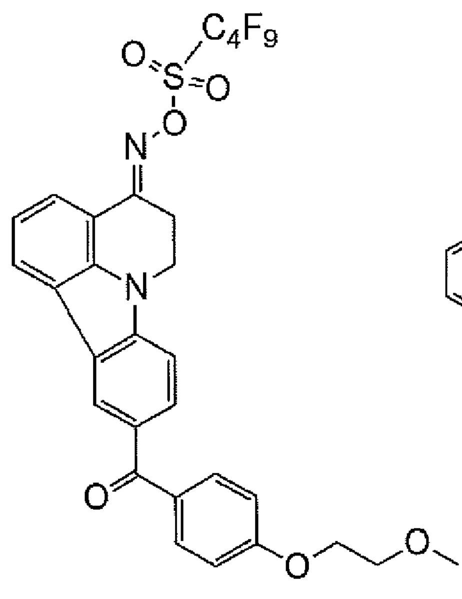

(270)

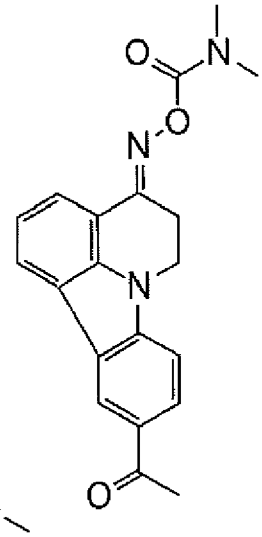

(271)

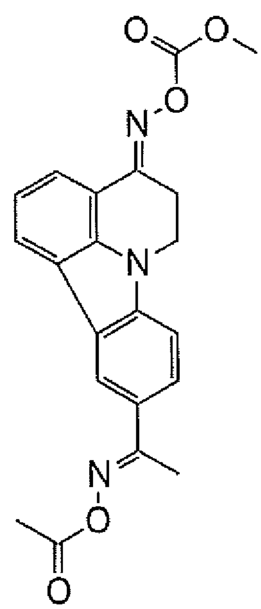

(272)

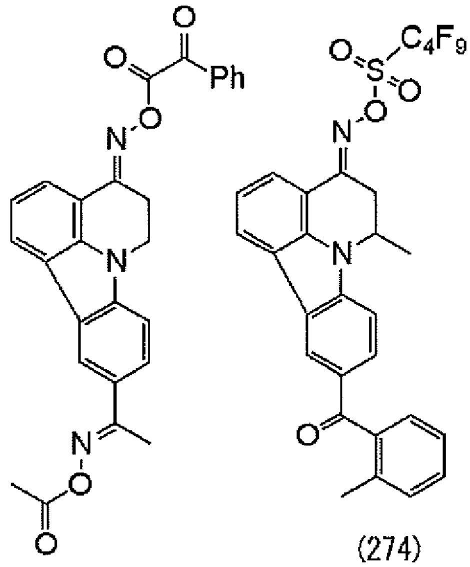

(273) (274)

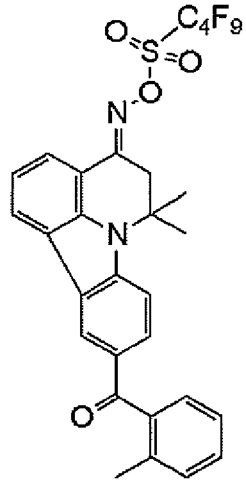

(275)

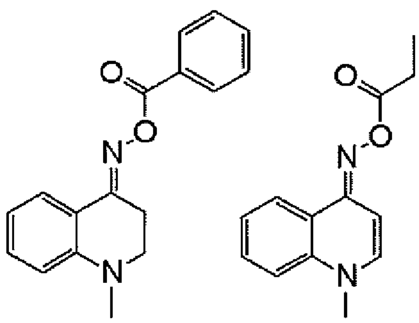

(276) (277)

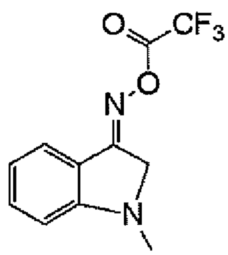

(278)

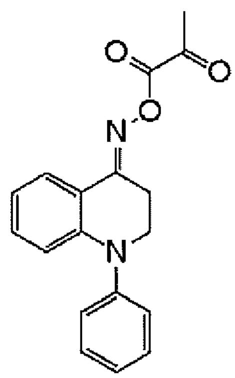

(279)

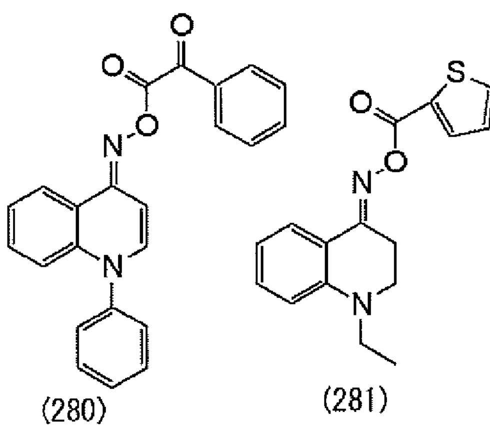

(280) (281)

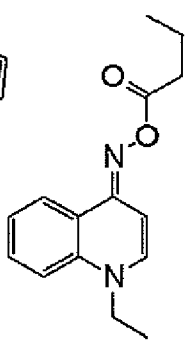

(282)

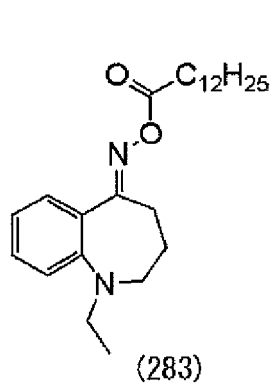

(283)

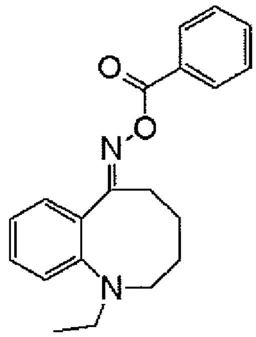
(284)

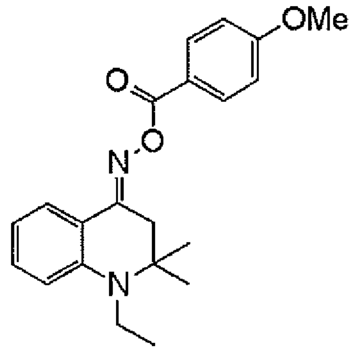
(285)

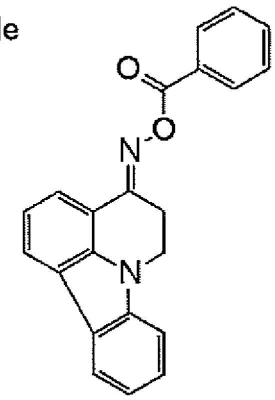
(286)

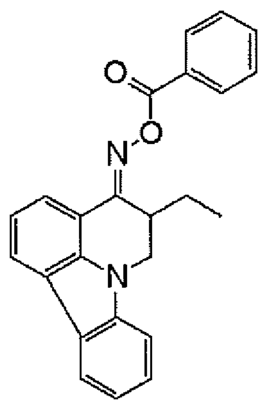
(287)

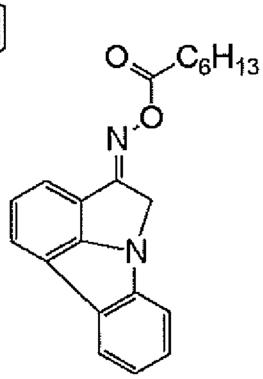
(288)

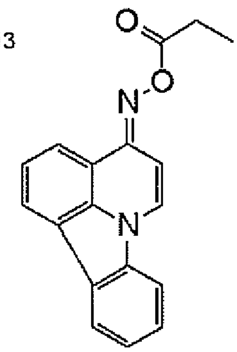
(289)

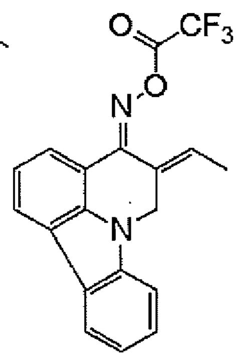
(290)

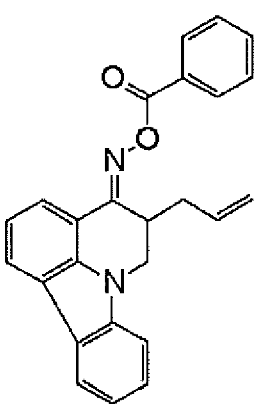
(291)

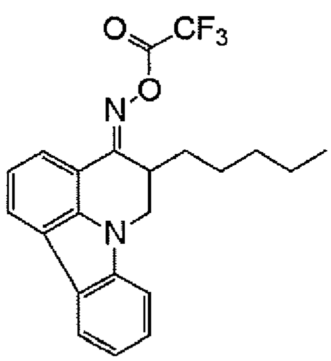
(292)

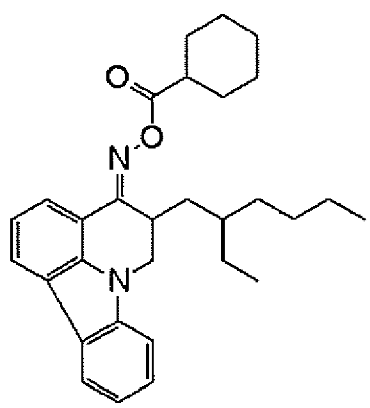
(293)

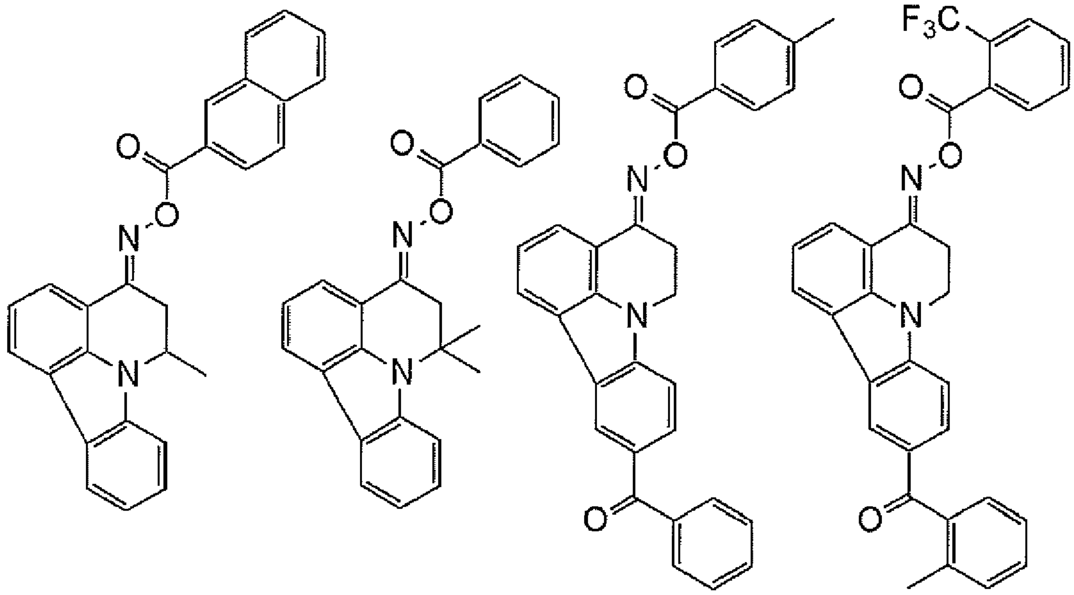

(294) (295) (296) (297)

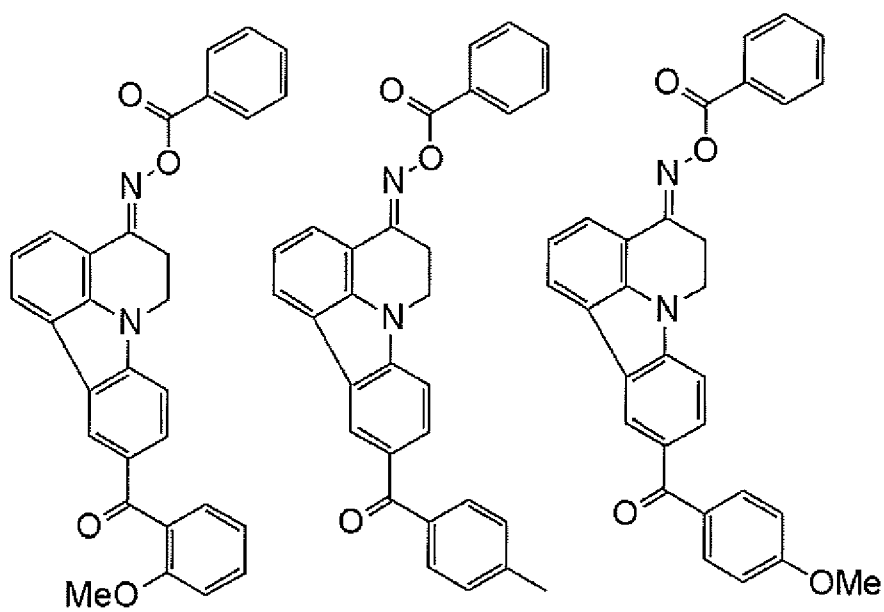

(298) (299) (300)

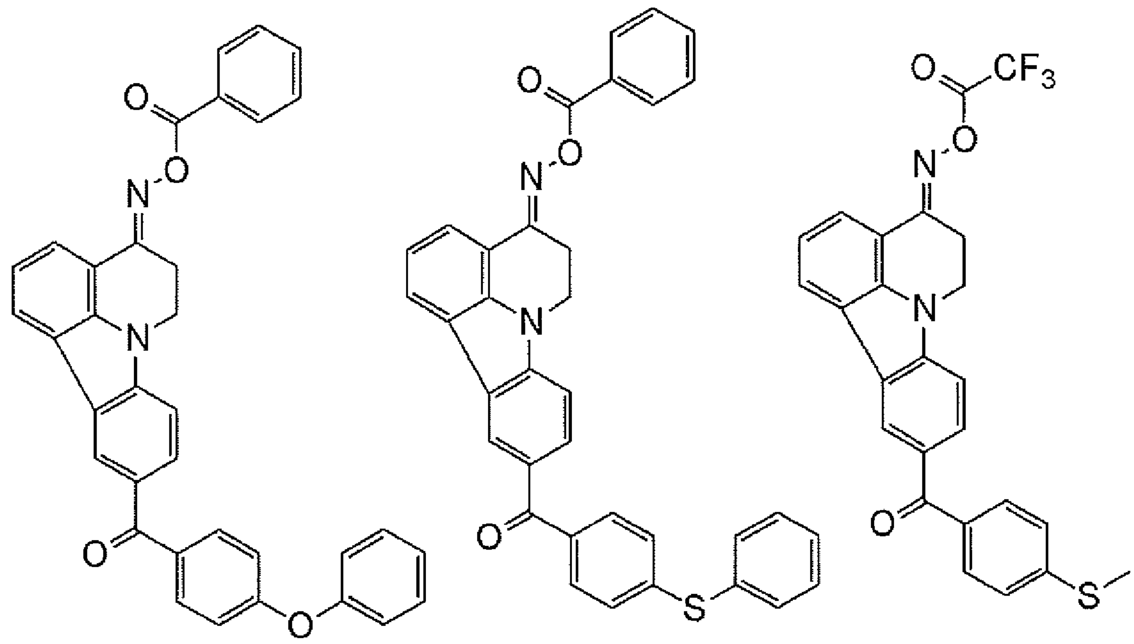

(301) (302) (303)

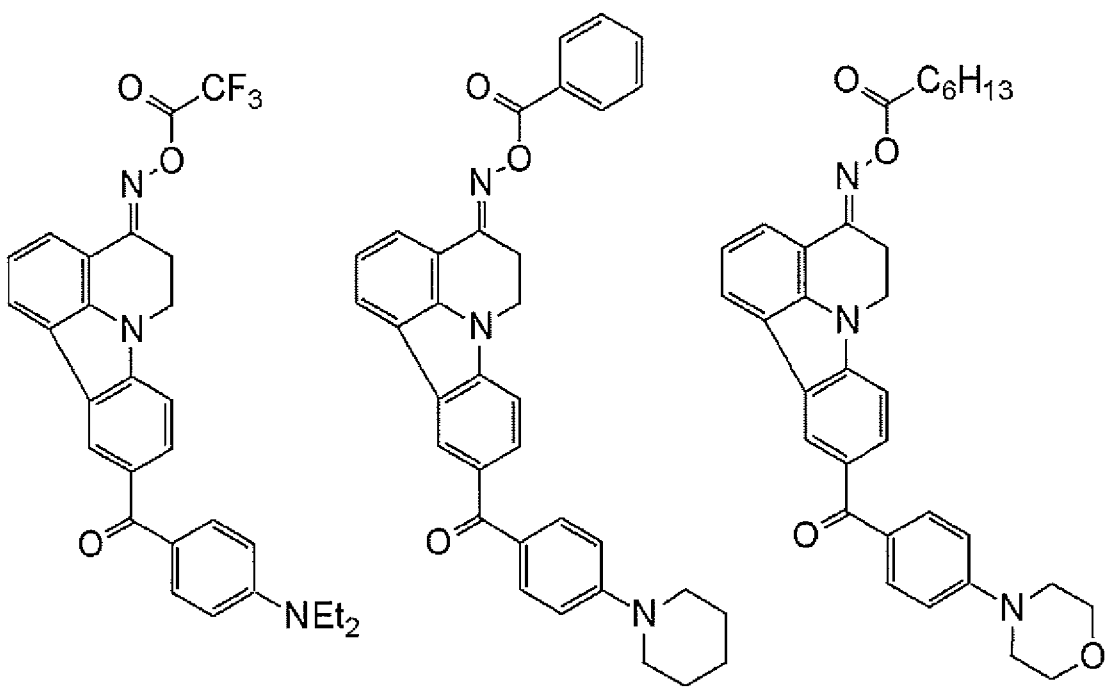

(304) (305) (306)

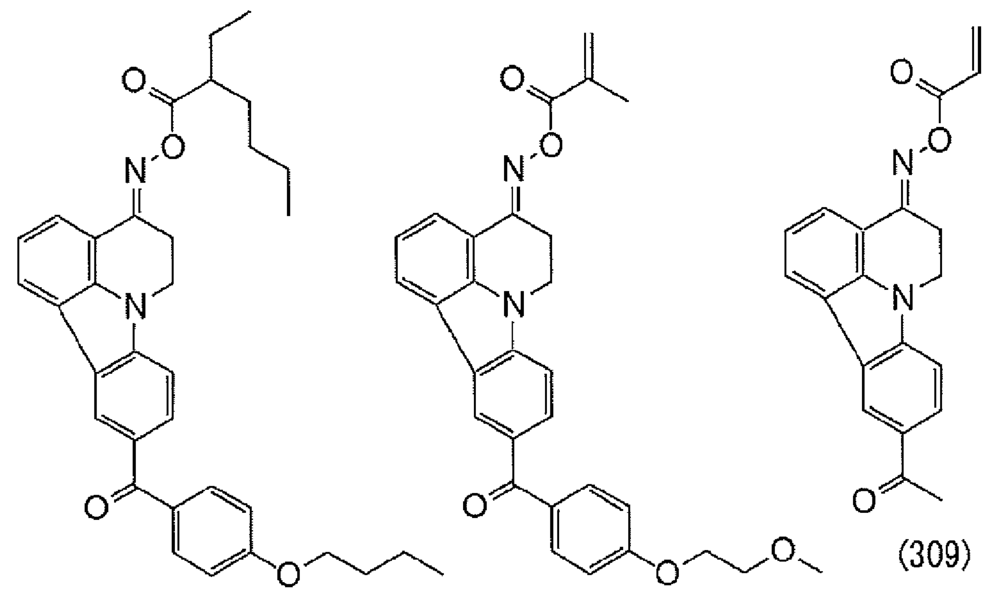

(307) (308) (309)

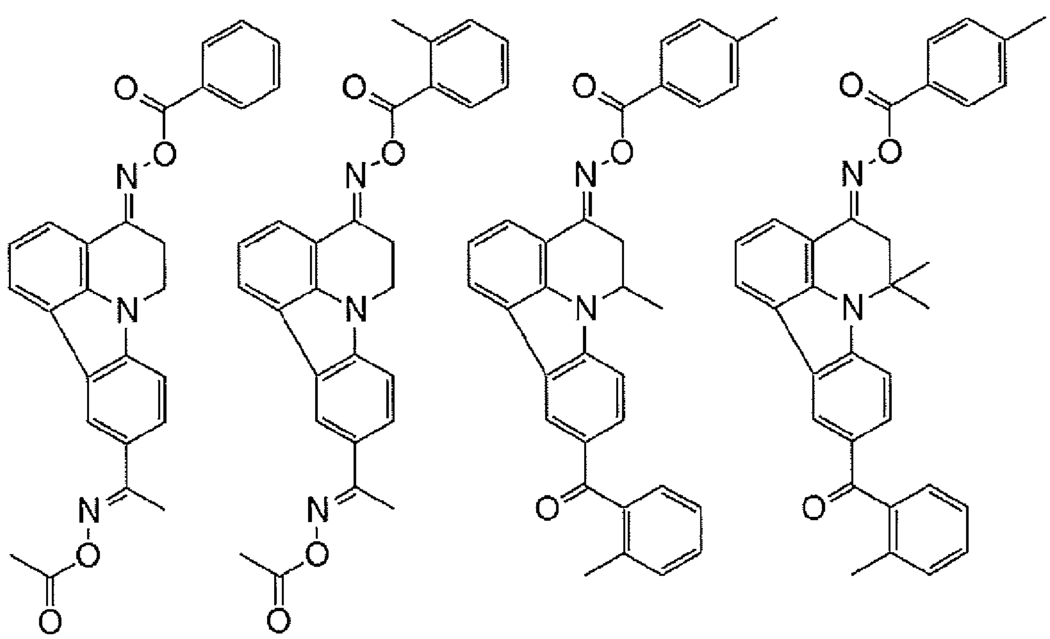

(310) (311) (312) (313)

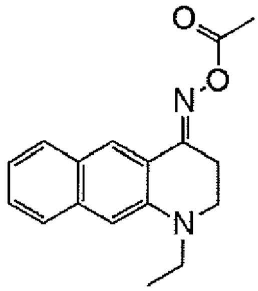

(314)

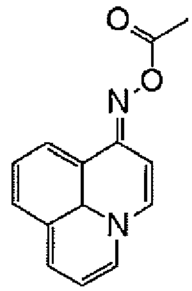

(315)

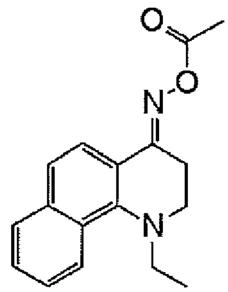

(316)

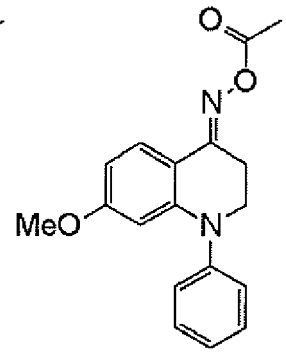

(317)

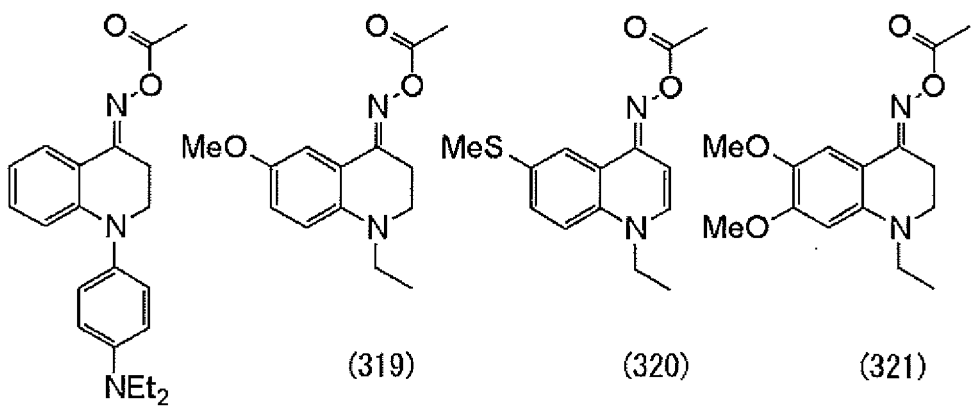

(319) (320) (321)

(318)

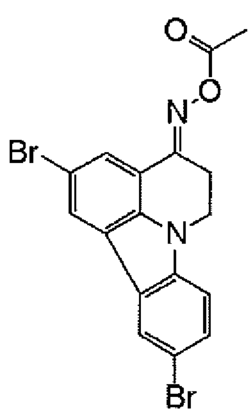

(322)

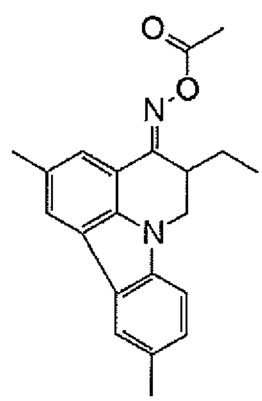

(323)

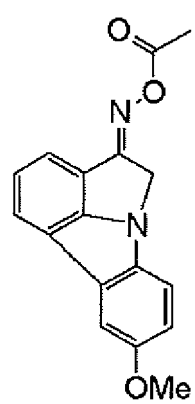

(324)

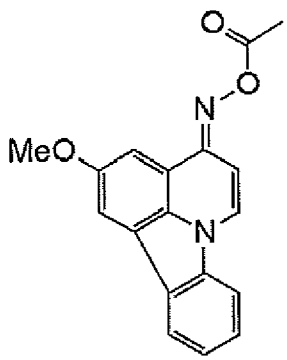
(325)

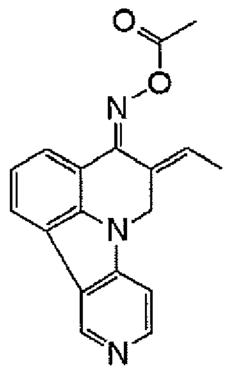
(326)

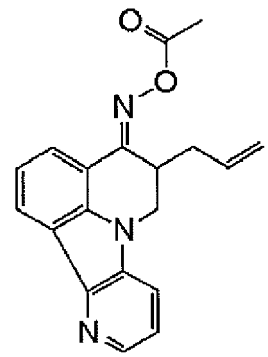
(327)

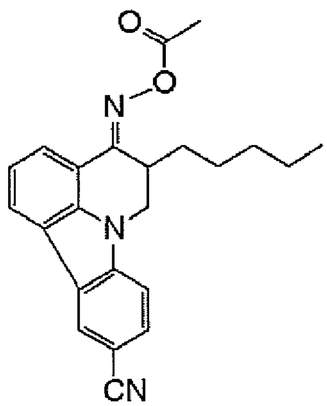
(328)

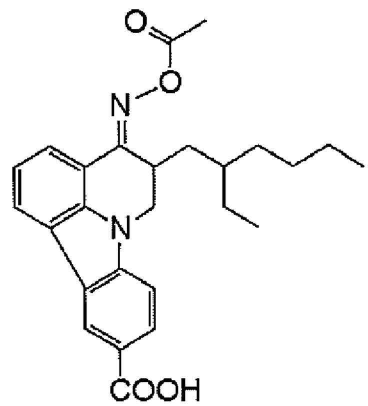
(329)

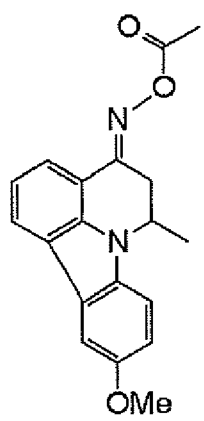
(330)

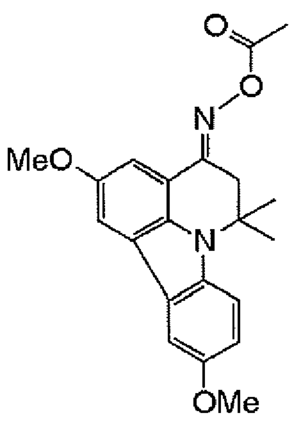
(331)

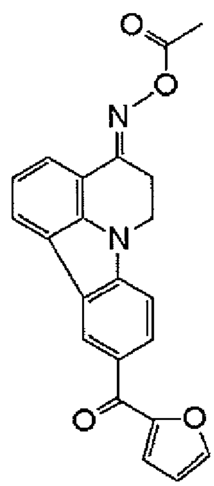
(332)

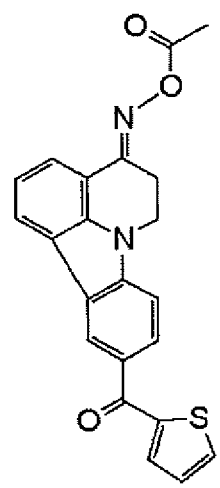
(333)

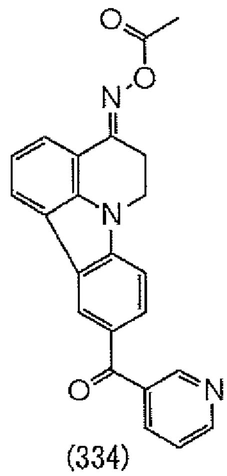

(334)

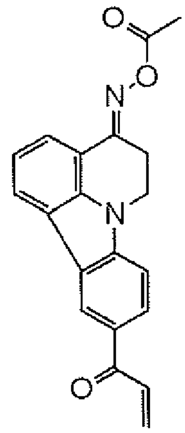

(335)

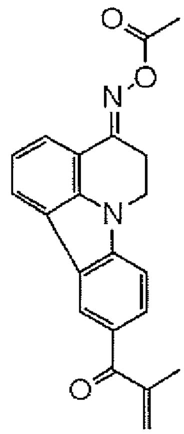

(336)

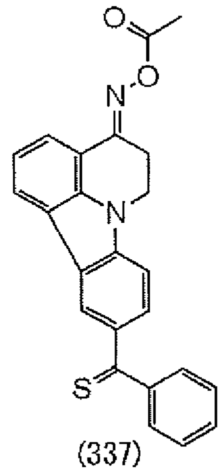

(337)

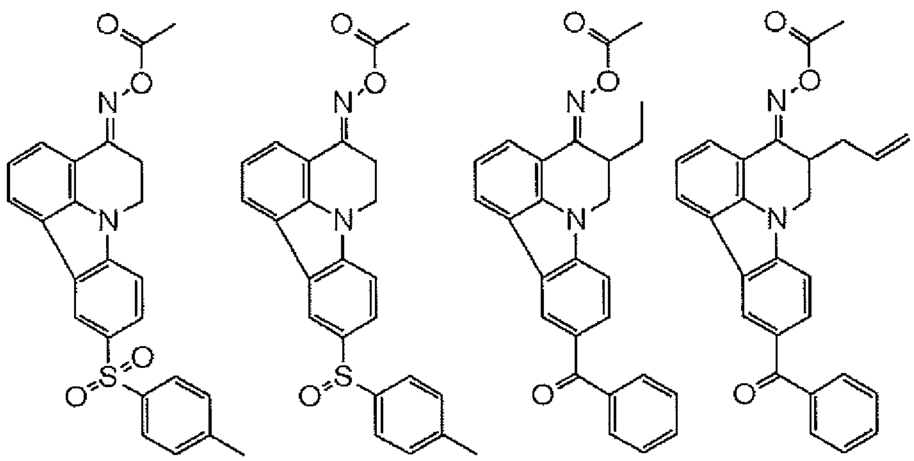

(338) (339) (340) (341)

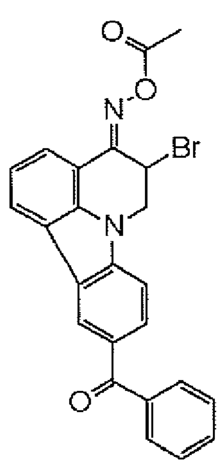

(342)

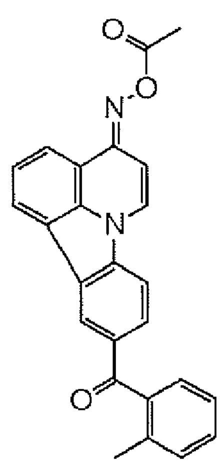

(343)

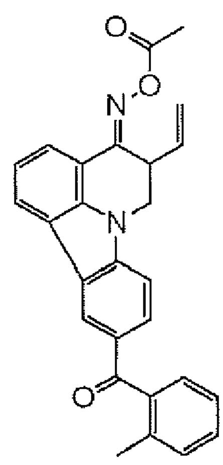

(344)

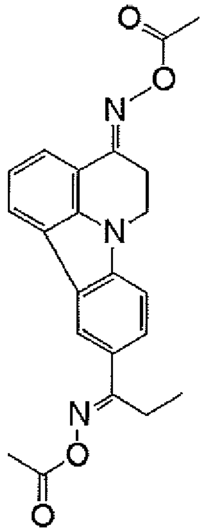

(345)

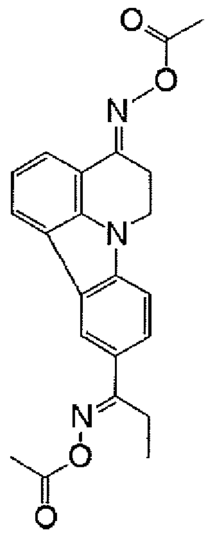

(346)

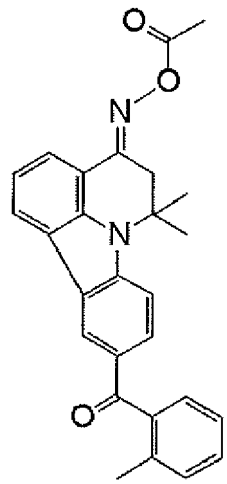

(347)

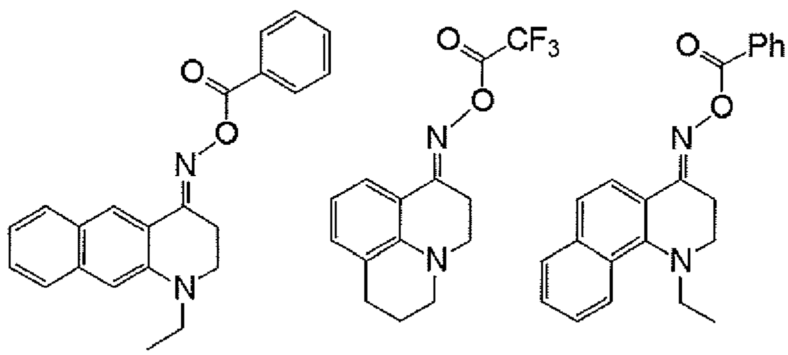

(348) (349) (350)

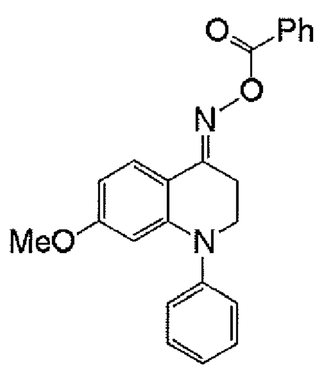

(351)

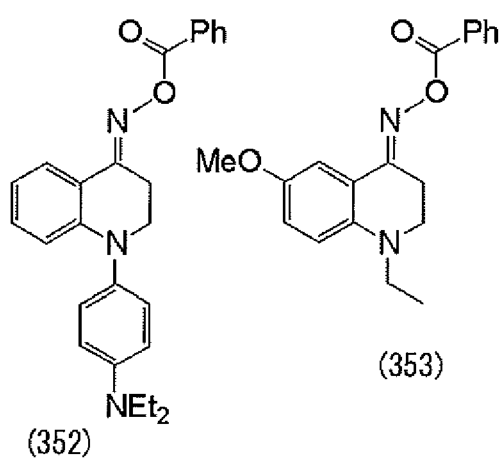

(352) (353)

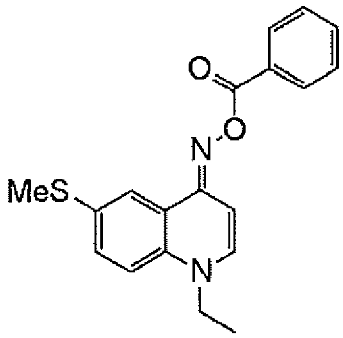

(354)

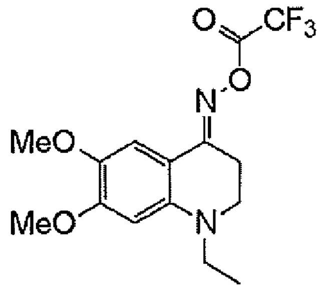

(355)

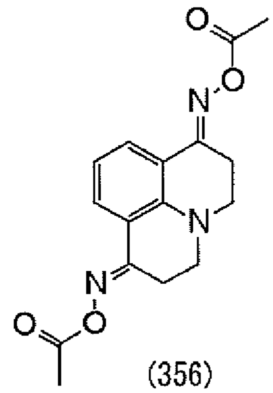

(356)

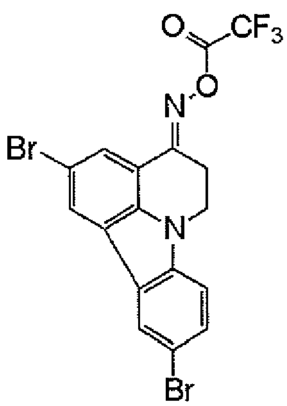

(357)

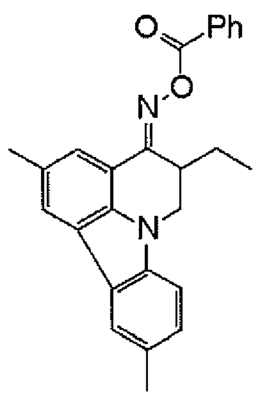

(358)

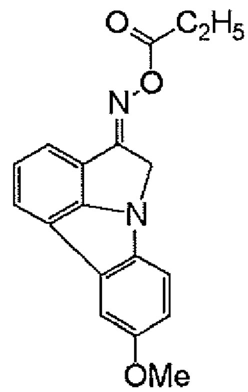

(359)

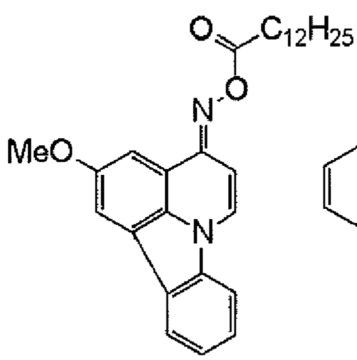

(360)

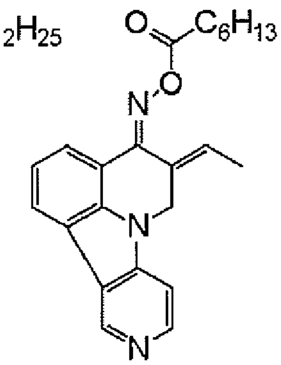

(361)

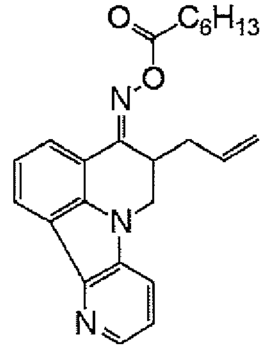

(362)

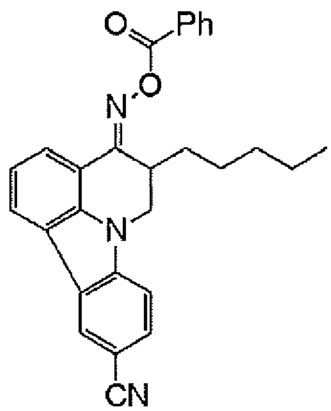

(363)

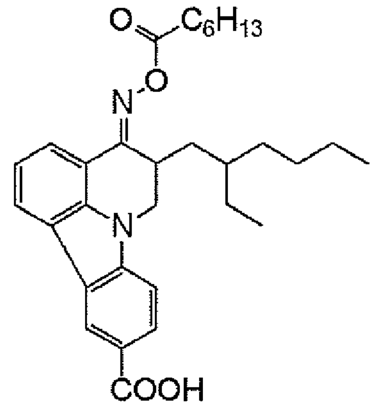

(364)

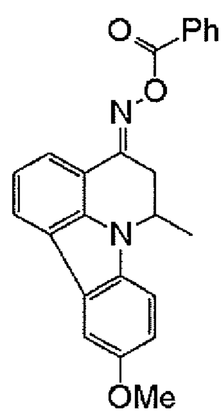

(365)

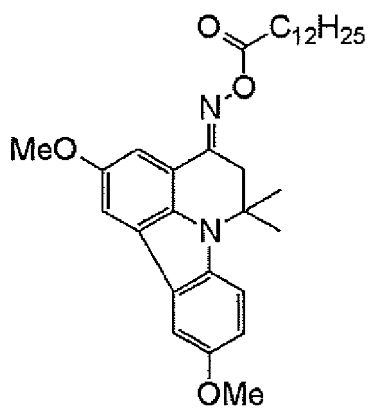

(366)

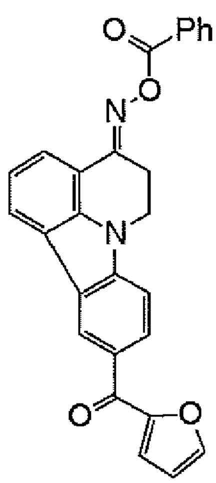

(367)

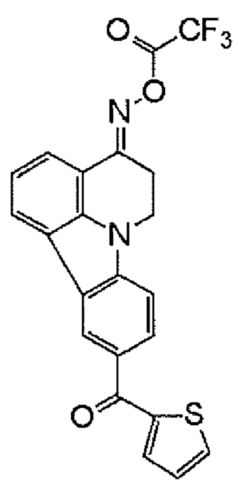

(368)

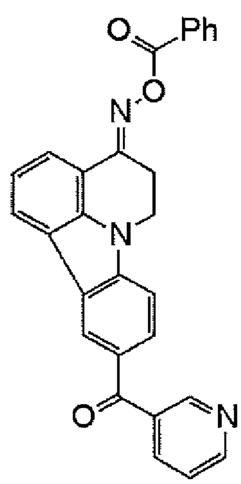

(369)

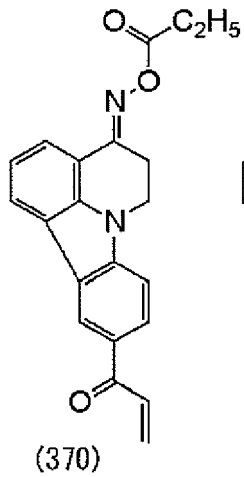
(370)

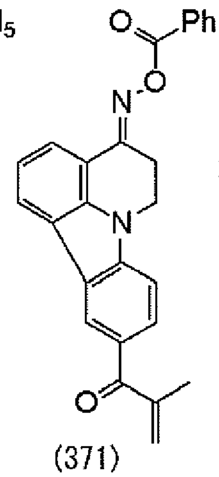
(371)

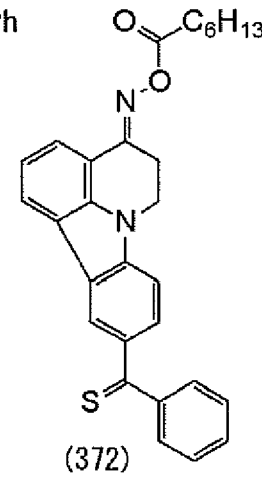
(372)

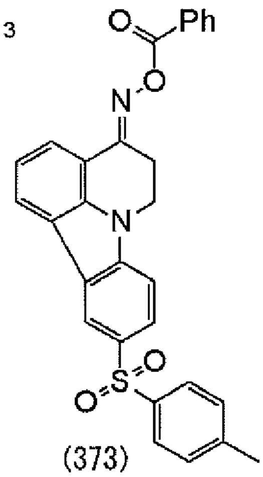
(373)

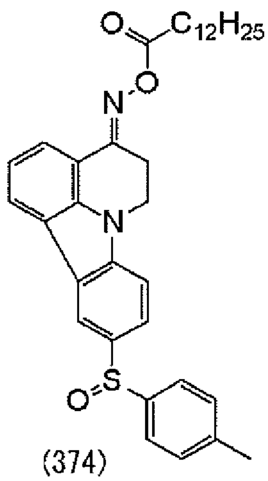
(374)

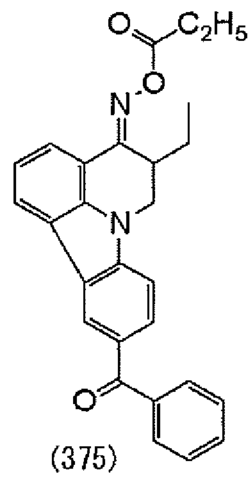
(375)

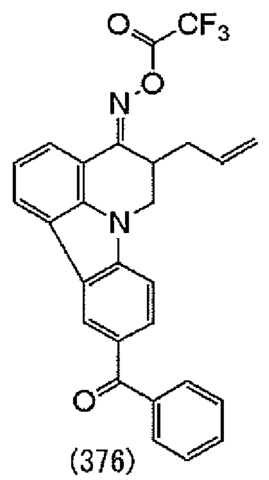
(376)

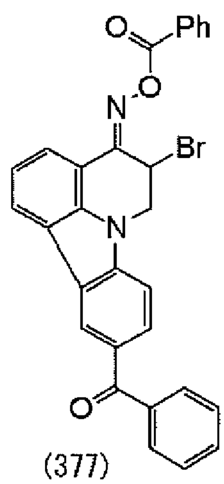
(377)

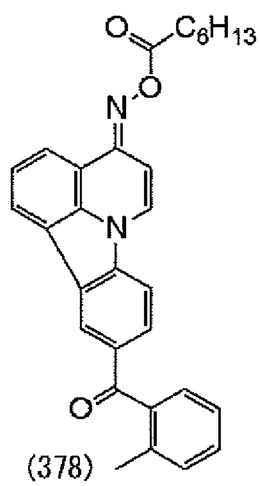
(378)

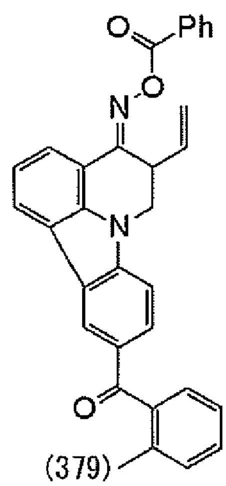
(379)

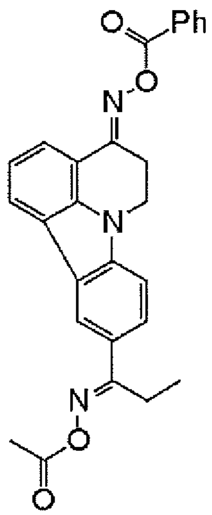

(380)

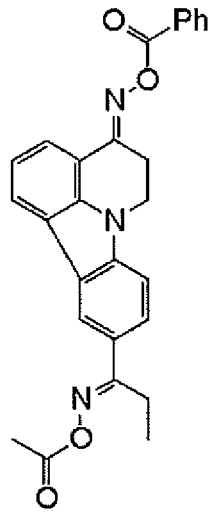

(381)

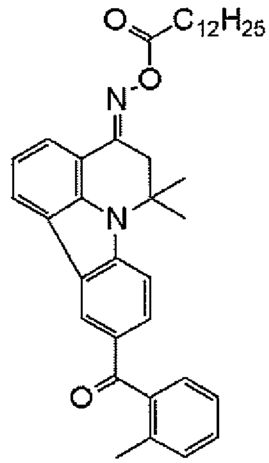

(382)

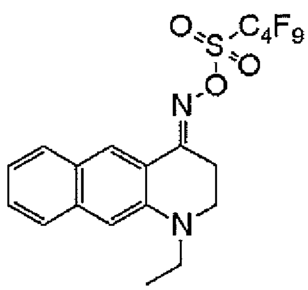

(383)

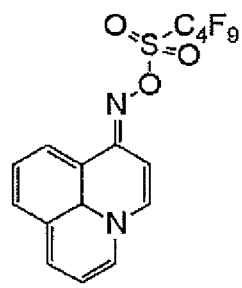

(384)

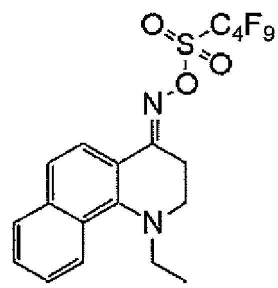

(385)

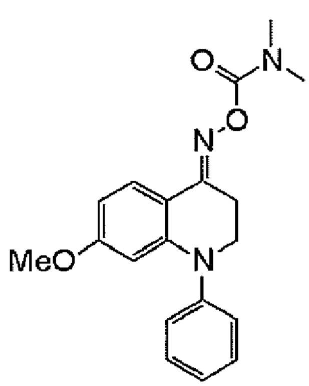

(386)

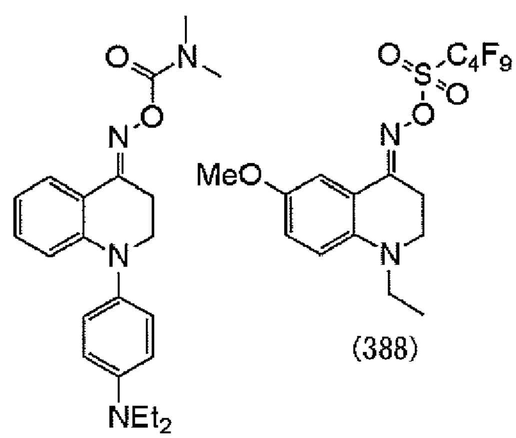

(387)

(388)

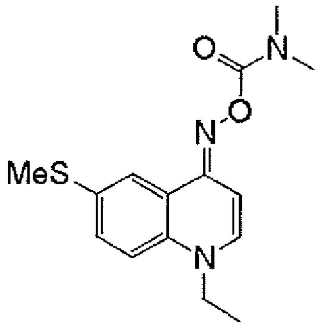

(389)

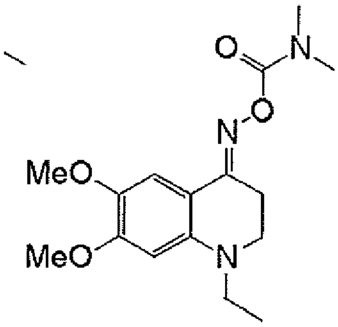

(390)

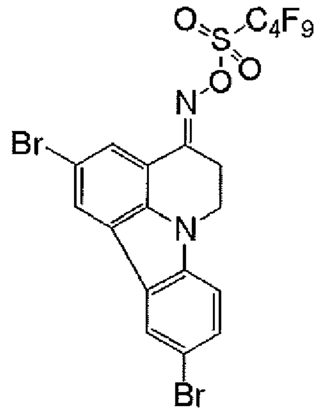

(391)

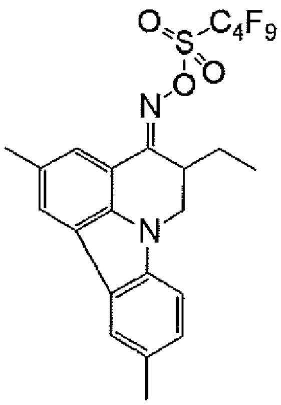

(392)

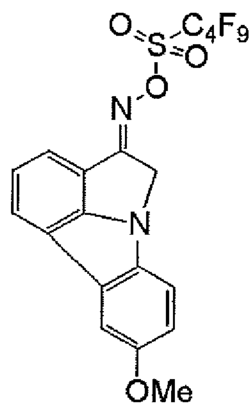

(393)

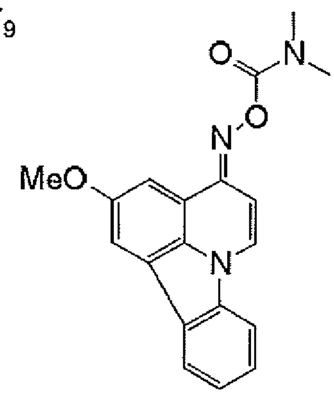

(394)

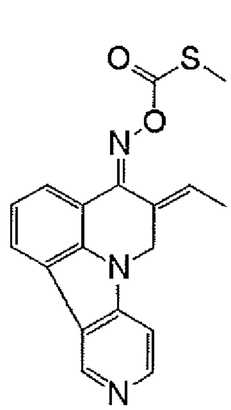

(395)

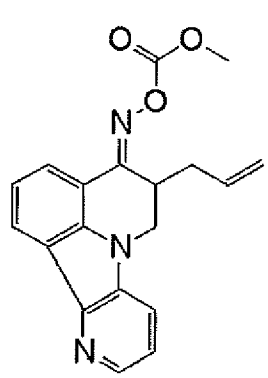

(396)

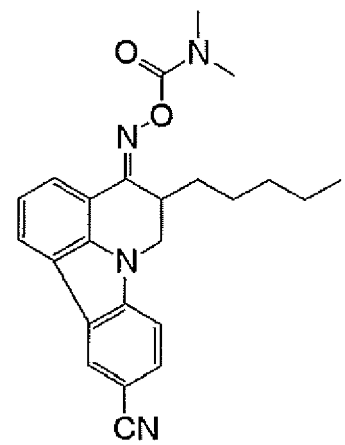

(397)

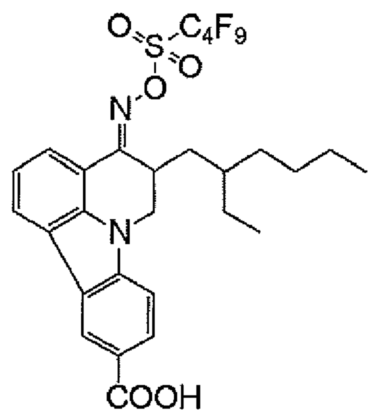

(398)

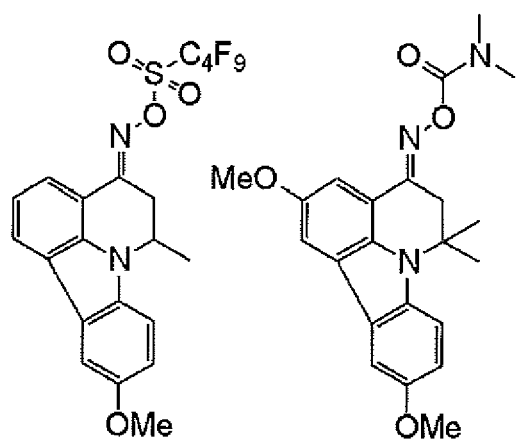

(399)

(400)

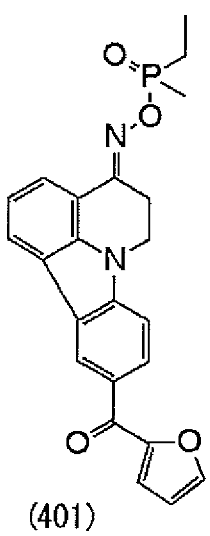

(401)

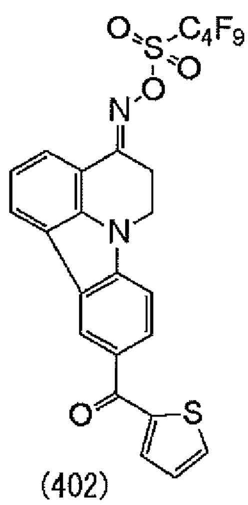

(402)

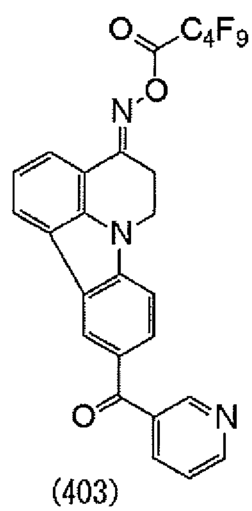

(403)

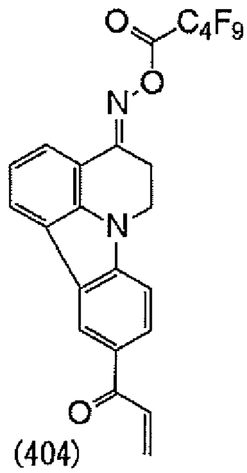
(404)

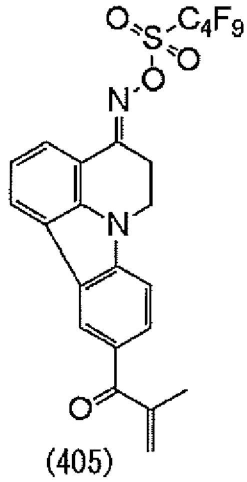
(405)

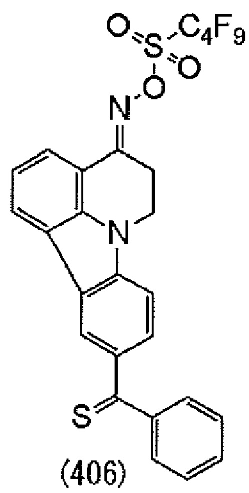
(406)

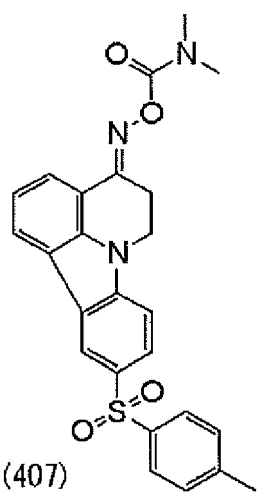
(407)

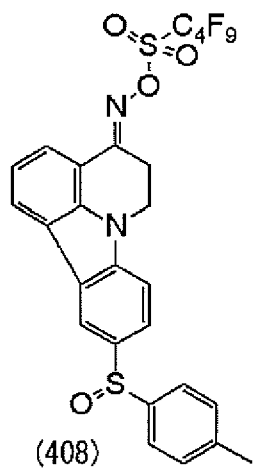
(408)

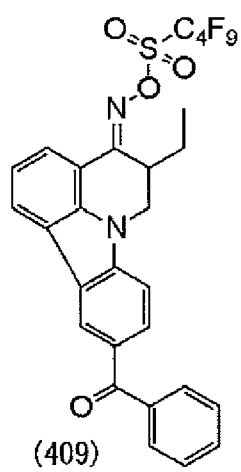
(409)

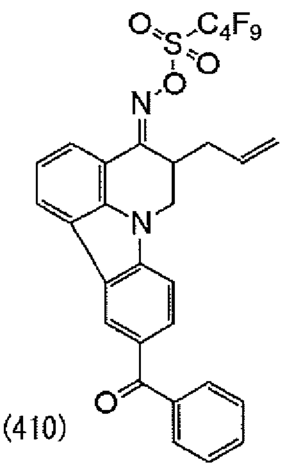
(410)

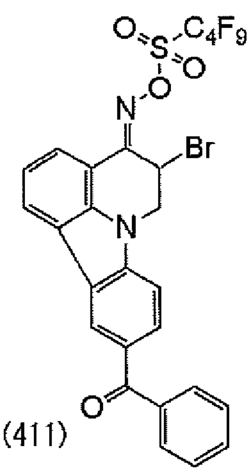
(411)

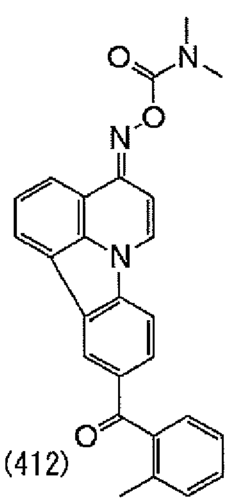
(412)

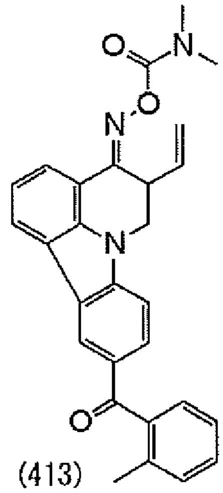
(413)

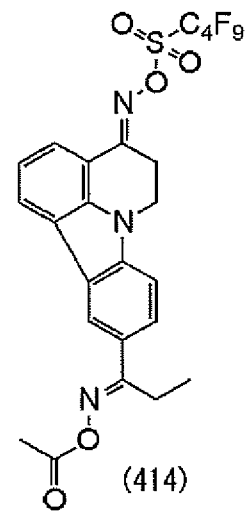
(414)

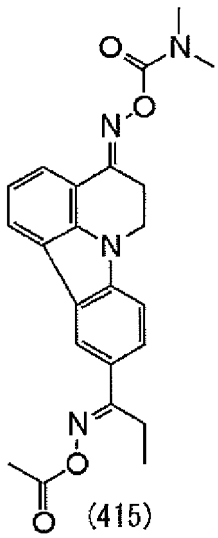
(415)

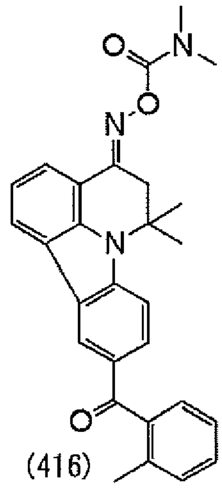
(416)

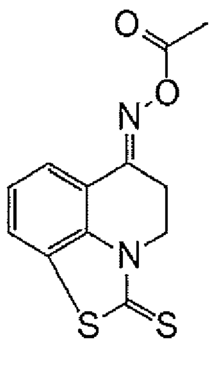
(417)

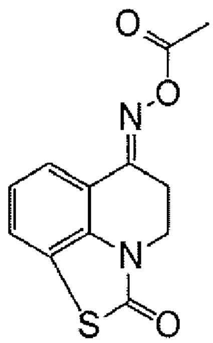
(418)

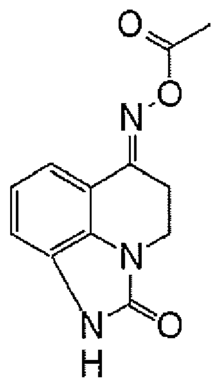
(419)

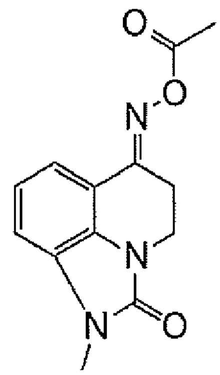
(420)

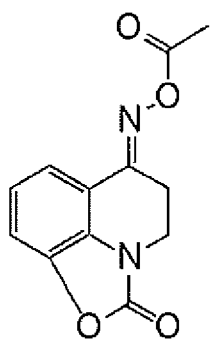
(421)

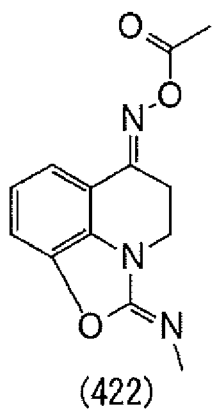
(422)

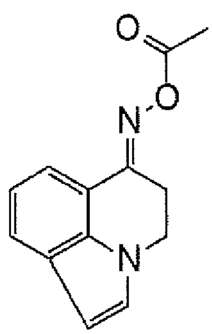
(423)

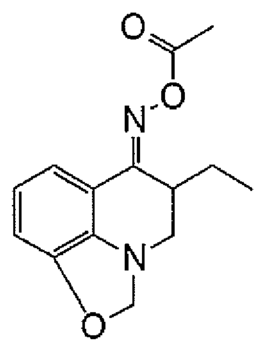
(424)

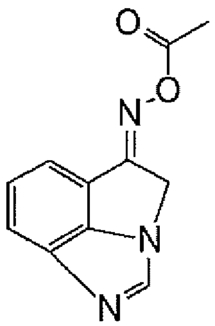

(425)

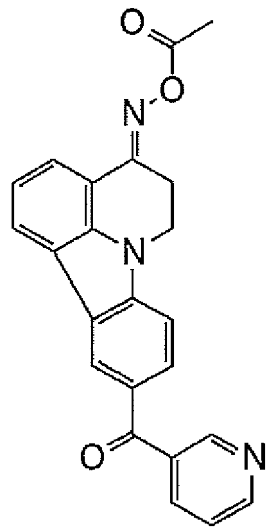

(426)

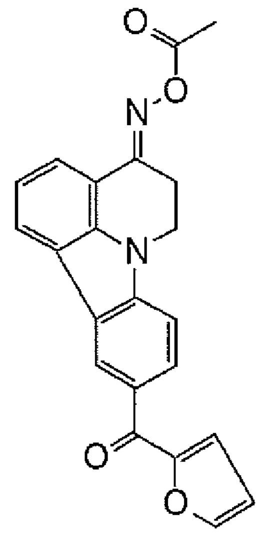

(427)

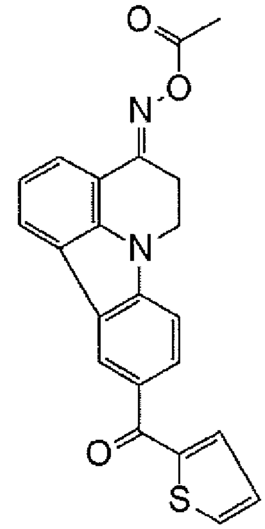

(428)

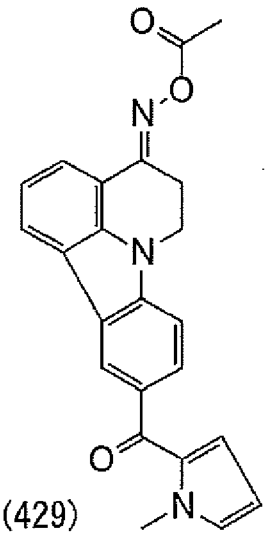

(429)

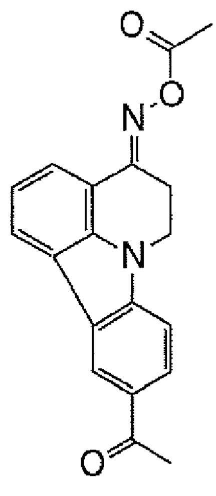

(430)

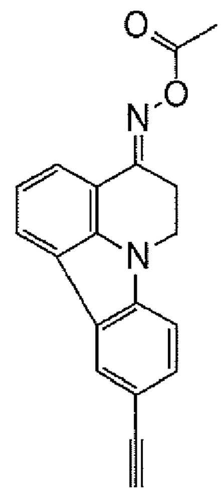

(431)

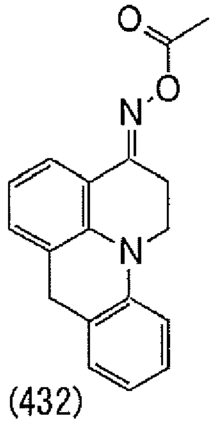

(432)

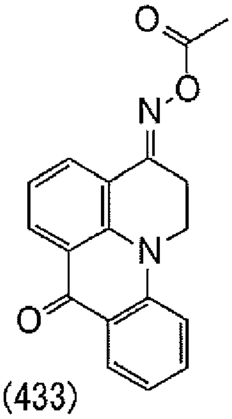

(433)

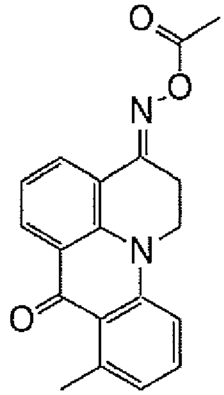

(434)

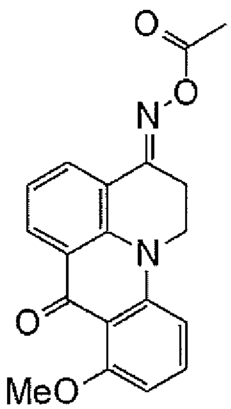

(435)

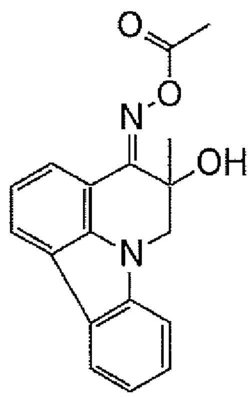

(436)

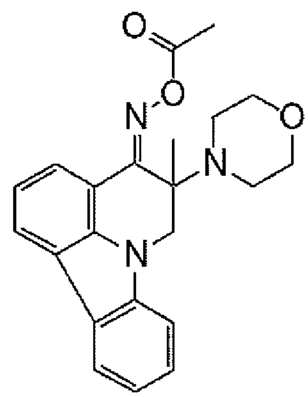

(437)

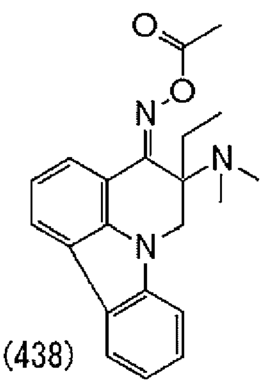

(438)

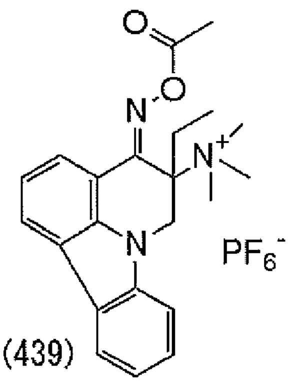

(439)

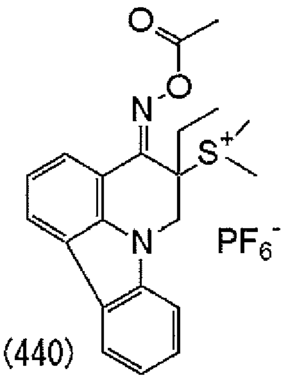

(440)

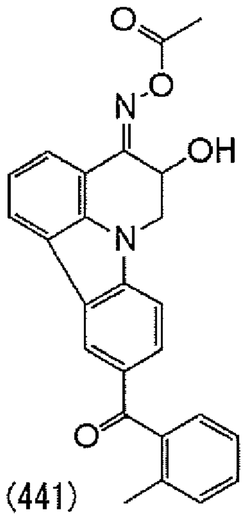

(441)

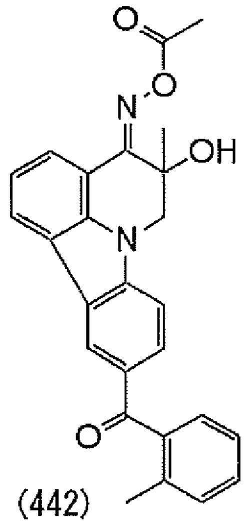

(442)

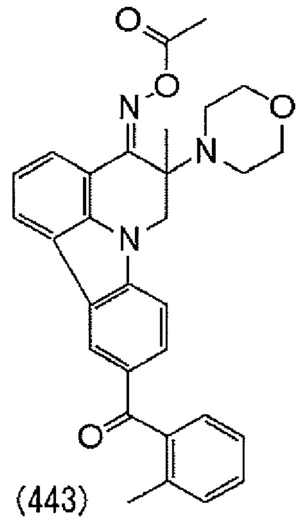

(443)

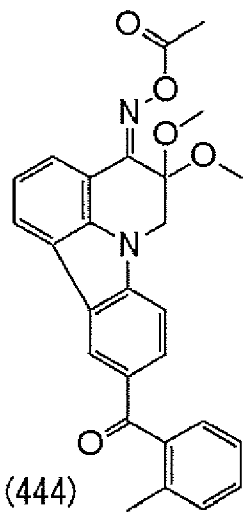

(444)

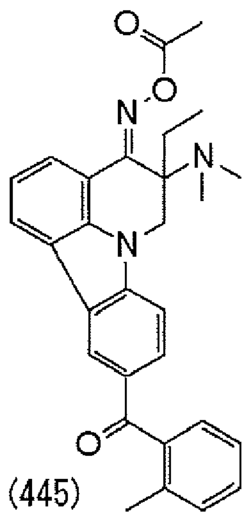

(445)

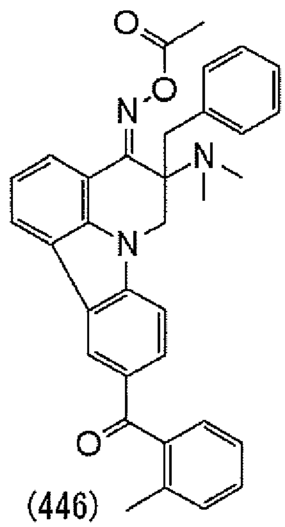

(446)

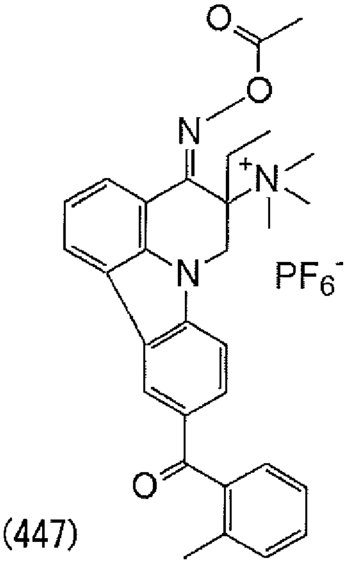
(447)

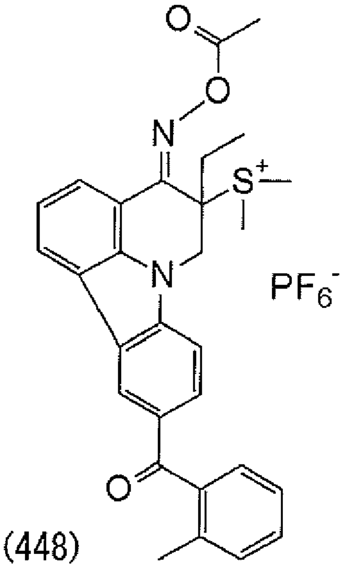
(448)

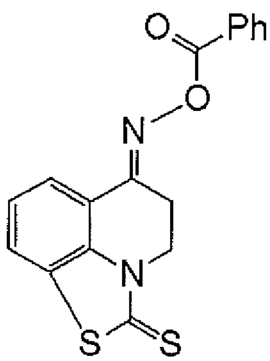
(449)

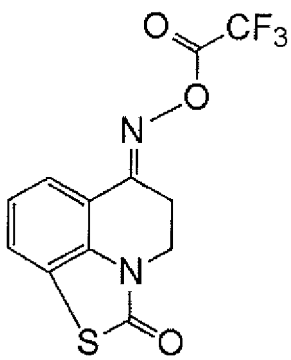
(450)

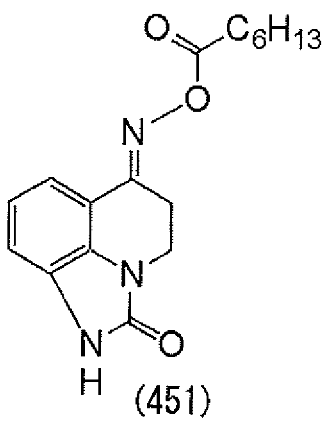
(451)

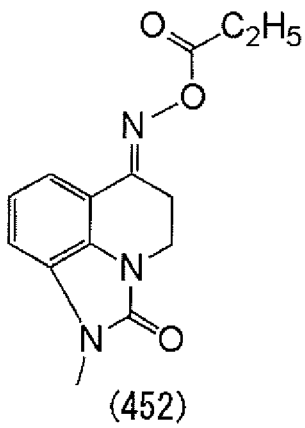
(452)

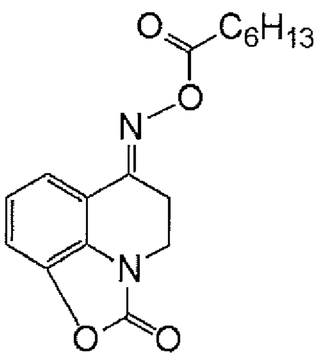
(453)

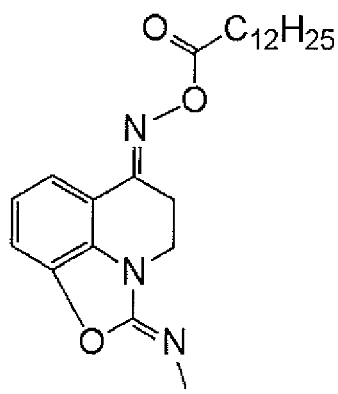
(454)

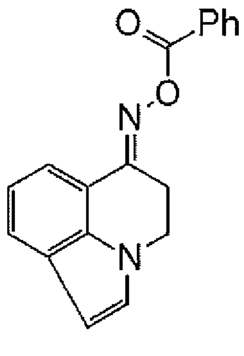
(455)

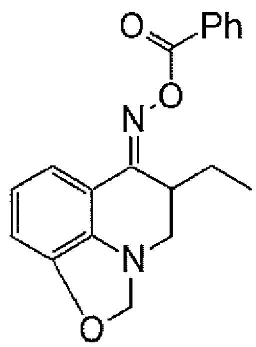
(456)

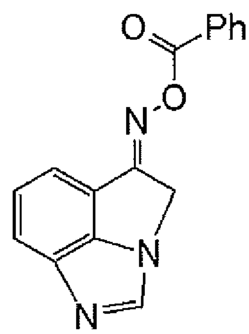
(457)

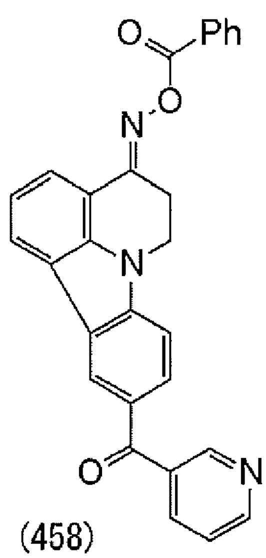
(458)

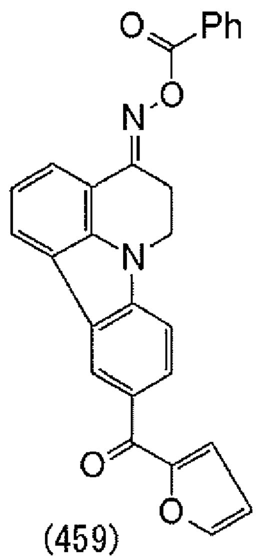
(459)

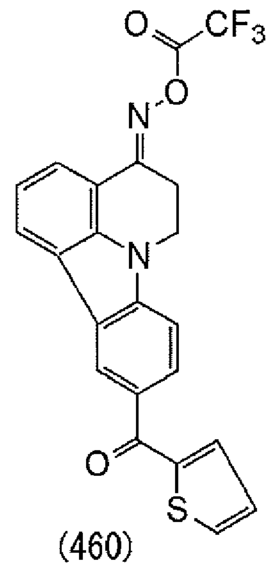
(460)

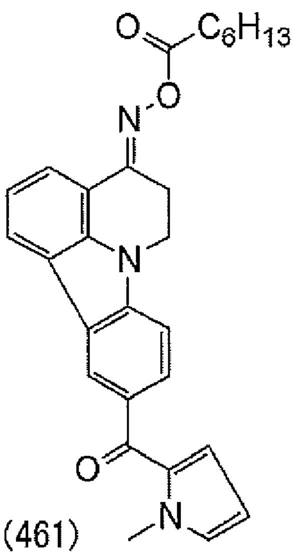
(461)

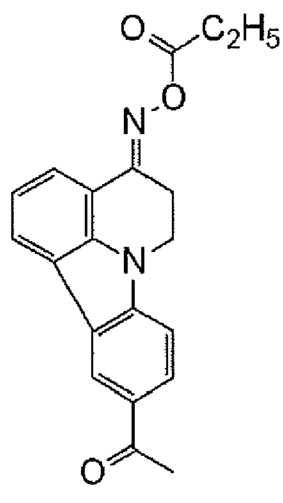
(462)

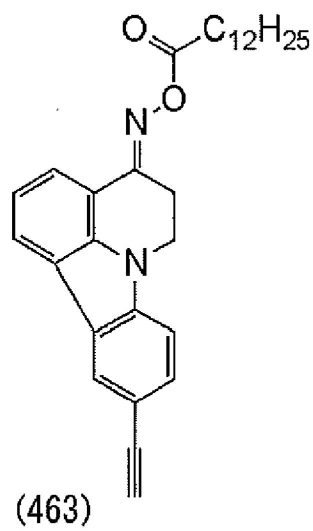
(463)

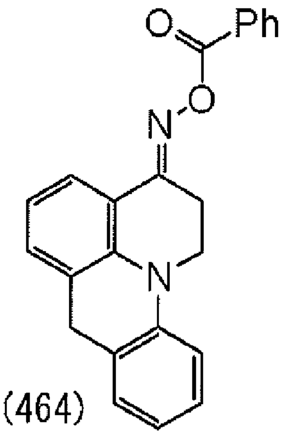
(464)

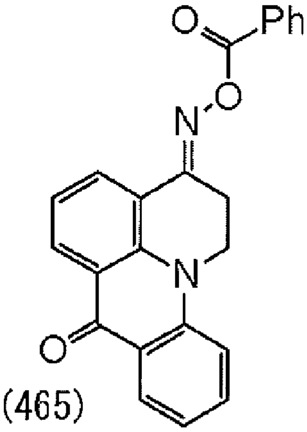
(465)

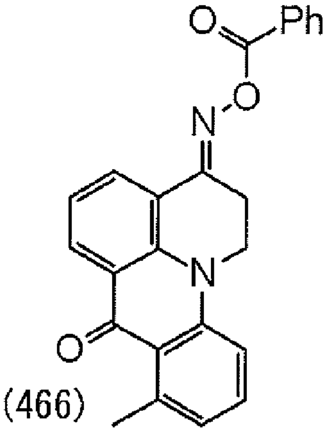
(466)

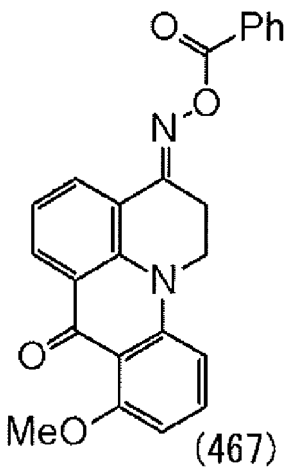
(467)

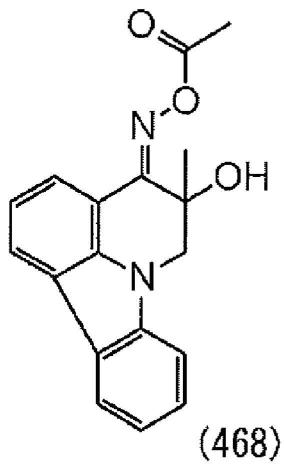
(468)

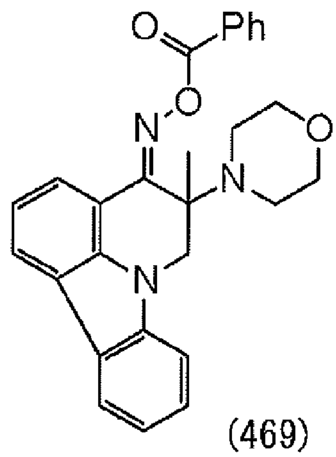
(469)

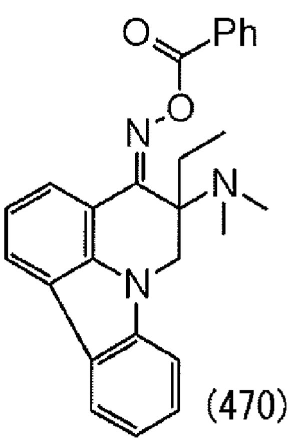
(470)

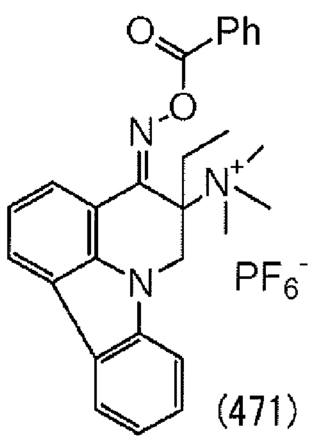
(471)

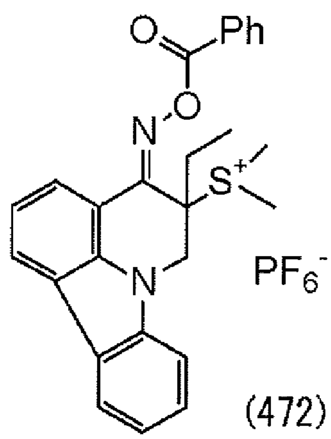
(472)

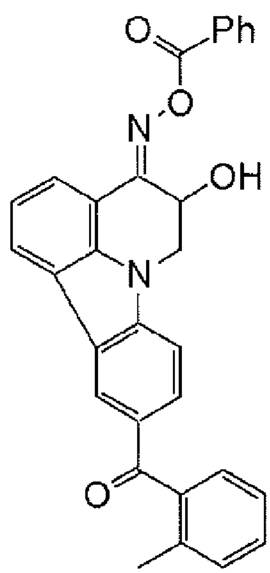

(473)

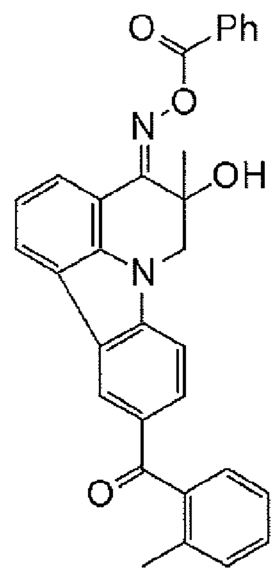

(474)

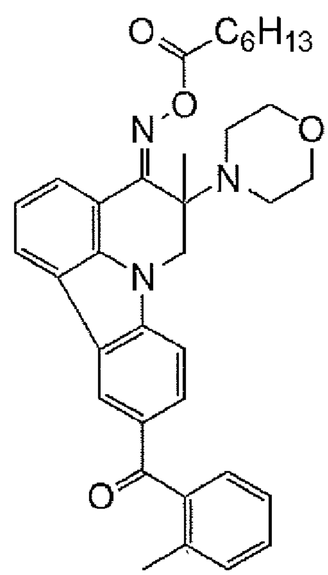

(475)

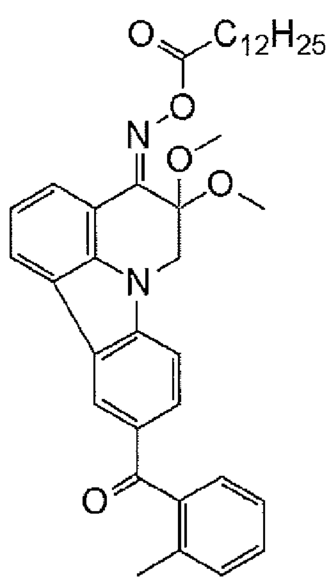

(476)

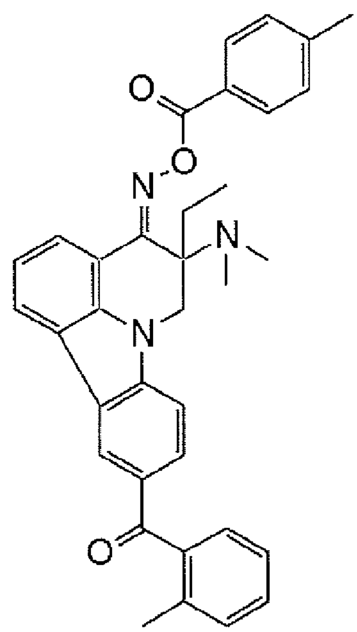

(477)

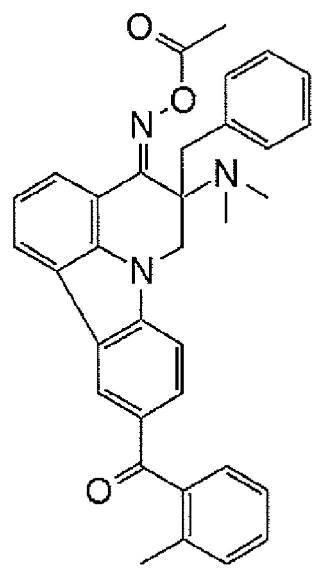

(478)

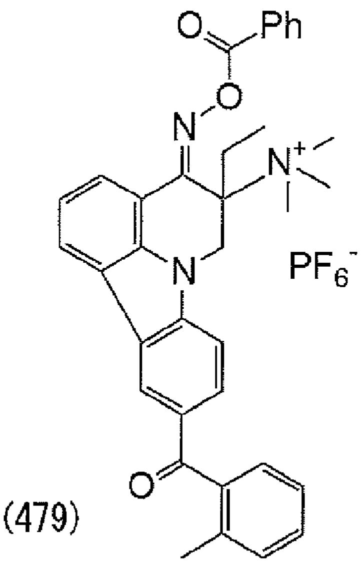

(479)

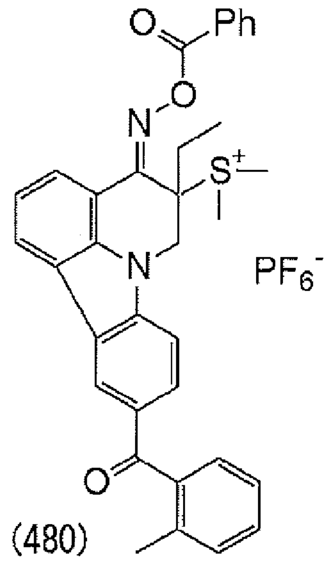

(480)

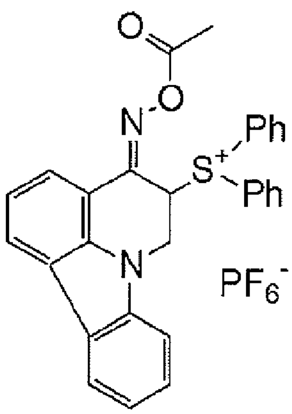

(481)

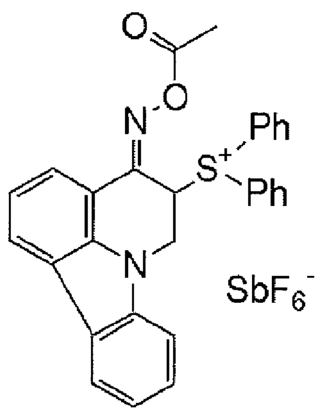

(482)

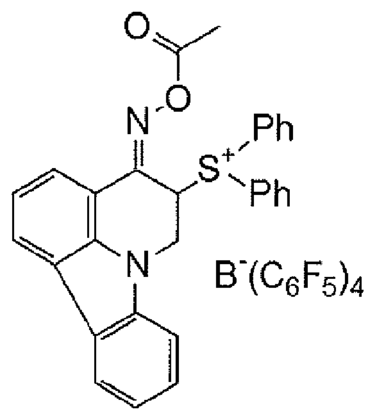

(483)

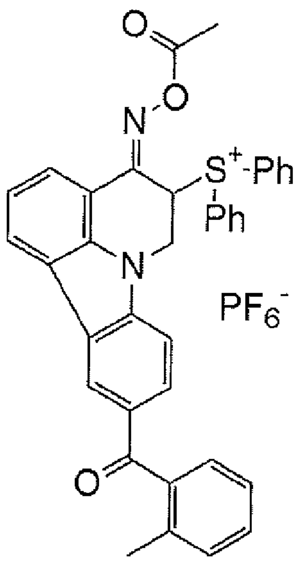

(484)

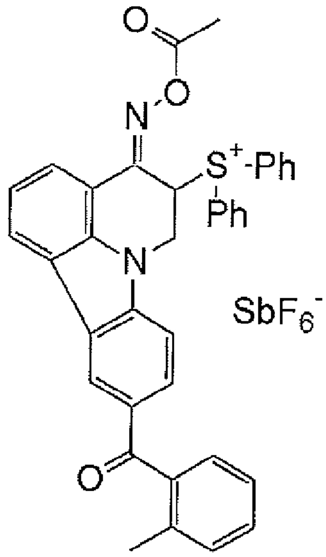

(485)

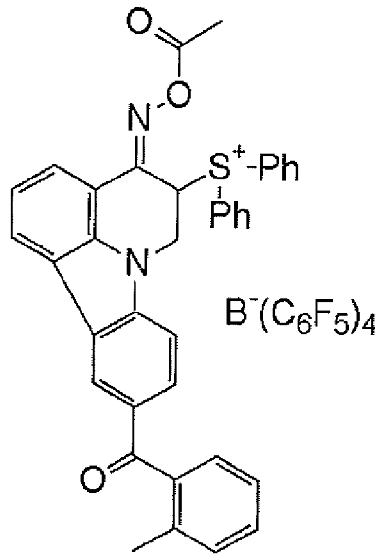

(486)

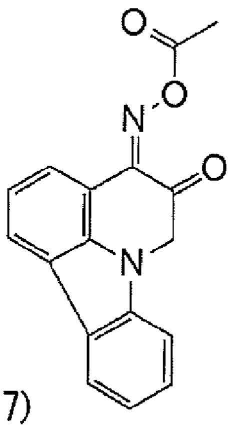

(487)

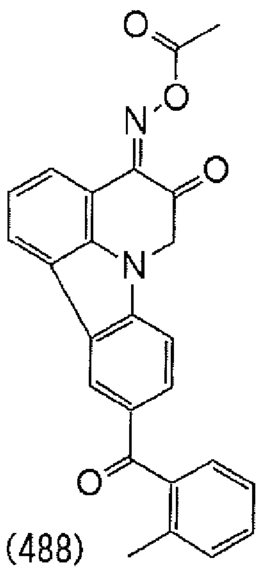

(488)

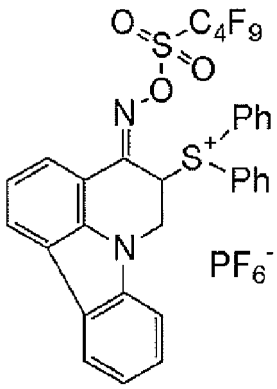

(489)

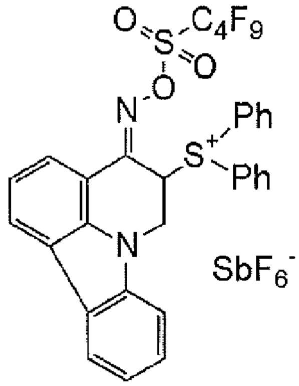

(490)

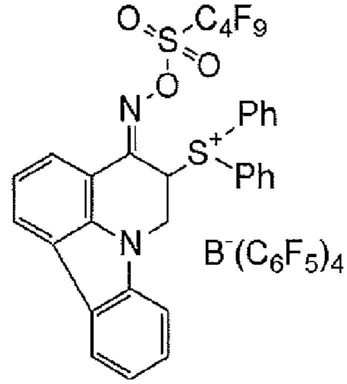

(491)

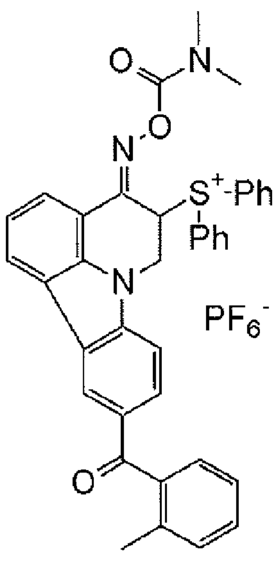

(492)

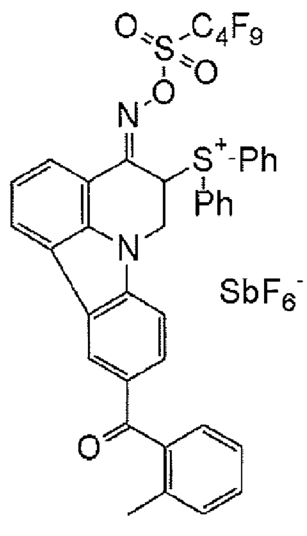

(493)

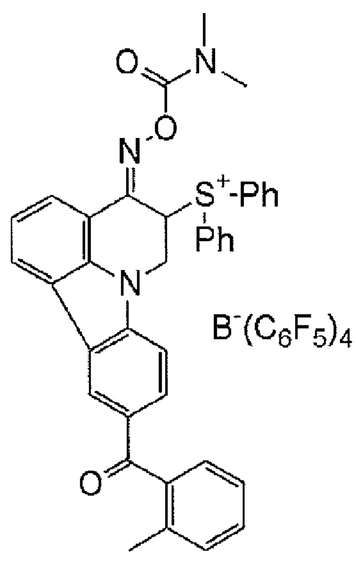

(494)

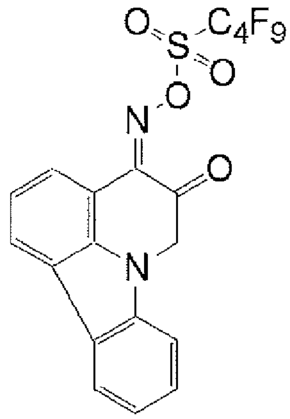

(495)

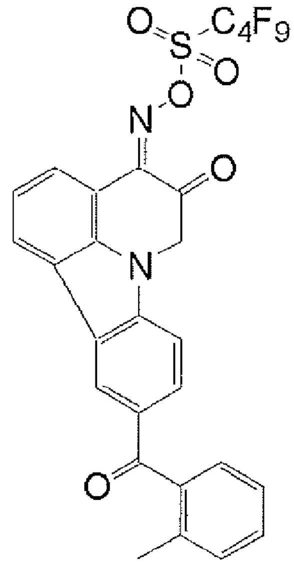

(496)

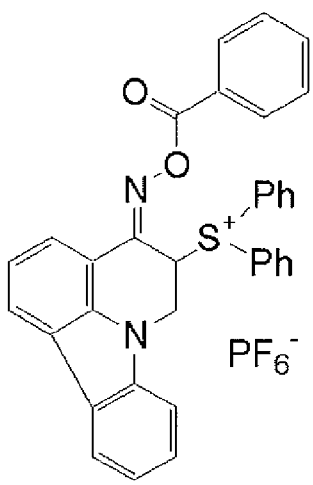

(497)

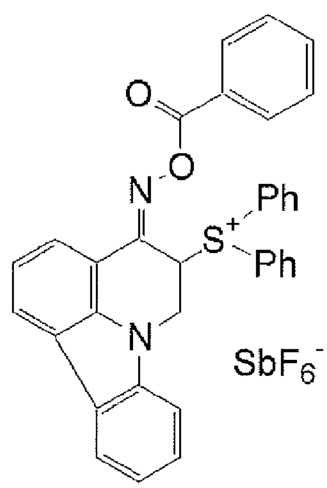

(498)

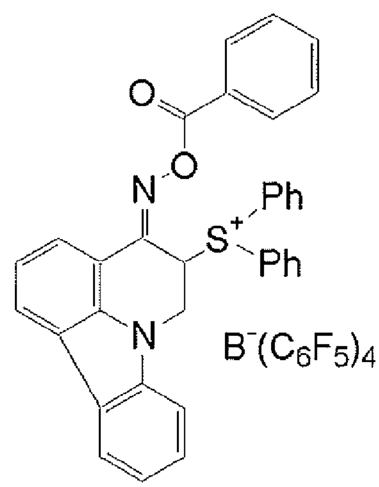

(499)

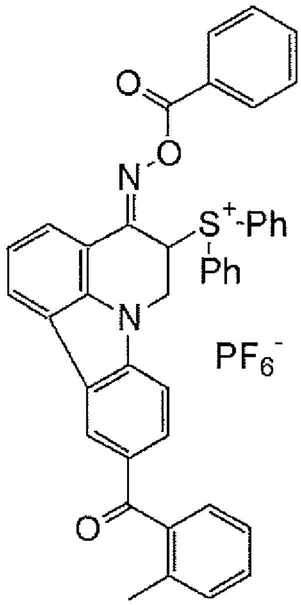

(500)

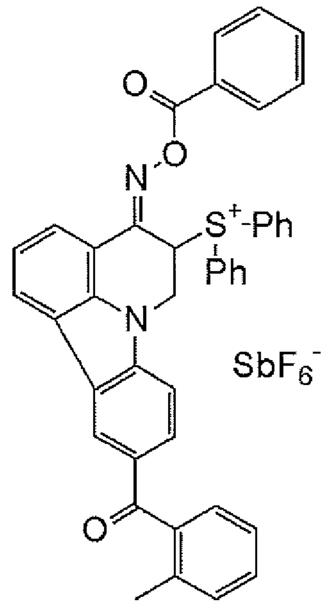

(501)

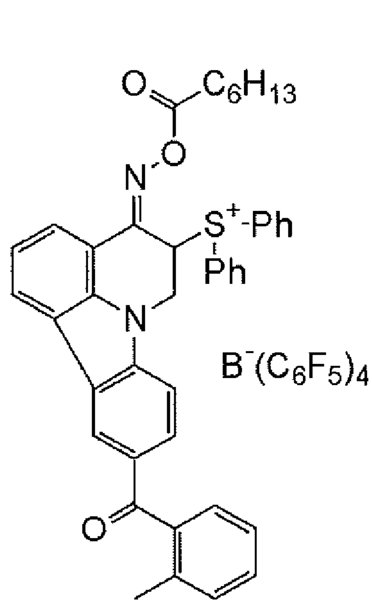

(502)

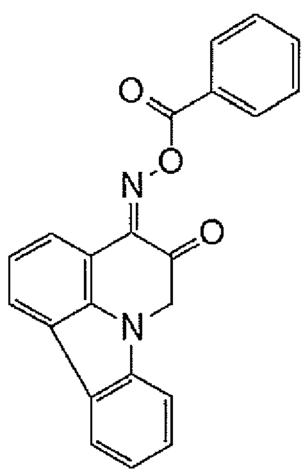

(503)

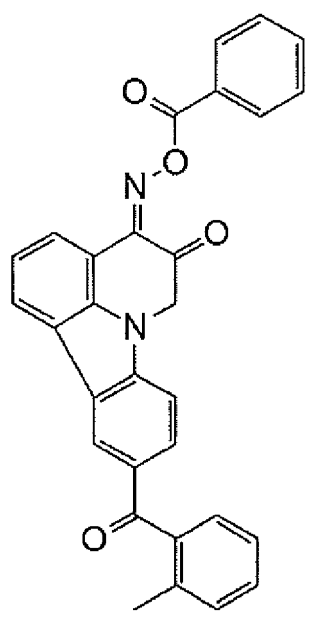

(504)

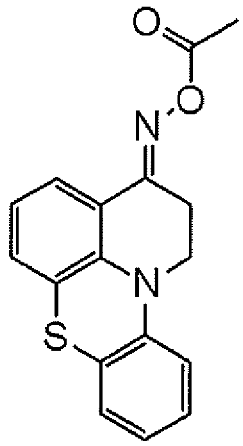

(505)

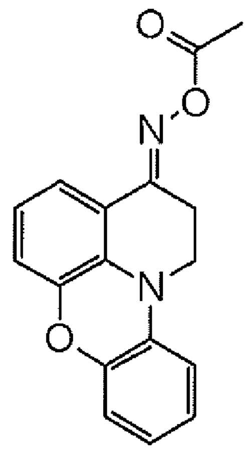

(506)

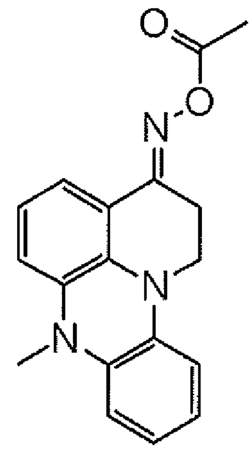

(507)

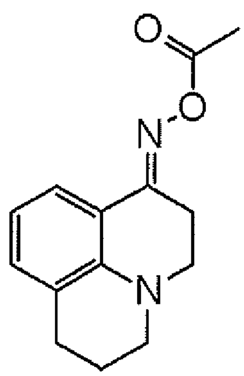

(508)

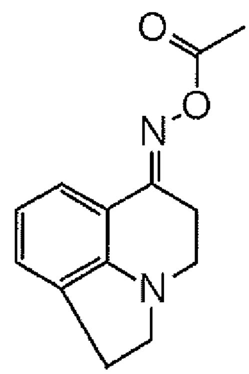

(509)

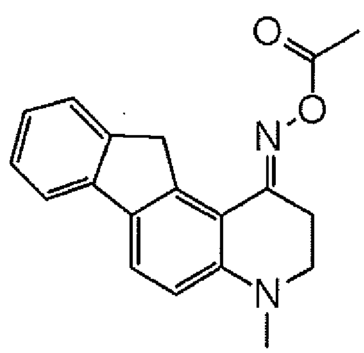

(510)

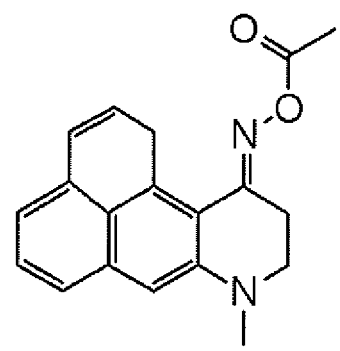

(511)

Among the specific examples (201) to (511) above, the specific examples (211) to (237), (248) to (275), (287) to (313), (322) to (347), (357) to (382), (391) to (416), (426) to (448), (458) to (463), and (468) to (504) are preferable, (211) to (237), (322) to (347), (426) to (431), (436) to (448), and (484) to (486) are more preferable, and (221) to (234),

(332) to (344), (347), (441) to (448), and (484) to (486) are most preferable, from the viewpoint of attaining higher sensitivity.

The specific oxime compound of the invention has a maximum absorption wavelength in the wavelength range of 350 nm to 500 nm, more preferably has a maximum absorption wavelength in the range of 360 nm to 480 nm. In particular, the novel oxime compound preferably has a high absorbance at 365 nm and 455 nm. Therefore, the specific oxime compound has absorption in a longer wavelength region as compared with conventional oxime compounds, so that it can exhibit high sensitivity when exposed to light from a 365 nm or 405 nm light source.

The specific oxime compound of the invention preferably has a molar absorption coefficient of 10,000 to 300,000, more preferably of 15,000 to 300,000, particularly preferably of 20,000 to 200,000 at 365 nm or 405 nm in view of sensitivity. The molar absorption coefficient of the specific oxime compound was measured at a concentration of 0.01 g/L in a solvent of ethyl acetate with an ultraviolet-visible spectrophotometer (trade name: CARRY-5 Spectrophotometer, manufactured by Varian Inc.).

The specific oxime compound of the invention (compound represented by Formula (1)) may be synthesized by methods including, but not limited to, the following method. In the following synthesis scheme, $Y^1$, Z, A, R, and $n^1$ have the same meanings as defined in $Y^1$, Z, A, R, and $n^1$ in Formula (1).

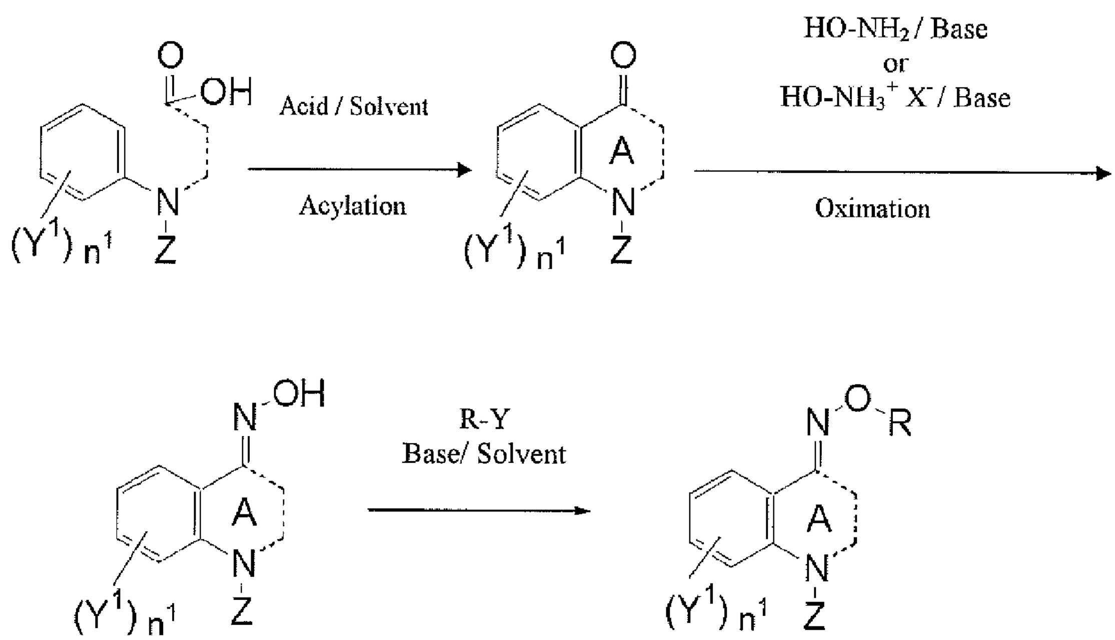


The specific oxime compound of the invention is decomposed by light and functions as a photopolymerization initiator that initiates and enhances the polymerization of polymerizable compounds. In particular, the specific oxime compound has high sensitivity to a 365 nm or 405 nm light source and thus can produce superior effects when used as a photopolymerization initiator in a photopolymerizable composition.

The specific oxime compound of the invention may also be used for applications as described blow. Examples of applications include printing inks such as screen printing inks, offset or flexographic printing inks, and UV-curable inks; white or color finishing of woods or metals; powder coatings, specifically coating materials for paper, wood, metal or plastics; building or road marking; graphic reproduction techniques; holographic recording materials; image recording techniques; manufacture of printing plate precursors developable with organic solvents or aqueous alkali; sunlight-curable coatings for use in manufacture of screen printing masks; dental filling compositions; adhesives; pressure-sensitive adhesives; lamination reins; etching resists for both wet and dry thin films; solder resists; electroplating or permanent resists; photo-forming dielectrics for printed circuit boards and electronic circuits; various displays; optical switches; optical grating (interference grating); manufacture of optical circuits; manufacture of three-dimensional products by large-scale curing (UV curing in a transparent mold) or stereo lithography techniques (for example, as described in U.S. Patent No. 4,575,330); manufacture of composite materials (such as styrene-based polyesters optionally containing

glass fibers and/or other fibers and other aids) or other thick layer compositions; resists for coating or sealing of electronic components and integrated circuits; optical fibers or optical lenses such as coatings for use in manufacture of contact lenses or Fresnel lenses; manufactured of medical equipment, aids or implants; and manufacture of thermo-tropic gels as described in German Patent No. 19,700,064 and European Patent No. 678,534.

The specific oxime compound of the invention can also generate an acid when it is irradiated with an energy ray, particularly light. Therefore, it may be used for other applications where the generated acid is used as a catalyst. For example, it may be used for image forming techniques utilizing a color reaction of a pigment precursor in the presence of the generated acid serving as a catalyst, anti-counterfeit techniques, materials for detection of energy-ray dose, and positive resists for use in manufacture of semiconductors, TFTs, color filters, micromachine components, and the like utilizing a decomposition reaction in the presence of the generated acid serving as a catalyst.

As described above, the specific oxime compound of the invention may be used as a photopolymerization initiator. Therefore, it may be preferably used in combination with a polymerizable compound to form a photopolymerizable composition (the photopolymerizable composition of the invention) that polymerizes and cures upon irradiation with light.

<Photopolymerizable Composition>

The photopolymerizable composition of the invention includes (A) the specific oxime compound and (B) a polymerizable compound.

The photopolymerizable composition of the invention has high sensitivity to light with a wavelength of 365 nm or 405 nm and also has high stability over time. In addition, it can form a cured film in which coloration caused by heating over time can be suppressed. Although the detailed mechanism is not clear, the novel oxime compound has a structure that can inhibit radical recombination when cleaved by light absorption so that it can produce a relatively large amount of radicals to achieve high sensitivity. Since radical recombination is inhibited, it is considered that the reaction between the decomposition product molecules of the novel oxime compound can be suppressed during heating over time, thereby suppressing coloration caused by the reaction.

In the invention, a color difference $\Delta Eab^*$ may be used to evaluate the coloration of the cured film caused by heating over time. The color difference $\Delta Eab^*$ may be measured with MCPD-3000 manufactured by Otsuka Electronics Co., Ltd.

The conditions for the evaluation may be as follows. First, cured films are formed by exposing the photopolymerizable composition of the invention to light at different exposure amounts ranging from 10 $mJ/cm^2$ to 2500 $mJ/cm^2$ in an ultra-high pressure mercury lamp proximity-type exposure system (manufactured by Hitachi High-Tech Electronics Engineering Co., Ltd.) or an i-line stepper exposure system (trade name: FPA-3000i5, manufactured by Canon Inc.) (365 nm). The cured films are developed as needed and then heated at 200˚C for 1 hour.

The color difference AEab* of the cured film between before and after the heating is measured so that the heat aging-induced coloration of the cured film can be evaluated.

The color difference $\Delta Eab^*$ between before and after the heating can be 5 or less, when the photopolymerizable composition of the invention is used.

The photopolymerization composition of the invention may be used for various applications such as molding resins, casting resins, photo-molding resins, sealing materials, dental polymerizing materials, printing inks, paints, photosensitive resins for printing plates, color proofs for printing, photopolymerizable compositions for color filters, resists for black matrices, resists for printed boards, resists for semiconductor processes, resists for microelectronics, resists for manufacture of micromachine components, insulating materials, hologram materials, waveguide materials, overcoat materials, adhesives, tackifiers, pressure-sensitive adhesives, and release coating agents.

The photopolymerizable composition of the invention is described below using, as examples, a photopolymerizable composition (1) suitable for forming color filters and the like and a photopolymerizable composition (2) suitable for forming photosensitive layers of planographic printing plate precursors. However, it is not limited thereto.

Photopolymerizable Composition (1)

(1)-(A) Specific Oxime Compound

The specific oxime compound (A) in the photopolymerizable composition (1) may function as a polymerization initiator.

The content of the specific oxime compound in the photopolymerizable composition (1) is preferably from 0.5 to 40% by mass, more preferably from 1 to 35% by mass, even more preferably from 1.5 to 30% by mass, base on the mass of the total solids of the composition.

The specific oxime compound may used alone or in combination of two or more.

Any known photopolymerization initiator other than the novel oxime compound may also be used in the photopolymerizable composition (1), as long as the effects of the invention are not reduced. In this case, known photopolymerization initiator is used preferably in the range of 50% by mass or less with respect to the specific oxime compound. The photopolymerization initiator that may be used in combination with the novel oxime compound is a compound that is decomposed by light to initiate and enhance the polymerization of the aftermentioned polymerizable compound. It preferably has absorption in the wavelength range of 300 to 500 nm. Examples of the photopolymerization initiator include organic halides, oxydiazole compounds, carbonyl compounds, ketal compounds, benzoin compounds, acridine compounds, organic peroxide compounds, azo compounds, coumarin compounds, azide compounds, metallocene compounds, biimidazole compounds, organic borate compounds, disulfonic acid compounds, oxime ester compounds, onium salt compounds, and acyl phosphine (oxide) compounds.

(1)-(B) Polymerizable Compound

The polymerizable compound that may be used in the photopolymerizable composition (1) may be an addition-polymerizable compound having at least one ethylenic unsaturated double bond and may be selected from compounds each having at least one ethylenic unsaturated terminal bond, preferably two or more ethylenic unsaturated terminal bonds. Such a class of compounds is widely known in the relevant industrial field, and such compounds may be used in the invention without particular limitations. Such compounds may be in the chemical form of a monomer or a prepolymer, specifically a dimer, a trimer or an oligomer, or any mixture thereof or any copolymer thereof. Examples of monomers and copolymer thereof include unsaturated carboxylic acids (such as acrylic acid, methacrylic acid, itaconic acid, crotonic acid, isocrotonic acid, and maleic acid), esters thereof, and amides thereof. Esters of unsaturated carboxylic acids and aliphatic polyhydric alcohol compounds, or amides of unsaturated carboxylic acids and aliphatic polyamines, are preferably used. Also preferably used are addition reaction products of unsaturated carboxylic acid esters or amides having a nucleophilic substituent such as a hydroxyl group, an amino group or a mercapto group, with monofunctional or polyfunctional isocyanates or epoxy compounds, and dehydration condensation reaction products of such esters or amides with monofunctional or polyfunctional carboxylic acids. Also preferred are addition reaction products of unsaturated carboxylic acid esters or amides having an electrophilic substituent such as an isocyanate group or an epoxy group, with monofunctional or polyfunctional alcohols, amines or thiols, and substitution reaction products of unsaturated carboxylic acid esters or amides having a halogen group or a leaving substituent such as a tosyloxy group, with monofunctional or polyfunctional alcohols, amines, or thiols. Other examples of preferable compounds include compounds obtained by replacing the unsaturated carboxylic acid in the above examples by an unsaturated phosphonic acid, styrene, vinyl ether, or the like.

Examples of the monomer of the ester of the aliphatic polyhydric alcohol compound and the unsaturated carboxylic acid include acrylates such as ethylene glycol diacrylate, triethylene glycol diacrylate, 1,3-butanediol diacrylate, tetramethylene glycol diacrylate, propylene glycol diacrylate, neopentyl glycol diacrylate, trimethylolpropane triacrylate, trimethylolpropane tri(acryloyloxypropyl)ether, trimethylolethane triacrylate, hexanediol diacrylate, 1,4-cyclohexanediol diacrylate, tetraethylene glycol diacrylate, pentaerythritol diacrylate, pentaerythritol triacrylate, pentaerythritol tetraacrylate, dipentaerythritol diacrylate, dipentaerythritol hexaacrylate, sorbitol triacrylate, sorbitol tetraacrylate, sorbitol pentaacrylate, sorbitol hexaacrylate, tri(acryloyloxyethyl)isocyanurate, polyester acrylate oligomers, and isocyanurate EO-modified triacrylate.

Examples of the methacrylates include tetramethylene glycol dimethacrylate, triethylene glycol dimethacrylate, neopentyl glycol dimethacrylate, trimethylolpropane trimethacrylate, trimethylolethane trimethacrylate, ethylene glycol dimethacrylate, 1,3-butanediol dimethacrylate, hexanediol dimethacrylate, pentaerythritol dimethacrylate, pentaerythritol trimethacrylate, pentaerythritol tetramethacrylate, dipentaerythritol dimethacrylate, dipentaerythritol hexamethacrylate, sorbitol trimethacrylate, sorbitol tetramethacrylate, bis[p-(3-methacryloxy-2-hydroxypropoxy)phenyl]dimethylmethane, and bis[p-(methacryloxyethoxy)phenyl]dimethylmethane.

Examples of itaconic acid esters include ethylene glycol diitaconate, propylene glycol diitaconate, 1,3-butanediol diitaconate, 1,4-butanediol diitaconate, tetramethylene glycol diitaconate, pentaerythritol diitaconate, and sorbitol tetraitaconate. Examples of crotonates include ethylene glycol dicrotonate, tetramethylene glycol dicrotonate, pentaerythritol dicrotonate, and sorbitol tetradicrotonate; isocrotonates such as ethylene glycol diisocrotonate, pentaerythritol diisocrotonate, and sorbitol tetraisocrotonate; and maleates such as ethylene glycol dimaleate, triethylene glycol dimaleate, pentaerythritol dimaleate, and sorbitol tetramaleate.

Examples of other esters include the aliphatic alcohol esters described in Japanese Patent Application Publication (JP-B) No. 51-47334 and JP-A No. 57-196231, the aromatic skeleton-containing compounds described in JP-A Nos. 59-5240, 59-5241 and 02-226149, and the amino group-containing compounds described in JP-A No. 01-165613. A mixtures of monomers selected from the ester monomers described above may also be used.

Examples of the monomer of the amide of the aliphatic polyamine compound and the unsaturated carboxylic acid include methylenebis-acrylamide, methylenebis-methacrylamide, 1,6-hexamethylenebis-acrylamide, 1,6-hexamethylenebis-methacrylamide, diethylenetriaminetrisacrylamide, xylylenebisacrylamide, and xylylenebismethacrylamide.

Examples of other preferred amide monomers include the cyclohexylene structure-containing compounds described in JP-B No. 54-21726.

Addition-polymerizable urethane compounds produced by an addition reaction of isocyanate with a hydroxyl group are also preferred, examples of which include the vinyl urethane compounds described in JP-B No. 48-41708, which have two or more polymerizable vinyl groups within a molecule and are produced by adding a hydroxyl group-containing vinyl monomer represented by Formula (A) below to a polyisocyanate compound having two or more isocyanate groups within a molecule.

$$CH_2=C(R^4)COOCH_2CH(R^5)OH \qquad (A)$$

In Formula (A), $R^4$ and $R^5$ each represent H or $CH_3$.

Also preferred are the urethane acrylates described in JP-A No. 51-37193, JP-B No. 02-32293 and JP-B No. 02-16765, and the ethylene oxide skeleton-containing urethane compounds described in JP-B Nos. 58-49860, 56-17654, 62-39417, and 62-39418. Photopolymerizable compositions having excellent photoresponsive speed can also be obtained using addition-polymerizable compounds having an amino or sulfide structure in the molecule, which are disclosed in JP-ANos. 63-277653, 63-260909 and 01-105238.

Other examples include polyfunctional acrylates and methacrylates such as polyester acrylates and epoxy acrylates produced by a reaction of epoxy resins with (meth)acrylic acid (for example those disclosed in JP-A No. 48-64183, JP-B No. 49-43191 and JP-B No. 52-30490); and the specific unsaturated compounds described in JP-B Nos. 46-43946, 01-40337 and 01-40336, and the vinylphosphonic acid compounds described in JP-A No. 02-25493. In some cases, the perfluoroalkyl group-containing structures described in JP-A No. 61-22048 are preferably used. Photosetting monomers and oligomers as described in Journal of the Adhesion Society of Japan Vol. 20, No. 7 pp. 300 to 308 (1984) may also be used.

Details of how to use the addition-polymerizable compounds, such as what structure should be used, whether they should be used alone or in combination, or what amount should be added, may be freely determined depending on the final performance design of the curable composition. For example, they may be selected from the following viewpoints.

In view of sensitivity, a structure having a higher content of the unsaturated groups per molecule is preferable, and difunctional or higher functional structures are preferred in many cases. In order to increase the strength of the cured film, tri- or higher-functional structures are preferred. A method of using a combination of compounds having different numbers of functional groups and/or different types of polymerizable groups (for example, compounds selected from an acrylic ester, a methacrylic ester, a styrene compound, a vinyl ether compound) is also effective for controlling both of sensitivity and strength.

How to select and use the addition-polymerizable compound is also an important factor for the compatibility with or dispersibility to other components of the photopolymerizable composition (such as a photopolymerization initiator, a colorant (a pigment and/or a dye), and a binder polymer). For example, in some cases, the compatibility may be improved by using a low-purity compound or by using a combination of two or more compounds. A particular structure may also be selected in order to improve adhesion to the hard surface of a support or the like.

(1)-(C) Colorant

The photopolymerizable composition (1) may contain (C) a colorant. When the colorant is added, a colored photopolymerizable composition of a desired color may be obtained.

The photopolymerizable composition (1) contains (A) the specific oxime compound having excellent sensitivity to a short-wavelength light source such as a 365 nm or 406 nm light source. Therefore, the photopolymerizable composition (1) can be cured with high sensitivity, even when it contains a colorant at a high concentration.

The colorant used in the photopolymerizable composition (1) is not particularly limited. One of, or a mixture of two or more of, various known conventional dyes and pigments may be used, which may be appropriately selected depending on the use of the photopolymerizable composition. When the colored photopolymerizable composition of the invention is used for the production of a color filter, either of a colorant of a chromatic color such as R, G or B for forming color pixels of the color filter or a black colorant generally used for forming black matrices may be employed.

The photopolymerizable composition of the invention containing the specific oxime compound (A) may be cured with high sensitivity even with a lower amount of exposure light, and may thus be particularly preferably used in a photopolymerizable composition containing a black colorant hardly permeable with light.

Colorants applicable to the photopolymerizable composition (1) are described in detail below, by describing colorants suitable for color filters as examples.

Chromatic pigments to be used may be various known conventional inorganic or organic pigments. Higher transparency

is preferable regardless of whether the pigment is inorganic or organic. From this point of view, pigment particles are preferably smaller. When also considering handleability, the average particle size of the pigments is preferably from 0.01 μm to 0.1 μm, more preferably from 0.01 μm to 0.05 μm.

Examples of the inorganic pigments include metal compounds, typical examples of which include metal oxides and metal complex salts. Specific examples include oxides of iron, cobalt, aluminum, cadmium, lead, copper, titanium, magnesium, chromium, zinc, antimony, or other metals, and complex oxides of the above metals.

Examples of the organic pigments include:

C.I. Pigment Yellow 11, 24, 31, 53, 83, 93, 99, 108, 109, 110, 138, 139, 147, 150, 151, 154, 155, 167, 180, 185, 199;
C.I. Pigment Orange 36, 38, 43, 71;
C.I. Pigment Red 81, 105, 122, 149, 150, 155, 171, 175, 176, 177, 209, 220, 224, 242, 254, 255, 264, 270;
C.I. Pigment Violet 19, 23, 32, 39;
C.I. Pigment Blue 1, 2, 15, 15:1, 15:3, 15:6, 16, 22, 60, 66;
C.I. Pigment Green 7, 36, 37;
C.I. Pigment Brown 25, 28;
C.I. Pigment Black 1, 7; and
carbon black.

In the invention, pigments having a basic N atom in their structures are particularly preferably used. The basic N atom-containing pigments exhibit high dispersibility in the photopolymerizable composition (1) of the invention. Although the reason for that has not been clearly understood yet, it is suggested that high affinity between the photosensitive polymerizable component and the pigment may have an effect.

In the invention, examples of pigments that are preferably used include, but are not limited to, the pigments listed below.

C.I. Pigment Yellow 11, 24, 108, 109, 110, 138, 139, 150, 151, 154, 167, 180, 185;
C.I. Pigment Orange 36, 71;
C.I. Pigment Red 122, 150, 171, 175, 177, 209, 224, 242, 254, 255, 264;
C.I. Pigment Violet 19, 23, 32;
C.I. Pigment Blue 15:1, 15:3, 15:6, 16, 22, 60, 66; and
C.I. Pigment Black 1.

Only a single organic pigment may be used, or a combination of plural organic pigments may be used to heighten color purity. Some examples of the combination are described below. For example, a red pigment of one or more of an anthraquinone pigment, a perylene pigment and a diketopyrrolopyrrole pigment may be mixed with a disazo yellow pigment, an isoindolin yellow pigment, a quinophthalone yellow pigment, or a perylene red pigment. For example, the anthraquinone pigment may be C.I. Pigment Red 177, the perylene pigment may be C.I. Pigment Red 155 or C.I. Pigment Red 224, and the diketopyrrolopyrrole pigment may be C.I. Pigment Red 254. In view of color reproducibility, C.I. Pigment Yellow 139 is preferably used to form a mixture. The mass ratio of the yellow pigment to the red pigment (yellow pigment / red pigment) is preferably in the range of from 5/100 to 50/100. If the mass ratio is 4/100 or lower, it can be difficult to inhibit the light transmittance in the range of 400 nm to 500 nm, and color purity cannot be heightened in some cases. If the ratio is 51/100 or higher, the dominant wavelength can be shifted to the shorter wavelength side, so that a relatively large deviation from the NTSC target hue can be observed in some cases. In particular, the mass ratio is most preferably in the range of from 10/100 to 30/100. In the case of a combination of different red pigments, the mass ratio thereof may be adjusted depending on the hue.

A green pigment of a halogenated phthalocyanine pigment may be used alone or in a form of a mixture with a disazo yellow pigment, a quinophthalone yellow pigment, an azomethine yellow pigment, or an isoindolin yellow pigment. For example, C.I. Pigment Green 7, 36 or 37 is preferably mixed with C.I. Pigment Yellow 83, C.I. Pigment Yellow 138, C.I. Pigment Yellow 139, C.I. Pigment Yellow 150, C.I. Pigment Yellow 180, or C.I. Pigment Yellow 185. The mass ratio of the yellow pigment to the green pigment (yellow pigment / green pigment) is preferably in the range of from 5/100 to 150/100. If the mass ratio is lower than 5/100, it can be difficult to inhibit the light transmittance in the range of 400 nm to 450 nm, so that color purity cannot be heightened in some cases. If the ratio is higher than 150/100, the dominant wavelength can be shifted to the longer wavelength side, so that a relatively large deviation from the NTSC target hue can be observed in some cases. In particular, the mass ratio is preferably in the range of from 30/100 to 120/100.

A blue pigment of a phthalocyanine pigment may be used alone or in a form of a mixture with a dioxazine violet pigment. For example, C.I. Pigment Blue 15:6 is preferably mixed with C.I. Pigment Violet 23. The mass ratio of the violet pigment to the blue pigment (violet pigment / blue pigment) is preferably in the range of from 0/100 to 30/100, more preferably 10/100 or less.

Carbon, titanium carbon, iron oxide, and titanium oxide may be used alone or in any combination thereof as a pigment for black matrices, and a combination of carbon and titanium carbon is preferred. The mass ratio of titanium

carbon to carbon (titanium carbon / carbon) is preferably from 0/100 to 60/100. If the ratio is 61/100 or higher, the dispersibility can be reduced in some cases.

In the photopolymerizable composition (1), when the colorant is a dye, a colored composition can be obtained in which the dye is uniformly dissolved.

Examples of the dye that may be used as the colorant in the photopolymerizable composition (1) include, but are not limited to, known dyes for conventional color filters. Examples of dyes that may be used include the dyes disclosed in JP-A Nos. 64-90403, 64-91102, 01-94301, and 06-11614, Japanese Patent No. 2592207, U.S. Patent Nos. 4,808,501, 5,667,920 and 5,059,500, and JP-ANos. 05-333207, 06-35183, 06-51115, 06-194828, 08-211599, 04-249549, 10-123316, 11-302283, 07-286107, 2001-4823, 08-15522, 08-29771, 08-146215, 11-343437, 08-62416, 2002-14220, 2002-14221, 2002-14222, 2002-14223, 08-302224, 08-73758, 08-179120, and 08-151531.

Concerning chemical structure, pyrazole azo dyes, anilino azo dyes, triphenylmethane dyes, anthraquinone dyes, anthrapyridone dyes, benzylidene dyes, oxonol dyes, pyrazolotriazole azo dyes, pyridone azo dyes, cyanine dyes, phenothiazine dyes, pyrrolopyrazole azomethine dyes, xanthene dyes, phthalocyanine dyes, benzopyran dyes, and indigo dyes may be used.

In some resist systems involving water or alkali development, acid dyes and/or derivatives thereof may be preferably used in order to completely remove the binder and/or dye in an unirradiated portion by development.

Other dyes such as direct dyes, basic dyes, mordant dyes, acid mordant dyes, azoic dyes, disperse dyes, oil-soluble dyes, food dyes, and/or derivatives thereof may be effectively used.

Any acid dye having an acidic group such a sulfonic acid group or a carboxylic acid group may be used. The acid dye may be selected taking into account all the necessary properties such as solubility in an organic solvent or developer, ability to form a salt with a basic compound, absorbance, interaction with other components in the composition, light resistance, and heat resistance.

Examples of the acid dye include, but are not limited to, the following dyes: Acid Alizarin Violet N: Acid Black 1, 2, 24, 48; Acid Blue 1, 7, 9, 15, 18, 23, 25, 27, 29, 40 to 45, 62, 70, 74, 80, 83, 86, 87, 90, 92, 103, 112, 113, 120, 129, 13 8, 147, 158, 171, 182, 192, 243, 324:1; Acid Chrome Violet K; Acid Fuchsin; Acid Green 1, 3, 5, 9, 16, 25, 27, 50; Acid Orange 6, 7, 8, 10, 12, 50, 51, 52, 56, 63, 74, 95; Acid Red 1, 4, 8, 14, 17, 18, 26, 27, 29, 31, 34, 35, 37, 42, 44, 50, 51, 52, 57, 66, 73, 80, 87, 88, 91, 92, 94, 97, 103, 111, 114, 129, 133, 134, 138, 143, 145, 150, 151, 158, 176, 183, 198, 211, 215, 216, 217, 249, 252, 257, 260, 266, 274; Acid Violet 6B, 7, 9, 17, 19; Acid Yellow 1, 3, 7, 9, 11, 17, 23, 25, 29, 34, 36, 42, 54, 72, 73, 76, 79, 98, 99, 111, 112, 114, 116, 184, 243; Food Yellow 3; and derivatives of these dyes.

Particularly preferred examples of the acid dye include Acid Black 24; Acid Blue 23, 25, 29, 62, 80, 86, 87, 92, 138, 158, 182, 243, 324:1; Acid Orange 8, 51, 56, 63, 74; Acid Red 1, 4, 8, 34, 37, 42, 52, 57, 80, 97, 114, 143, 145, 151, 183, 217; Acid Violet 7; Acid Yellow 17, 25, 29, 34, 42, 72, 76, 99, 111, 112, 114, 116, 184, 243; Acid Green 25; and derivatives of these dyes.

Acid dyes such as azo, xanthene and phthalocyanine dyes other than the above are also preferred. Acid dyes such as C.I. Solvent Blue 44, 38, C.I. Solvent Orange 45, Rhodamine B, and Rhodamine 110, and derivatives of these acid dyes are also preferably used.

In particular, the colorant is preferably selected from triarylmethane dyes, anthraquinone dyes, azomethine dyes, benzylidene dyes, oxonol dyes, cyanine dyes, phenothiazine dyes, pyrrolopyrazole azomethine dyes, xanthene dyes, phthalocyanine dyes, benzopyran dyes, indigo dyes, pyrazole azo dyes, anilino azo dyes, pyrazolotriazole azo dyes, pyridone azo dyes, and anthrapyridone dyes.

The colorant that may be used in the photopolymerizable composition (1) is preferably a pigment whose average particle size (unit: nm) satisfies the relation $20 \leq r \leq 300$, more preferably $125 \leq r \leq 250$, particularly preferably $30 \leq r \leq 200$. Red and green pixels with high contrast ratio and high light transmittance can be obtained by using pigments with such an average particle size r. As used herein, the term "average particle size" refers to the average particle size of secondary particles formed by aggregation of primary particles (single fine crystals) of a pigment.

The particle size distribution of the secondary particles of the pigment for use in the invention (hereinafter, simply referred to as "particle size distribution") is preferably such that 70% by mass or more, more preferably 80% by mass or more, of the whole of the secondary particles fall within (the average particle size $\pm$ 100) nm.

The pigment with the average particle size and the particle size distribution described above may be prepared by a process including mixing and dispersing a commercially available pigment and another optional pigment (generally having an average particle size more than 300 nm), preferably as a pigment mixture liquid with a dispersing agent and a solvent, while grinding them with a grinding machine such as a bead mill or a roll mill. The resulting pigment is generally in the form of a pigment dispersion liquid.

The content of the colorant contained in the photopolymerizable composition (1) is preferably 30 to 95% by mass, more preferably 40 to 90% by mass, even more preferably 50 to 80% by mass, based on the total solid content of the photopolymerizable composition.

When the content of the colorant is in the above range, a color filter prepared from the photopolymerizable composition (1) can attain suitable chromaticity. The photopolymerization of the photopolymerizable composition can sufficiently

proceed to maintain the strength thereof as a film, and thus development latitude upon alkali development can be prevented from narrowing. That is, the specific oxime compound (A) used in the invention has high light absorption efficiency and thus significantly exhibits an effect of improving sensitivity even if the colorant is contained at high concentration in the photopolymerizable composition.

(1)-(D) Pigment Dispersing Agent

When the photopolymerizable composition (1) contains a pigment as the colorant, a pigment dispersing agent (D) is preferably added to the composition in order to improve the dispersibility of the pigment.

Examples of pigment dispersing agents that may be used in the invention include polymeric dispersants (such as polyamide amines, salts thereof, polycarboxylic acids, salts thereof, high-molecular-weight unsaturated acid esters, modified polyurethanes, modified polyesters, modified poly(meth)acrylates, (meth)acrylic copolymers, and naphthalenesulfonic acid formalin condensates), and polyoxyethylene alkyl phosphates, polyoxyethylene alkylamines, alkanolamines, and pigment derivatives.

Polymeric dispersants may be further classified by structure into linear polymers, terminal-modified polymers, graft polymers, and block polymers.

The polymeric dispersant is adsorbed on the surface of the pigment and acts to prevent reaggregation. Therefore, terminal-modified polymers, graft polymers and block polymers having an anchor moiety to the surface of the pigment are preferred structures.

On the other hand, a pigment derivative has an effect of facilitating adsorption of a polymer dispersant by modifying the surface of a pigment.

Examples of pigment dispersing agents that may be used in the invention include DISPERBYK 101 (polyamide amine phosphate), 107 (carboxylic acid ester), 110 (acid group-containing copolymer), 130 (polyamide), 161, 162, 163, 164, 165, 166, and 170 (high-molecular-weight polymer), and BYK-P104 and P105 (high-molecular-weight unsaturated polycarboxylic acid) each manufactured by BYK Chemie; EFKA 4047, 4050 - 4010 - 4165 (polyurethane dispersants), EFKA 4330 - 4340 (block copolymer), 4400 - 4402 (modified polyacrylate), 5010 (polyester amide), 5765 (high-molecular-weight polycarboxylate), 6220 (fatty acid polyester), 6745 (phthalocyanine derivative), and 6750 (azo pigment derivative) each manufactured by EFKA; AJISPER PB821 and PB822 manufactured by Ajinomoto Fine-Techno Co., Inc.; FLOWLEN TG-710 (urethane oligomer) and POLYFLOW Nos. 50E and 300 (acrylic copolymer) each manufactured by Kyoeisha Chemical Co., Ltd.; DISPARLON KS-860, 873SN, 874, #2150 (aliphatic polycarboxylic acid), #7004 (polyether ester), DA-703-50, DA-705, and DA-725 each manufactured by Kusumoto Chemicals, Ltd.; DEMOL RN, N (naphthalenesulfonic acid formalin polycondensate), MS, C, SN-B (aromatic sulfonic acid formalin polycondensate), HOMOGENOL L-18 (high-molecular weight polycarboxylic acid), EMULGEN 920, 930, 935, and 985 (polyoxyethylene nonyl phenyl ether), and ACETAMIN 86 (stearylamine acetate) each manufactured by Kao Corporation; SOLSPERSE 5000 (phthalocyanine derivative), 22000 (azo pigment derivative), 13240 (polyester amine), 3000, 17000, 27000 (polymer having a functional part at a terminal), 24000, 28000, 32000, and 38500 (graft polymers) each manufactured by The Lubrizol Corporation; and NIKKOL T106 (polyoxyethylene sorbitan monooleate) and MYS-IEX (polyoxyethylene monostearate) each manufactured by Nikko Chemicals Co., Ltd.

Only a single dispersing agent may be used, or two or more of dispersing agents may be used in combination.

In the invention, it is particularly preferable to use a pigment derivative and a polymeric dispersant in combination.

In the photopolymerizable composition (1), the content of the dispersing agent is preferably from 1 to 80% by mass, more preferably from 5 to 70% by mass, even more preferably from 10 to 60% by mass, based on the mass of the pigment.

For example, when a polymeric dispersant is used, the content thereof is preferably from 5 to 100% by mass, more preferably from 10 to 80% by mass of the pigment.

When a pigment derivative is used, the content thereof is preferably from 1 to 30% by mass, more preferably from 3 to 20% by mass, particularly preferably from 5 to 15% by mass of the pigment.

In the photopolymerizable composition (1), when a pigment as a colorant and a dispersing agent are used, the total content of the colorant and the dispersing agent is preferably from 30 to 90% by mass, more preferably from 40 to 85% by mass, even more preferably from 50 to 80% by mass, based on the mass of the total solids of the curable composition, in view of curing sensitivity and color density.

If necessary, the photopolymerizable composition (1) may further contain any of the optional components described in detail below.

A description is given below of optional components that may be added to the photopolymerizable composition (1).

(1)-(E) Sensitizer

The photopolymerizable composition (1) may contain a sensitizer for the purpose of improving the radical

generation efficiency of a radical initiator or making the photosensitive wavelength longer.
The sensitizer that may be used in the invention preferably heighten the sensitivity of (A) the specific oxime compound based on the electron-transfer mechanism or the energy-transfer mechanism.

The sensitizer for use in the photopolymerizable composition (1) may belong to any of the groups of compounds described below and may have an absorption wavelength in the range of 300 nm to 450 nm.
Examples include polynuclear aromatic compounds (such as phenanthrene, anthracene, pyrene, perylene, triphenylene, and 9,10-dialkoxyanthracene), xanthenes (such as fluorescein, eosin, erythrosine, rhodamine B, and rosebengal), thioxanthones (such as isopropylthioxanthone, diethylthioxanthone and chlorothioxanthone), cyanines (such as thiacarbocyanine and oxacarbocyanine), merocyanines (such as merocyanine and carbomerocyanine), phthalocyanines, thiazines (such as thionine, methylene blue and toluidine blue), acridines (such as acridine orange, chloroflavin and acriflavin), anthraquinones (such as anthraquinone), squaliums (such as squalium), acridine orange, coumarins (such as 7-diethylamino-4-methylcoumarin), ketocoumarin, phenothiazines, phenazines, styrylbenzenes, azo compounds, diphenylmethane, triphenylmethane, distyrylbenzenes, carbazoles, porphyrin, spiro compounds, quinacridone, indigo, styryl compounds, pyrylium compounds, pyrromethene compounds, pyrazolotriazole compounds, benzothiazole compounds, barbituric acid derivatives, thiobarbituric acid derivatives, aromatic ketone compounds such as acetophenone, benzophenone, thioxanthone, and Michler's ketone, and heterocyclic compounds such as N-aryloxazolidinone.

Examples of more preferred sensitizers include compounds represented by Formulae (e-1) to (e-4) below.

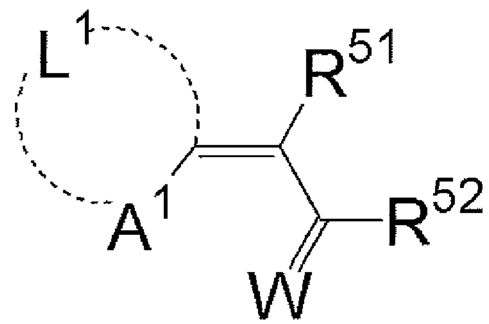

(e-1)

In Formula (e-1), $A^1$ represents a sulfur atom or $NR^{50}$, $R^{50}$ represents an alkyl group or an aryl group, $L^1$ represents a nonmetallic atom group that forms a basic nuclear of the pigment together with $A^1$ and the carbon atom adjacent to $L^1$, $R^{51}$ and $R^{52}$ each independently represent a hydrogen atom or a monovalent nonmetallic atom group, and $R^{51}$ and $R^{52}$ may be bonded to each other to form an acidic nuclear of the pigment, and W represents an oxygen atom or a sulfur atom.

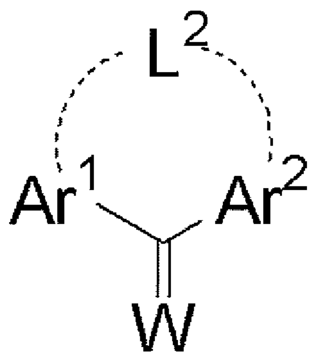

(e-2)

In Formula (e-2), $Ar^1$ and $Ar^2$ each independently represent an aryl group and are linked to each other via $-L^2-$, wherein $-L^2-$ represents -O- or -S-, and W has the same definition as in Formula (e-1).

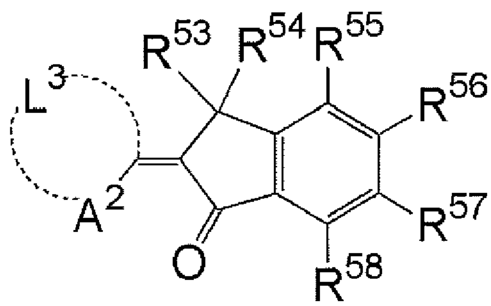

(e-3)

In Formula (e-3), $A^2$ represents a sulfur atom or $NR^{59}$; $L^3$ represents a nonmetallic atom group that forms a basic nuclear of the pigment together with $A^2$ and the carbon atom adjacent to $L^3$; $R^{53}$, $R^{54}$, $R^{55}$, $R^{56}$, $R^{57}$, and $R^{58}$ each independently represent a monovalent nonmetallic atom group; and $R^{59}$ represents an alkyl group or an aryl group.

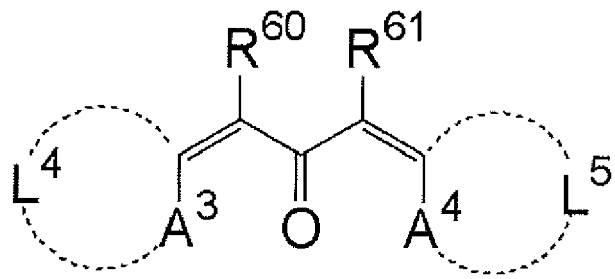

(e-4)

In Formula (e-4), $A^3$ and $A^4$ each independently represent -S- or $-NR^{62}$; $R^{62}$ represents a substituted or unsubstituted alkyl group or a substituted or unsubstituted aryl group; $L^4$ and $L^5$ each independently represent a nonmetallic atom group that forms a basic nuclear of the pigment together with adjacent $A^3$ or $A^4$ and the carbon atom adjacent to $L^4$ or $L^5$; and $R^{60}$ and $R^{61}$ each independently represent a monovalent nonmetallic atom group or may be bonded to each other to form an aliphatic or aromatic ring.

The content of the sensitizer in the photopolymerizable composition (1) is preferably from 0.1 to 20% by mass, more preferably from 0.5 to 15% by mass, based on the mass of the solids, in view of the efficiency of light absorption efficiency at a deep portion or the efficiency of initiation decomposition.

A single sensitizer may be used, or two or more sensitizers may be used in combination.

The sensitizer that is preferably contained in the photopolymerizable composition (1) may be at least one selected from a compound represented by Formula (II) below and a compound represented by Formula (III) shown later other than above mentioned sensitizer.

A single compound selected from these sensitizers may be used, or two or more compounds selected from these sensitizers may be used in combination.

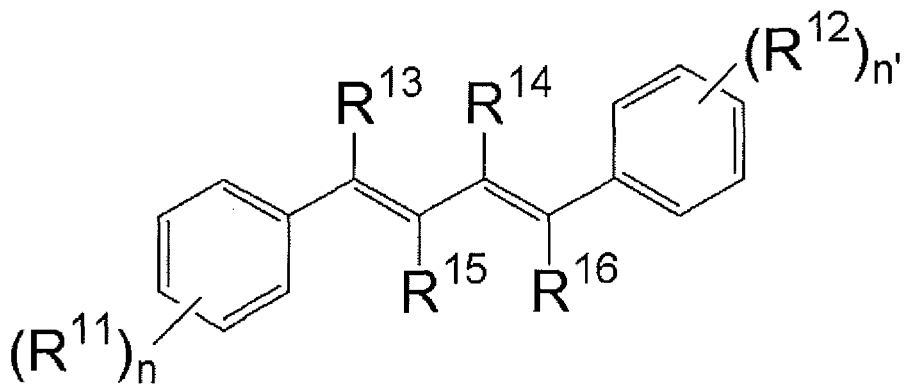

(II)

In Formula (II), $R^{11}$ and $R^{12}$ each independently represent a monovalent substituent, $R^{13}$, $R^{14}$, $R^{15}$, and $R^{16}$ each independently represent a hydrogen atom or a monovalent substituent, n represents an integer of 0 to 5, n' represents an integer of 0 to 5, and n and n' are not simultaneously 0. When n is 2 or greater, there are plural $R^{11}$s, and they may be the same or different. When n' is 2 or greater, there are plural $R^{12}$s, and they may be the same or different. Formula (II) may represent any of isomers caused by the presence of the double bonds.

The compound represented by Formula (II) preferably has a molar absorption coefficient ε at a wavelength of 365 nm of 500 $mol^{-1} \cdot L \cdot cm^{-1}$ or more, more preferably of 3000 $mor^{-1} \cdot L \cdot cm^{-1}$ or more, most preferably of 20000 $mol^{-1} \cdot L \cdot cm^{-1}$ or more. If the molar absorption coefficient ε is in the above range at each wavelength, the sensitivity enhancing effect may be high in terms of light absorption efficiency, which is preferable.

Preferred examples of the compound represented by Formula (II) include, but are not limited to, the compounds illustrated below.

In the description, some chemical formulae are simplified structural formulae in which solid lines represent hydrocarbon groups, unless elements and substituents are explicitly indicated.

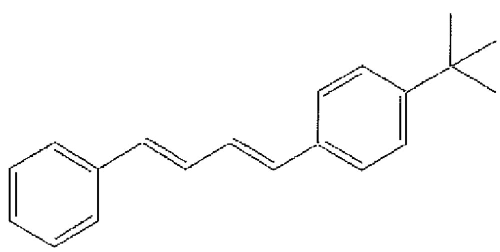

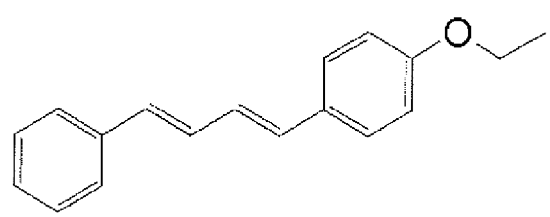

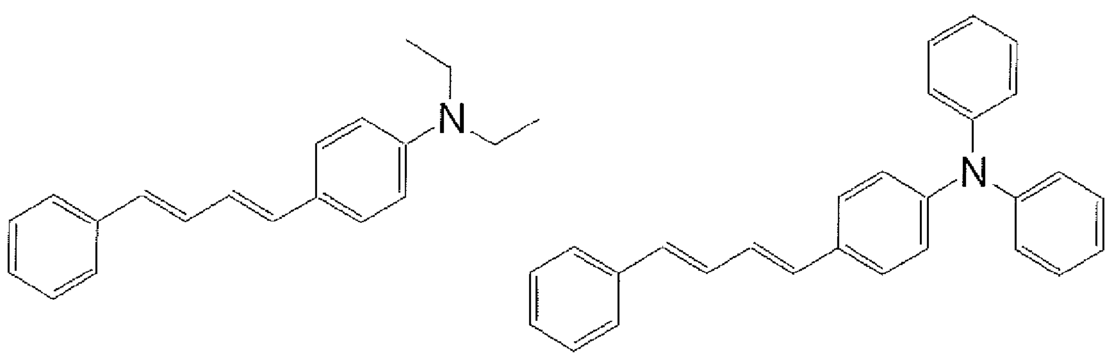

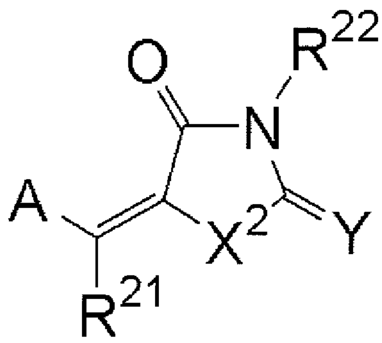

(III)

In Formula (III), A represents an optionally substituted aromatic or heterocyclic group; $X^2$ represents an oxygen atom, a sulfur atom or $-N(R^{23})-$; Y represents an oxygen atom, a sulfur atom or $=N(R^{23})$; $R^{21}$, $R^{22}$ and $R^{23}$ each independently represent a hydrogen atom or a monovalent nonmetallic atom group; and A, $R^{21}$, $R^{22}$, and $R^{23}$ may be bonded to one another to form one or more aliphatic or aromatic rings.

In Formula (III), $R^{21}$, $R^{22}$ and $R^{23}$ each independently represent a hydrogen atom or a monovalent nonmetallic atom group. The monovalent nonmetallic atom group represented by $R^{21}$, $R^{22}$ or $R^{23}$ is preferably a substituted or unsubstituted alkyl group, a substituted or unsubstituted aryl group, a substituted or unsubstituted alkenyl group, a substituted or unsubstituted aromatic heterocyclic residue, a substituted or unsubstituted alkoxy group, a substituted or unsubstituted alkylthio group, a hydroxyl group, or a halogen atom.

In the compound represented by Formula (III), Y is preferably an oxygen atom or $=N(R^{23})$ in terms of increasing the efficiency of decomposition of the photopolymerization initiator. $R^{23}$ each independently represent a hydrogen atom or a monovalent nonmetallic atom group. Y is most preferably $=N(R^{23})$.

Preferred examples of the compound represented by Formula (III) include, but are not limited to. In the invention, the isomers caused by the presence of the double bond(s) between the acidic nuclear and the basic nuclear are not explicitly specified, and the structure is not limited to a particular isomer. Accordingly, any isomer may be used in the invention.

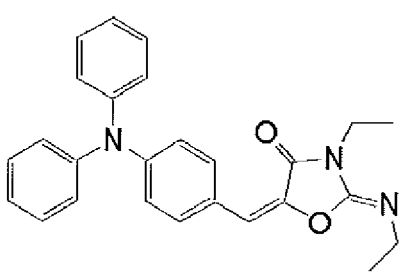

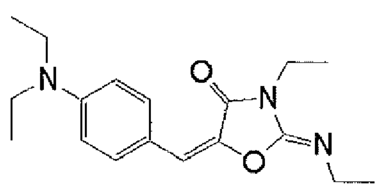

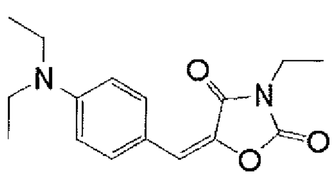

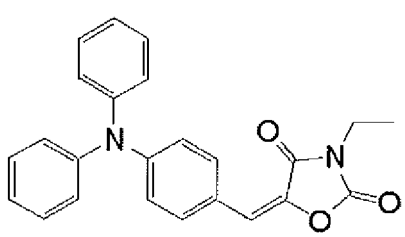

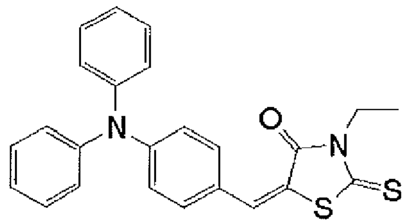
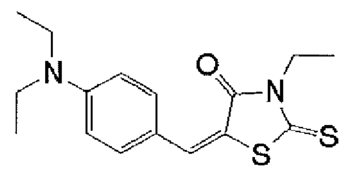

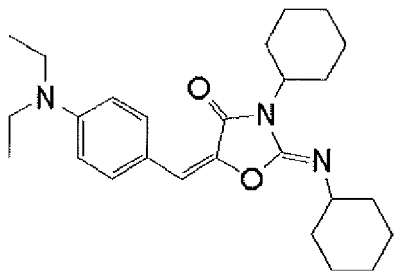
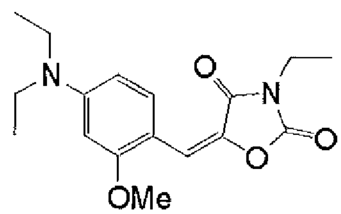

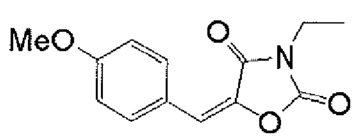
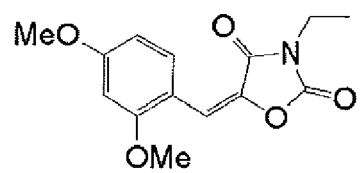

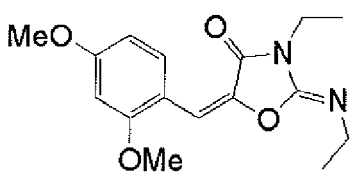
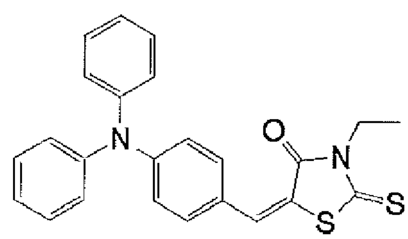

(1)-(F) Co-Sensitizer

The photopolymerizable composition (1) preferably further contains (F) a co-sensitizer.
In the invention, the co-sensitizer has effects of further improving the sensitivity to active radiation of (A) the specific oxime compound or (E) the sensitizer and/or an effect of suppressing inhibition of polymerization of (B) the polymerizable compound caused by oxygen.

Examples of the co-sensitizer include amines such as the compounds described in M. R. Sander et al., Journal of Polymer Society, Vol. 10, p. 3173 (1972), JP-B No. 44-20189, JP-ANos. 51-82102, 52-134692, 59-138205, 60-84305, 62-18537, and 64-33104, and Research Disclosure 33825. Specific examples include triethanolamine, ethyl p-dimethylaminobenzoate, p-formyldimethylaniline, and p-methylthiodimethylaniline.

Other examples of the co-sensitizer include thiols and sulfides such as the thiol compounds described in JP-A No. 53-702, JP-B No. 55-500806 and JP-A No. 05-142772 and the disulfide compounds described in JP-A No. 56-75643, and specific examples include 2-mercaptobenzothiazole, 2-mercaptobenzoxazole, 2-mercaptobenzimidazole, 2-mercapto-4(3H)-quinazoline, and β-mercaptonaphthalene.

Other examples of the co-sensitizer also include amino acid compounds (such as N-phenylglycine), the organometallic compounds described in JP-B No. 48-42965 (such as tributyltin acetate), the hydrogen donators described in JP-B No. 55-34414, and the sulfur compounds (such as trithiane) described in JP-A No. 06-308727.

In order to increase the curing rate with a balance between polymer growth rate and chain transfer, the content

of the co-sensitizer is preferably from 0.1 to 30% by mass, more preferably from 1 to 25% by mass, even more preferably from 1.5 to 20% by mass, based on the mass of the total solids of the photopolymerizable composition (1).

The photopolymerizable composition (1) preferably contains a thiol compound as the co-sensitizer.
The thiol compound that may be contained in the photopolymerizable composition (1) is preferably a compound represented by Formula (IV) below.

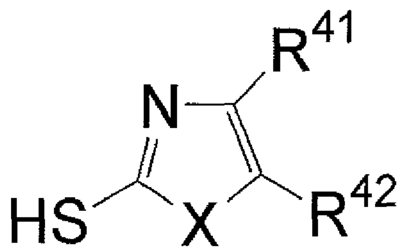

(IV)

In Formula (IV), X represents a sulfur atom, an oxygen atom or -N($R^{43}$)-; $R^{43}$ represents a hydrogen atom, an alkyl group having 1 to 5 carbon atoms or an aryl group having 6 to 13 carbon atoms; $R^{41}$ and $R^{42}$ each independently represent a hydrogen atom, an alkyl group having 1 to 5 carbon atoms, a hydroxyalkyl group having 1 to 3 carbon atoms, a phenyl group optionally substituted by an alkoxy group having 1 to 8 carbon atoms, a nitro group, an alkoxycarbonyl group having an alkyl group having 1 to 8 carbon atoms, a phenoxycarbonyl group, an acetyl group, or a carboxyl group, or $R^{41}$, $R^{42}$ and the double bond therebetween together may form a benzene ring; and the double bond between $R^{41}$ and $R^{42}$ may be hydrogenated.

The thiol compound may be, for example, selected from the compounds described in JP-A No. 53-702, JP-B No. 55-500806, and JP-A No. 05-142772.

In the invention, the thiol compound is preferably a compound represented by Formula (V) below.

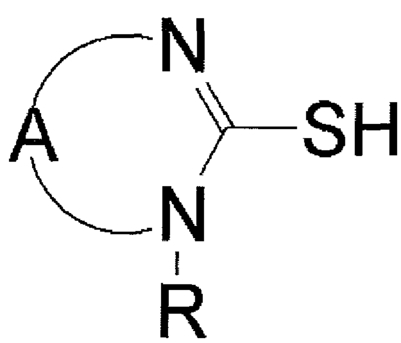

(V)

In Formula (V), R represents an alkyl group or an aryl group, and A represents an atom group that forms a heterocycle together with N=C-N.

In Formula (V), R represents an alkyl group or an aryl group.
The alkyl group may be a linear, branched or cyclic alkyl group having 1 to 20 carbon atoms, more preferably a linear alkyl group having 1 to 12 carbon atoms, a branched alkyl group having 3 to 12 carbon atoms or a cyclic alkyl group having 5 to 10 carbon atoms.
Examples of the alkyl group include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicocyl group, an isopropyl group, an isobutyl group, a sec-butyl group, a tert-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group.

The aryl group may have a monocyclic structure, structure having a condensed ring formed by condensation of 1 to 3 benzene rings, or a structure having a condensed ring formed by condensation of at least one benzene ring and at least one five-membered unsaturated ring. Examples thereof include a phenyl group, a naphthyl group, an anthryl group, a phenanthryl group, an indenyl group, an acenaphthenyl group, and a fluorenyl group. In particular, a phenyl

group or a naphthyl group is more preferred.

The alkyl group or the aryl group may further have at least one substituent. Examples of the substituent that may be introduced include a linear, branched or cyclic alkyl group having 1 to 20 carbon atoms, a linear group, a branched or cyclic alkenyl group having 2 to 20 carbon atoms, an alkynyl group having 2 to 20 carbon atoms, an aryl group having 6 to 20 carbon atoms, an acyloxy group having 1 to 20 carbon atoms, an alkoxycarbonyloxy group having 2 to 20 carbon atoms, an aryloxycarbonyloxy group having 7 to 20 carbon atoms, a carbamoyloxy group having 1 to 20 carbon atoms, a carbonamide group having 1 to 20 carbon atoms, a sulfonamide group having 1 to 20 carbon atoms, a carbamoyl group having 1 to 20 carbon atoms, a sulfamoyl group, a substituted sulfamoyl group having 1 to 20 carbon atoms, an alkoxy group having 1 to 20 carbon atoms, an aryloxy group having 6 to 20 carbon atoms, an aryloxycarbonyl group having 7 to 20 carbon atoms, an alkoxycarbonyl group having 2 to 20 carbon atoms, an N-acylsulfamoyl group having 1 to 20 carbon atoms, an N-sulfamoylcarbamoyl group having 1 to 20 carbon atoms, an alkylsulfonyl group having 1 to 20 carbon atoms, an arylsulfonyl group having 6 to 20 carbon atoms, an alkoxycarbonylamino group having 2 to 20 carbon atoms, an aryloxycarbonylamino group having 7 to 20 carbon atoms, an amino group, a substituted amino group having 1 to 20 carbon atoms, an imino group having 1 to 20 carbon atoms, an ammonio group having 3 to 20 carbon atoms, a carboxyl group, a sulfo group, an oxy group, a mercapto group, an alkylsulfinyl group having 1 to 20 carbon atoms, an arylsulfinyl group having 6 to 20 carbon atoms, an alkylthio group having 1 to 20 carbon atoms, an arylthio group having 6 to 20 carbon atoms, a ureide group having 1 to 20 carbon atoms, a heterocyclic group having 2 to 20 carbon atoms, an acyl group having 1 to 20 carbon atoms, a sulfamoylamino group, a substituted sulfamoylamino group having 1 to 2 carbon atoms, a silyl group having 2 to 20 carbon atoms, an isocyanate group, an isocyanide group, a halogen atom (such as a fluorine, chlorine or bromine atom), a cyano group, a nitro group, and an onium group.

The at least one atom that forms the atom group may include atoms selected from carbon, nitrogen, hydrogen, sulfur, and selenium atoms.

The heterocycle formed by A and N=C-N may further have at least one substituent. Examples of introducible substituents may include the same substituents as those introducible into the above-mentioned alkyl group or aryl group.

In the photopolymerizable composition (1), the thiol compound is more preferably a compound represented by Formula (VI) or (VII) below.

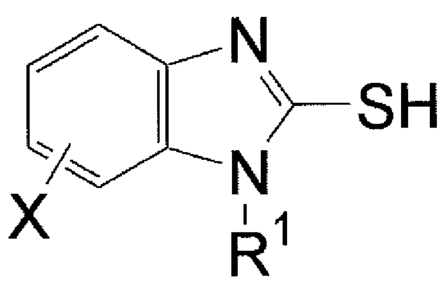

(VI)

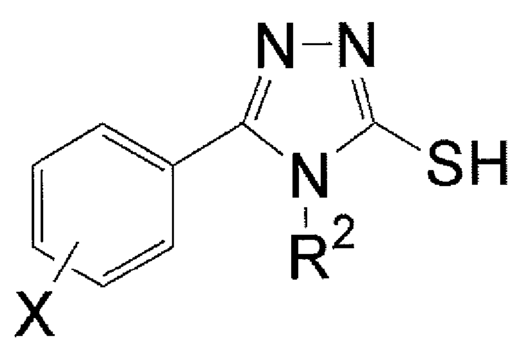

(VII)

In Formula (VI), $R^1$ represents an aryl group, and X represents a hydrogen atom, a halogen atom, an alkoxy group, an alkyl group, or an aryl group.

In Formula (VII), $R^2$ represents an alkyl group or an aryl group, and X represents a hydrogen atom, a halogen atom, an alkoxy group, an alkyl group, or an aryl group.

In Formulae (VI) and (VII), the halogen atom is preferably a fluorine, chlorine, bromine, or iodine atom.

In Formulae (VI) and (VII), the alkoxy group may be a methoxy group, an ethoxy group, a propyloxy group, an isopropyloxy group, a butyloxy group, a pentyloxy group, a hexyloxy group, a dodecyloxy group, a benzyloxy group, an allyloxy group, a phenethyloxy group, a carboxyethyloxy group, a methoxycarbonylethyloxy group, an ethoxycarbonylethyloxy group, a methoxyethoxy group, a phenoxyethoxy group, a methoxyethoxyethoxy group, an ethoxyethoxyethoxy group, a morpholinoethoxy group, a morpholinopropyloxy group, an allyloxyethoxyethoxy group, a phenoxy group, a tolyloxy group, a xylyloxy group, a mesityloxy group, a cumetyloxy group, a methoxyphenyloxy group, an ethoxyphenyloxy group, a chlorophenyloxy group, a bromophenyloxy group, an acetyloxy group, a benzoyloxy group, or a naphthyloxy group.

In Formulae (VI) and (VII), the alkyl group has the same definition as the alkyl group represented by R in Formula (V), and their preferred ranges are also the same.

In Formulae (VI) and (VII), the aryl group has the same definition as the aryl group represented by R in Formula (V), and their preferred ranges are also the same.

In Formulae (VI) and (VII), each group may further have at least one substituent. Examples of the substituent include those mentioned above as substituents introducible to the alkyl or aryl group represented by R in Formula (R).

In Formulae (VI) and (VII), X is preferably a hydrogen atom in view of solubility in PGMEA (propylene glycol monomethylether acetate) that is preferably used for preparation of the photopolymerizable composition.

In Formula (VI), $R^1$ is most preferably a phenyl group in view of sensitivity and solubility in PGMEA.

In Formula (VII), $R^2$ is more preferably a methyl group, an ethyl group, a phenyl group, or a benzyl group in view of sensitivity and solubility in PGMEA.

Among the compounds represented by Formulae (VI) and (VII), the compound represented by Formula (VII) is most preferred in terms of solubility in PGMEA.

Preferred examples of the thiol compound that may be used in an embodiment of the invention include, but are not limited to, the compounds illustrated below together with solubility in PGMEA.

In the description, some chemical formulae are simplified structural formulae in which solid lines represent hydrocarbon groups, unless elements and substituents are explicitly indicated. In the examples below, Me represents a methyl group.

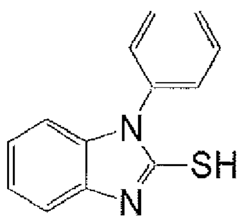

Solubility 20g/L or more

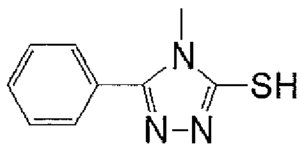

Solubility 20g/L or more

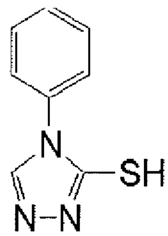

Solubility 20g/L or more

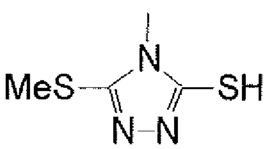

Solubility 20g/L or more

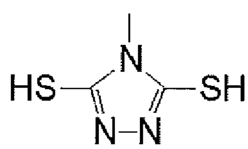

Solubility 20g/L or more

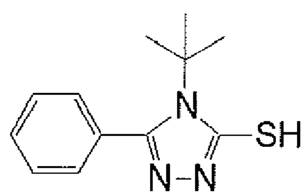

Solubility 20g/L or more

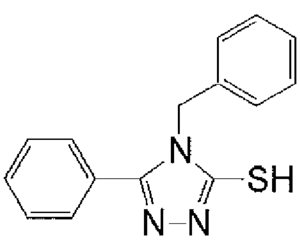

Solubility 20g/L or more

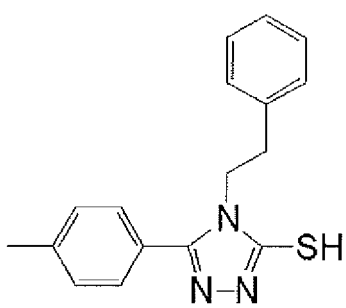

Solubility 20g/L or more

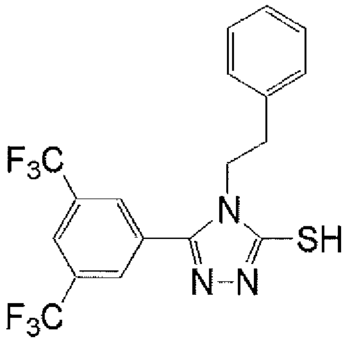

Solubility 20g/L or more

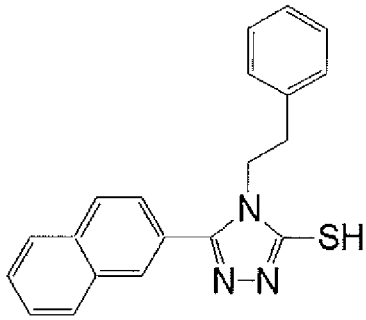

Solubility 20g/L or more

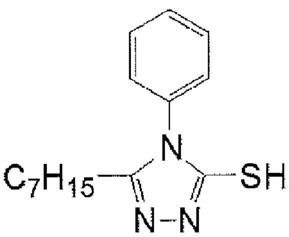

Solubility 20g/L or more

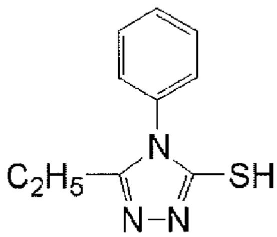

Solubility 20g/L or more

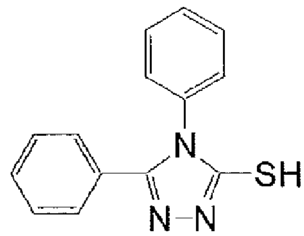

Solubility 20g/L or more

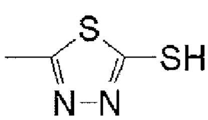

Solubility 20g/L or more

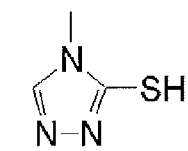

Solubility 20g/L or more

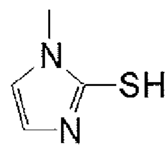

Solubility 20g/L or more

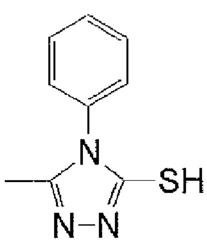

Solubility 20g/L or more

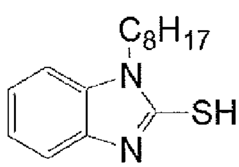

Solubility 20g/L or more

Further, examples of the thiol compound whose solubility in PGMEA is 20 g/L or less or less than 20 g/L are illustrated below.

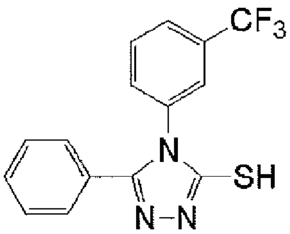

Solubility 20g/L or less

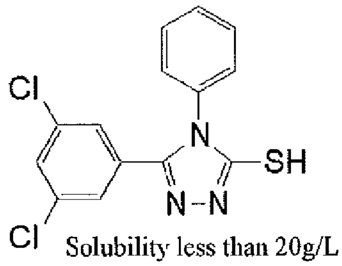

Solubility less than 20g/L

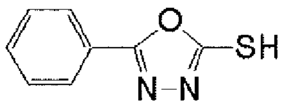

Solubility less than 20g/L

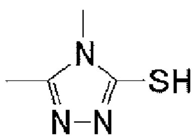

Solubility less than 20g/L

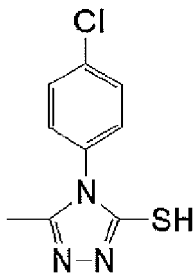

Solubility less than 20g/L

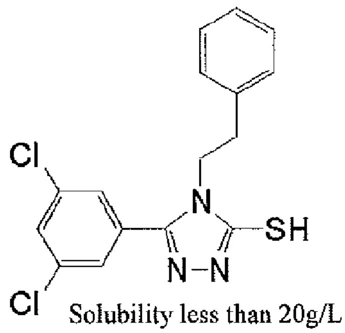

Solubility less than 20g/L

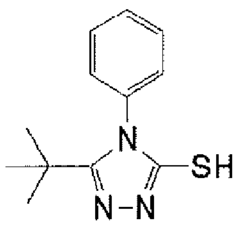

Solubility less than 20g/L

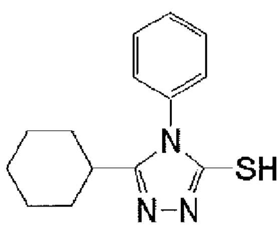

Solubility less than 20g/L

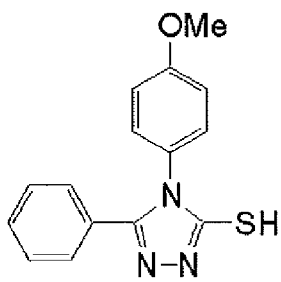

Solubility less than 20g/L

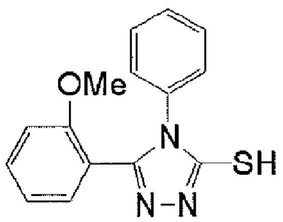

Solubility less than 20g/L

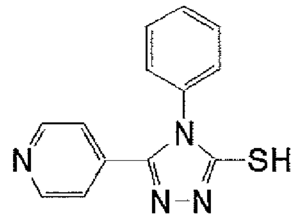

Solubility less than 20g/L

The solubility of these thiol compounds in PGMEA solvent is preferably 20 g/L or more, more preferably from 20 g/L to 50 g/L, even more preferably from 20 g/L to 40 g/L, in view of coating uniformity.

Solubility Measurement Method

In the description, the solubility of the thiol compound is defined as follows.
The specific thiol compound is added to 5 mL of propylene glycol monomethyl ether acetate (PGMEA) solvent and stirred at 25°C for one hour. In this process, the greatest amount of the specific thiol compound that can completely dissolve in the solvent is assumed as the solubility.
These thiol compounds may be synthesized by the method described in J. Appl. Chem., 34, 2203-2207 (1961).
Only a single thiol compound may be used alone, or two or more thiol compounds may be used in combination. When a combination of thiol compounds is used, the thiol compounds may include two or more compounds represented by any one of the abovem-described formulae, or may include compounds respectively represented by different formulae (in an embodiment, for example, at least one compound selected from the class of compounds represented by Formula (VI) may be used in combination with at least one compound selected from the class of compounds represented by Formula (VII)).
When the photopolymerizable composition (1) of the invention contains the thiol compound, the content of the thiol compound is preferably from 0.5 to 30% by mass, more preferably from 1 to 25% by mass, even more preferably from 3 to 20% by mass, based on the mass of the total solids of the photopolymerizable composition (1), in terms of increasing the curing rate with a balance between polymer growth rate and chain transfer.

(1)-(G) Binder Polymer

If necessary, a binder polymer may also be used in the photopolymerizable composition (1) of the invention for the purpose of improving film characteristics and the like. A linear organic polymer is preferably used as the binder. Any known "linear organic polymer" may be used. In order to enable development with water or a weakly alkaline aqueous solution, a linear organic polymer soluble or swellable in water or a weakly alkaline aqueous solution is preferably selected. The linear organic polymer may be selected and used as depending on applications not only as a film-forming agent but also in consideration of the developer such as water, a weakly alkaline aqueous solution or an organic solvent. For example, water development can be performed when a water-soluble organic polymer is used. Examples of such a linear organic polymer include radical polymerization products having a carboxylic acid group in a side chain, such as those described in JP-A No. 59-44615, JP-B Nos. 54-34327, 58-12577 and 54-25957, and JP-A Nos. 54-92723, 59-53836 and 59-71048, specifically, resins produced by homopolymerization or copolymerization of carboxyl group-containing monomers, resins produced by homopolymerization or copolymerization of acid anhydride-containing monomers and subsequent hydrolysis, half-esterification or half-amidation of the acid anhydride units, and epoxy acrylates produced by modification of epoxy reins with unsaturated monocarboxylic acids and/or acid anhydrides. Examples of the carboxyl group-containing monomers include acrylic acid, methacrylic acid, itaconic acid, crotonic acid, maleic acid, fumaric acid, and 4-carboxylstyrene. Examples of the acid anhydride-containing monomers include maleic anhydride.
Examples also include acidic cellulose derivatives having carboxylic acid groups in side chains. Besides the above, a product of addition of a cyclic acid anhydride to a hydroxyl group-containing polymer is also useful.
When the alkali-soluble resin to be used is a copolymer, monomers other than the above monomers may also be used as the compounds to be copolymerized. Examples of other monomers include the following compounds of (1) to (12):
(1) aliphatic hydroxyl group-containing acrylates and methacrylates such as 2-hydroxyethyl acrylate, 2-hydroxypropyl acrylate, 3-hydroxypropyl acrylate, 4-hydroxybutyl acrylate, 2-hydroxyethyl methacrylate, 2-hydroxypropyl methacrylate, 3-hydroxypropyl methacrylate, and 4-hydroxybutyl methacrylate;

(2) alkyl acrylates such as methyl acrylate, ethyl acrylate, propyl acrylate, butyl acrylate, isobutyl acrylate, amyl acrylate, hexyl acrylate, 2-ethylhexyl acrylate, octyl acrylate, benzyl acrylate, 2-chloroethyl acrylate, glycidyl acrylate, 3,4-epoxycyclohexylmethyl acrylate, vinyl acrylate, 2-phenylviyl acrylate, 1-propenyl acrylate, allyl acrylate, 2-allyloxyethyl acrylate, and propargyl acrylate;

(3) alkyl methacrylates such as methyl methacrylate, ethyl methacrylate, propyl methacrylate, butyl methacrylate, isobutyl methacrylate, amyl methacrylate, hexyl methacrylate, 2-ethylhexyl methacrylate, cyclohexyl methacrylate, benzyl methacrylate, 2-chloroethyl methacrylate, glycidyl methacrylate, 3,4-epoxycyclohexylmethyl methacrylate, vinyl methacrylate, 2-phenylvinyl methacrylate, 1-propenyl methacrylate, allyl methacrylate, 2-allyloxyethyl methacrylate, and propargyl methacrylate;

(4) acrylamides and methacrylamides such as acrylamide, methacrylamide, N-methylolacrylamide, N-ethylacrylamide, N-hexylmethacrylamide, N-cyclohexylacrylamide, N-hydroxyethylacrylamide, N-phenylacrylamide, N-nitrophenylacrylamide, N-ethyl-N-phenylacrylamide, vinyl acrylamide, vinyl methacrylamide, N,N-diallylacrylamide, N, N-diallylmethacrylamide, allylacrylamide, and allylmethacrylamide;

(5) vinyl ethers such as ethyl vinyl ether, 2-chloroethyl vinyl ether, hydroxyethyl vinyl ether, propyl vinyl ether, butyl vinyl ether, octyl vinyl ether, and phenyl vinyl ether;

(6) vinyl esters such as vinyl acetate, vinyl chloroacetate, vinyl butyrate, and vinyl benzoate;
(7) styrenes such as styrene, $\alpha$-methylstyrene, methylstyrene, chloromethylstyrene, and p-acetoxystyrene;
(8) vinyl ketones such as methyl vinyl ketone, ethyl vinyl ketone, propyl vinyl ketone, and phenyl vinyl ketone;
(9) olefins such as ethylene, propylene, isobutylene, butadiene, and isoprene;

(10) N-vinylpyrrolidone, acrylonitrile, methacrylonitrile, and the like;

(11) unsaturated imides such as maleimide, N-acryloylacrylamide, N-acetylmethacrylamide, N-propionylmethacrytamide, and N-(p-chlorobenzoyl)methacrylamide; and
(12) methacrylic acid monomers having a hetero atom at the $\alpha$-position, such as the compounds described in Japanese Patent Application Nos. 2001-115595 and 2001-115598.

Particularly preferred are (meth)acrylic resins having an allyl or vinyl ester group and a carboxyl group in a side chain, the alkali-soluble resins having a double bond in a side chain described in JP-A Nos. 2000-187322 and 2002-62698, and the alkali-soluble resins having an amide group in a side chain described in JP-A No. 2001-242612, in view of excellent balance between film strength, sensitivity and developability.
The acid group-containing urethane binder polymers described in JP-B Nos. 07-12004, 07-120041, 07-120042, and 08-12424, JP-A Nos. 63-287944, 63-287947 and 01-271741, and Japanese Patent Application No. 10-116232 and the urethane binder polymers having acid groups and double bonds in side chains described in JP-A No. 2002-107918 are advantageous in terms of printing durability or low exposure properties, because they have very high strength.
The acid group-containing, acetal-modified, polyvinyl alcohol binder polymers such as those described in European Patent Nos. 993966 and 1204000 and JP-A No. 2001-318463 are also preferred, because they have an excellent balance between film strength and developability.
Useful water-soluble linear organic polymers also include polyvinyl pyrrolidone and polyethylene oxide. Alcohol-soluble nylon or polyether of 2,2-bis-(4-hydroxyphenyl)-propane and epichlorohydrin is also useful for increasing the strength of the cured film.
Binder polymers that may be used in the photopolymerizable composition (1) preferably have a weight average molecular weight of 5,000 or more, more preferably of 10,000 to 300,000, and preferably have a number average molecular weight of 1,000 or more, more preferably of 2,000 to 250,000. The polydispersity (weight average molecular weight/number average molecular weight) is preferably 1 or more, more preferably from 1.1 to 10.
These binder polymers may be any of random, block or graft polymers.
Binder polymers that may be used in the invention may be synthesized by known conventional methods. Examples of solvents that may be used for the synthesis include tetrahydrofuran, ethylene dichloride, cyclohexanone, methyl ethyl ketone, acetone, methanol, ethanol, ethylene glycol monomethyl ether, ethylene glycol monoethyl ether, 2-methoxyethyl acetate, diethylene glycol dimethyl ether, 1-methoxy-2-propanol, 1-methoxy-2-propyl acetate, N,N-dimethylformamide, N,N-dimethylacetamide, toluene, ethyl acetate, methyl lactate, ethyl lactate, dimethylsulfoxide, and water. Only one solvent may be used, or a mixture of two or more solvents may be used.

In the process of synthesizing binder polymers that may be used in the photopolymerizable composition (1), known compounds such as azo initiators and peroxide initiators may be used as radical-polymerization initiators.

(1)-(H) Polymerization Inhibitor

In the photopolymerizable composition (1), a small amount of a thermal polymerization inhibitor is preferably added in order to inhibit unnecessary thermal polymerization of (B) the polymerizable compound during the production or storage of the curable composition.
Examples of the thermal polymerization inhibitor that may be used in the invention include hydroquinone, p-methoxyphenol, di-tert-butyl-p-cresol, pyrogallol, tert-butylcatechol, benzoquinone, 4,4'-thiobis(3-methyl-6-tert-butylphenol), 2,2'-methylenebis(4-methyl-6-tert-butylphenol), and cerous N-nitrosophenyl-hydroxylamine.
The content of the thermal polymerization inhibitor is preferably from about 0.01 to about 5% by mass, based on the mass of the total solids of the photopolymerizable composition (1).
If necessary, behenic acid or a higher fatty acid derivative such as behenic acid amide may be added to prevent oxygen-induced inhibition of polymerization and may be localized to the surface of a coating film during a drying process after coating. The content of the higher fatty acid derivative is preferably from about 0.5 to about 10% by mass based on the entire composition.

(1)-(I) Adhesion Improving Agent

The photopolymerizable composition (1) may contain an adhesion improving agent for increasing adhesion to a hard surface, such as of a support. The adhesion improving agent may be a silane coupling agent, a titanium coupling agent or the like.
Examples of the silane coupling agent include
γ-(2-aminoethyl)aminopropyltrimethoxysilane,
γ-(2-aminoethyl)aminopropyldimethoxysilane,
β-(3,4-epoxycyclohexyl)ethyltrimethoxysilane, γ-aminopropyltrimethoxysilane,
γ-aminopropyltriethoxysitane, γ-methactyloxypropyltrimethoxysilane,
γ-methacryloxypropyltriethoxysilane, γ-acryloxypropyltrimethoxysilane,
γ-acryloxypropyltriethoxysilane, γ-isocyanatopropyltrimethoxysilane,
γ-isocyanatopropyltriethoxysilane,
N-β-(N-vinylbenzylaminoethyl)-γ-aminopropyltrimethoxysilane hydrochloride,
γ-glycidoxypropyltrimethoxysilane, γ-glycidoxypropyltriethoxysilane, aminosilane,
γ-mercaptopropyltrimethoxysilane, γ-mercaptopropyltriethoxysilane, methyltrimethoxysilane, methyltriethoxysilane, vinyltriacetoxysilane,
γ-chloropropyltrimethoxysilane, hexamethyldisilazane, γ-anilinopropyltrimethoxysilane, vinyltrimethoxysilane, vinyltriethoxysilane, vinyltris(β-methoxyethoxy)silane, octadecyldimethyl[3-(trimetboxysilyl)propyl]ammonium chloride,
γ-chloropropylmethyldimethoxysilane, γ-mercaptopropylmethyldimethoxysilane, methyltrichlorosilane, dimethyldichlorosilane, trimethylchlorosilane, 2-(3,4-epoxycyclohexyl)ethyltrimethoxysilane, bisallyltrimethoxysilane, tetraethoxysilane, bis(trimethoxysilyl)hexane, phenyltrimethoxysilane,
N-(3-actyloxy-2-hydroxypropyl)-3-aminopropyltriethoxysilane,
N-(3-methacryloxy-2-hydroxypropyl)-3-aminopropyltriethoxysilane, (methacryloxymethyl)methyldiethoxysilane, and (acryloxymethyl)methyldimethoxysilane.
In particular, γ-methacryloxypropyltrimethoxysilane, γ-methacryloxypropyltriethoxysilane, γ-acryloxypropyltrimethoxysilane, γ-acryloxypropyltriethoxysilane, γ-mercaptopropyltrimethoxysilane, γ-aminopropyltriethoxysilane, and phenyltrimethoxysilane are preferred, and γ-methacryloxypropyltrimethoxysilane is most preferred.
The content of the adhesion improving agent is preferably from 0.5 to 30% by mass, more preferably from 0.7 to 20% by mass, based on the mass of the total solids of the photopolymerizable composition (1).

(1)-(I) Diluent

Any of various organic solvents may be used as a diluent for the photopolymerizable composition (1).
Examples of the organic solvent that may be used include acetone, methyl ethyl ketone, cyclohexane, ethyl acetate, ethylene dichloride, tetrahydrofuran, toluene, ethylene glycol monomethyl ether, ethylene glycol monoethyl ether, ethylene glycol dimethyl ether, propylene glycol monomethyl ether, propylene glycol monoethyl ether, acetyl acetone, cyclohexanone, diacetone alcohol, ethylene glycol monomethyl ether acetate, ethylene glycol ethyl ether acetate, ethylene glycol monoisopropyl ether, ethylene glycol monobutyl ether acetate, 3-methoxypropanol, methoxymethoxyethanol, diethylene glycol monomethyl ether, diethylene glycol monoethyl ether, diethylene glycol dimethyl ether, diethylene

glycol diethyl ether, propylene glycol monomethyl ether acetate, propylene glycol monoethyl ether acetate, 3-methoxypropyl acetate, N,N-dimethylformamide, dimethylsulfoxide, γ-butyrolactone, methyl lactate, and ethyl lactate.
Only a single solvent may be used, or a mixture of two or more solvents may be used. The solids content relative to the organic solvent is preferably from 2 to 60% by mass.

(1)-(K) Other Additives

In addition, known additives for modifying the physical properties of cured films, such as an inorganic filler, a plasticizer and a lipophilizing agent, may also be added to the photopolymerizable composition (1).
Examples of the plasticizer include dioctyl phthalate, didodecyl phthalate, triethylene glycol dicaprylate, dimethyl glycol phthalate, tricresyl phosphate, dioctyl adipate, dibutyl sebacate, and triacetylglycerol. When a binder is used, the plasticizer may be added in an amount of 10% by mass or less, based on the total mass of the polymerizable compound and the binder polymer.
As described above, the photopolymerizable composition (1) contains the specific oxime compound (A), and is thus capable of curing with high sensitivity, is excellent in storage stability, and is capable of preventing coloration with time during heating. The photopolymerizable composition (1), when applied onto the surface of a rigid material and cured thereon, shows high adhesiveness to the surface.
The photopolymerizable composition (1) containing the specific oxime compound (A), the polymerizable compound (B), and the colorant (C), is used preferably as a photopolymerizable composition for color filter (the photopolymerizable composition for color filter of the invention).

Photopolymerizable Composition (2)

(2)-(A) Specific Oxime Compound

The specific oxime compound in the photopolymerizable composition (2) may function as a polymerization initiator.
The content of the specific oxime compound in the photopolymerizable composition (1) is preferably from 0.5 to 40% by mass, more preferably from 1 to 35% by mass, even more preferably from 1.5 to 30% by mass, base on the mass of the total solids of the composition.
The specific oxime compound may used alone or in combination of two or more.
Besides the specific oxime compound, another known polymerization initiator may also be used in the photopolymerizable composition (2) in such a range that the effect of the invention is not impaired.
Examples of another polymerization initiator include (a) aromatic ketones, (b) an aromatic onium salt compound, (c) an organic peroxide, (d) a thio compound, (e) a hexaarylbiimidazole compound, (f) a ketooxime ester compound, (g) a borate compound, (h) an azinium compound, (i) a metallocene compound, (j) an active ester compound, and (k) a compound having a carbon-halogen bond. More specific examples include polymerization initiators described in paragraphs [0081] to [0139] in JP-A No. 2006-78749, etc.

(2)-(B) Polymerizable Compound

Examples of (B) a polymerizable compound that may be contained in the photopolymerizable composition (2) include the addition-polymerizable compounds described above for the curable composition or the photopolymerizable composition (1).
Details of how to use addition-polymerizable compounds, such as what structure should be used, whether they should be used alone or in combination, or what amount should be added, may be freely determined depending on the final performance design of the photosensitive material. For example, they may be selected from the following viewpoints. In view of photosensitive speed, a structure having a higher content of unsaturated group per molecule is preferable, and di- or higher-functional structures are preferred in many cases. In order to increase the strength of the image portion, namely the cured film, tri- or higher-functional structures are preferred. A method of using a combination of compounds having different numbers of functional groups and/or different types of polymerizable groups (for example, compounds selected from an acrylic ester, a methacrylic ester, a styrene compound, a vinyl ether compound) is also effective for controlling both of photosensitivity and strength. High-molecular-weight compounds or highly hydrophobic compounds can have high sensitive speed or form high-strength films but are not preferred in some cases in view of development speed or precipitation in a developing solution.
How to select and use the addition-polymerizable compound is also an important factor for the compatibility with or dispersibility to other components of the photosensitive layer (such as the binder polymer, the initiator and the colorant). For example, in some cases, the compatibility may be improved by using a low-purity compound or by using

a combination of two or more compounds. A particular structure may also be selected in order to improve adhesion to a support, an overcoat layer or the like. A higher content of the addition-polymerizable compound in the photosensitive layer is advantageous in terms of sensitivity. If the content is too high, however, undesirable phase separation, manufacturing process problems due to the tackiness of the photosensitive layer (such as manufacturing failures resulting from transfer or adhesion of the photosensitive material components), precipitation from a developing solution, and the like may occur.

From these points of view, the content of the addition-polymerizable compound is preferably from 5 to 80% by mass, more preferably from 25 to 75% by mass, base on the mass of the total solids of the photopolymerizable composition (2).

Only a single addition-polymerizable compound may be used, or two or more addition-polymerizable compounds may be used in combination. Concerning how to use the addition-polymerizable compounds, an appropriate structure, composition or content may also be freely selected in consideration of the degree of inhibition of polymerization caused by oxygen, resolution, fogging properties, changes in refractive index, surface stickiness, or other points of view. In some cases, a layered structure or coating method using an undercoat or an overcoat may also be performed.

(2)-(C) Binder Polymer

The photopolymerizable composition (2) preferably contains a binder polymer. The binder polymer may be contained in terms of improving film characteristics. Any binder polymer having the function of improving film characteristics may be used.

A linear organic high-molecular weight polymer is preferably contained as the binder polymer. Such a linear organic high-molecular-weight polymer to be used is not particularly limited, and may be any linear organic polymer. In order to enable development with water or a weakly alkaline aqueous solution, a linear organic high-molecular-weight polymer soluble or swellable in water or a weakly alkaline aqueous solution is preferably selected. The linear organic high-molecular-weight polymer may be selected and used not only as a film-forming agent for the photopolymerizable composition but also in consideration of the specifications of the developer such as water, a weakly alkaline aqueous solution or an organic solvent. For example, water development can be performed when a water-soluble organic high-molecular-weight polymer is used.

Examples of such a linear organic high-molecular-weight polymer include addition polymerization products having a carboxylic acid group in a side chain, such as those described in JP-A No. 59-44615, JP-B Nos. 54-34327, 58-12577 and 54-25957, and JP-A Nos. 54-92723, 59-53836 and 59-71048, specifically, methacrylic acid copolymers, acrylic acid copolymers, itaconic acid copolymers, crotonic acid copolymers, maleic acid copolymers, and partially-esterified maleic acid copolymers.

Examples also include acidic cellulose derivatives having a carboxylic acid group in a side chain. Besides the above, a product of an addition of a cyclic acid anhydride to a hydroxyl group-containing addition polymer is also useful.

In particular, copolymers of benzyl (meth)acrylate and (meth)acrylic acid and optionally any other addition-polymerizable vinyl monomer; and copolymers of allyl (meth)acrylate and (meth)acrylic acid and optionally any other addition-polymerizable vinyl monomer are preferred, because they have an excellent balance between film strength, sensitivity and developability.

An amide bond-containing binder polymer or urethane bond-containing binder polymer may also be contained. The amide bond- or urethane bond-containing binder polymer is preferably an amide bond- or urethane bond-containing linear organic high-molecular-weight polymer. Such an amide bond- or urethane bond-containing linear organic high-molecular-weight polymer may be of any type. In order to enable development with water or a weakly alkaline aqueous solution, an amide bond- or urethane bond-containing linear high-molecular-weight organic polymer soluble or swellable in water or a weakly alkaline aqueous solution is preferably selected. The amide bond- or urethane bond-containing linear organic high-molecular-weight polymer may be selected and used depending on applications not only as a film-forming agent but also in consideration of the developer such as water, a weakly alkaline aqueous solution or an organic solvent. For example, water development can be performed when a water-soluble high-molecular-weight organic polymer is used. The amide group-containing binder described in JP-A No. 11-171907 is preferably used as the amide bond- or urethane bond-containing linear organic high-molecular-weight polymer, because it has both excellent developability and high film strength.

The acid group-containing urethane binder polymers described in JP-B Nos. 07-120040, 07-120041, 07-120042, and 08-12424, JP-A Nos. 63-287944, 63-287947 and 01-271741, and Japanese Patent Application No. 10-116232 are advantageous in terms of printing durability or low exposure properties, because they have very high strength. The amide group-containing binder described in JP-A No. 11-171907 is also preferred, because it has both excellent developability and high film strength.

Useful water-soluble linear organic high-molecular-weight polymers also include polyvinyl pyrrolidone and polyethylene oxide. Alcohol-soluble nylon or polyether of 2,2-bis-(4-hydroxyphenyl)-propane and epichlorohydrin is also

useful for increasing the strength of cured films.

Any amount of the binder polymer may be mixed into the photopolymerizable composition (2). In view of image strength or the like, the content of the binder polymer is preferably from 30 to 85% by mass, base on the mass of the total solids content of the photosensitive layer. The mass ratio of the addition-polymerizable compound to the binder polymer is preferably in the range of from 1/9 to 7/3.

In a preferred exemplary embodiment, the binder polymer to be used is substantially water-insoluble but alkali-soluble. This can eliminate the use of an environmentally-unfriendly organic solvent for a developer or limit the used amount to a very low level. In such a method of use, the acid value (the acid content per g of polymer expressed in chemical equivalent numbers) and the molecular weight of the binder polymer may be appropriately selected in consideration of image strength and developability. The acid value is preferably from 0.4 to 3.0 meq/g, more preferably from 0.6 to 2.0 meq/g, and the molecular weight is preferably from 3,000 to 500,000, more preferably from 10,000 to 300,000.

(2)-(D) Sensitizer

The photopolymerizable composition (2) preferably contains a sensitizer together with the specific oxime compound (A) or the like as a polymerization initiator. Examples of the sensitizer that may be used in the invention include spectral sensitizing dyes and pigments or dyes capable of interacting with polymerization initiators upon absorption of light from a light source.

Examples of preferred spectral sensitizing dyes or pigments include polynuclear aromatic compounds (such as pyrene, perylene and triphenylene), xanthenes (such as fluorescein, eosin, erythrosine, rhodamine B, and rose bengal), cyanines (such as thiacarbocyanine and oxacarbocyanine), melocyanines (such as melocyanine and carbomelocyanine), thiazines (such as thionine, methylene blue and toluidine blue), acridines (such as acridine orange, chloroflavin, and acriflavin), phthalocyanines (such as phthalocyanine and metal phthalocyanine), porphyrins (such as tetraphenylporphyrin and center metal-substituted porphyrin), chlorophylls (such as chlorophyll, chlorophyllin and center metal-substituted chlorophyll), metal complexes (such as the compound shown below), anthraquinones (such as anthraquinone), and squaryliums (such as squarylium).

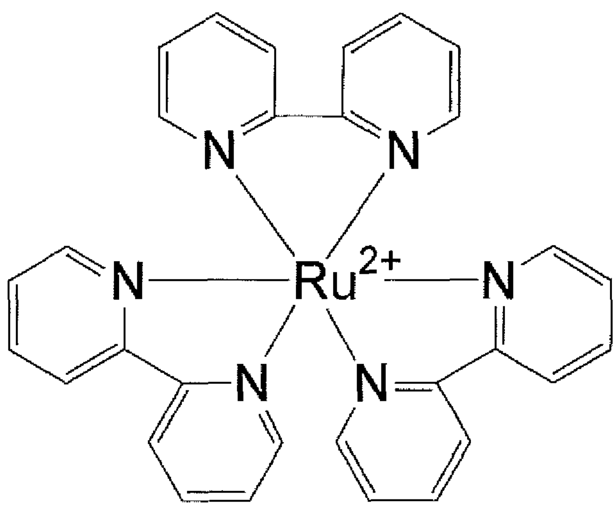

Examples of more preferred spectral sensitizing dyes or pigments include the compounds described in Paragraphs [0144] to [0202] of JP-A No. 2006-78749.

Examples of sensitizers that may be used for the photopolymerizable composition (2) also include those described above for the curable composition or the photopolymerizable composition (1).

Only one sensitizer may be used, or two or more sensitizers may be used in combination. In the photopolymerizable composition (2), the molar ratio of all the polymerization initiator(s) to the sensitizing dye (all the polymerization initiator(s) : sensitizing dye) may be from 100:0 to 1:99, more preferably from 90:10 to 10:90, most preferably from 80:20 to 20:80.

(2)-(E) Co-Sensitizer

A known compound having the effect of further improving the sensitivity or the effect of suppressing inhibition of polymerization caused by oxygen may be added as a co-sensitizer to the photopolymerizable composition (2).
Examples of the co-sensitizer also include those described above for the curable composition or the photopolymerizable composition (1). Other examples include the phosphorus compounds (diethyl phosphite and the like) described in JP-A No. 06-250387 and the Si-H and Ge-H compounds described in Japanese Patent Application No. 06-191605.
When the co-sensitizer is used, the amount of the co-sensitizer may appropriately be from 0.01 to 50 parts by mass, based on 1 part by mass of the polymerization initiator in the photopolymerizable composition (2).

(2)-(F) Polymerization Inhibitor

A small amount of a thermal polymerization inhibitor is preferably added to the photopolymerizable composition (2) in order to inhibit unnecessary thermal polymerization of the compound containing a polymerizable ethylenic unsaturated double bond during the production or storage of the composition. Examples of the thermal polymerization inhibitor include hydroquinone, p-methoxyphenol, di-tert-butyl-p-cresol, pyrogallol, tert-butylcatechol, benzoquinone, 4,4'-thiobis (3-methyl-6-tert-butylphenol), 2,2'-methylenebis(4-methyl-6-tert-butylphenol), and cerous N-nitrosophenyl-hydroxylamine.
The content of the thermal polymerization inhibitor is preferably from about 0.01 to about 5% by mass, based on the mass of the composition.
If necessary, behenic acid or a higher fatty acid derivative such as behenic acid amide may be added to prevent oxygen-induced inhibition of polymerization and may be localized to the surface of the photosensitive layer in a drying process after coating. The content of the higher fatty acid derivative is preferably from about 0.5 to about 10% by mass of the entire composition.

(2)-(G) Colorant and the like

In order to form a colored photosensitive layer, a dye or a pigment may be added to the composition. This allows an improvement in the so-called plate checking capability of a printing plate, such as the visibility of the photosensitive layer after plate making and suitability for measurements with image density measuring machines. Many dyes used as colorants can reduce the sensitivity of the photopolymerizable photosensitive layer. In particular, therefore, pigments are preferably used as colorants. Examples of colorants include pigments such as phthalocyanine pigments, azo pigments, carbon blacks, and titanium oxide; and dyes such as ethyl violet, crystal violet, azo dyes, anthraquinone dyes, and cyanine dyes. The content of the dye or the pigment is preferably from about 0.5 to about 5% by mass of the entire composition.

(2)-(H) Other Additives

In addition, known additives such as an inorganic filler or plasticizer for modifying the physical properties of the cured film, and lipophilizing agents capable of improving the fixation of inks on the surface of photosensitive layers, may also be added to the composition.
Examples of the plasticizer include dioctyl phthalate, didodecyl phthalate, triethylene glycol dicaprylate, dimethyl glycol phthalate, tricresyl phosphate, dioctyl adipate, dibutyl sebacate, and triacetylglycerol. When a binder is used, the plasticizer may be added in an amount of 10% by mass or less, based on the total mass of the ethylenic unsaturated double bond-containing compound and the binder.
In order to improve the film strength (printing durability), a UV initiator or a thermal crosslinking agent for enhancing the effect of heating and/or exposure after development may also be added to the composition.

<Color Filter and Methods for Production Thereof>

A description is given blow of the color filter and the method for producing a color filter according to the invention. The color filter of the invention includes a colored pattern provided on a support, the colored pattern being produced by using the photopolymerizable composition for a color filter of the invention.
The color filter of the invention is described in detail below together with the method for production thereof includes applying the photopolymerizable composition (1) for a color filter of the invention to a support to form a colored photopolymerizable composition layer (hereinafter also simply referred to as "the colored photopolymerizable composition layer-forming step"); exposing the colored photopolymerizable composition layer to light through a mask (hereinafter

also simply referred to as "the exposure step"); and developing the exposed composition layer to form a colored pattern (hereinafter also simply referred to as "the development step").

Specifically, the photopolymerizable composition for a color filter of the invention is applied to a support (substrate) directly or with another layer interposed therebetween to form a photopolymerizable composition layer (the colored photopolymerizable composition layer-forming step); the coating film is exposed to light through a specific patterned mask to cure the irradiated portion of the coating film (the exposure step); and the coating film is developed with a developing solution to form a patterned film including pixels of the respective colors (three or four colors) so that a color filter of the invention is produced.

Each step of the method of the invention for producing a color filter is described below.

Colored Photopolymerizable Composition Layer-Forming Step

In the colored photopolymerizable composition layer forming step, the photopolymerizable composition for a color filter of the invention is applied to a support to form a colored photopolymerizable composition layer.

Examples of the support that may be used in this step include soda glass substrates, PYREX (registered trademark) glass substrates, quartz glass substrates, and a substrate obtained by attaching a transparent electrically-conductive film onto any of the above glass substrates, which are for use in liquid crystal displays and the like, and photoelectric converting substrates for use in imaging devices, such as silicon substrates and complementary metal oxide film semiconductors (CMOS). In some cases, these substrates may have black stripes for separating pixels from one another.

If necessary, an undercoat layer may be formed on the support in order to improve adhesion to the upper layer, prevent the substance diffusion or flatten the substrate surface.

The photopolymerizable composition for a color filter of the invention may be applied to the support by various coating methods such as slit coating, inkjet method, spin coating, cast coating, roll coating, and screen printing.

The thickness of the coating film of the photopolymerizable composition for a color filter is preferably from 0.1 μm to 10 μm, more preferably from 0.2 μm to 5 μm, even more preferably from 0.2 μm to 3 μm.

When producing a color filter for use in a solid-state imaging device, the thickness of the coating film of the photopolymerizable composition for a color filter is preferably from 0.35 μm to 1.5 μm, more preferably from 0.40 μm to 1.0 μm, in view of resolution and developability.

The coating of the photopolymerizable composition for a color filter on the support is generally dried under the conditions of 70 to 110˚C for about 2 to 4 minutes to form a colored photopolymerizable composition layer.

Exposure Step

In the exposure step, the colored photopolymerizable composition layer formed in the colored photopolymerizable composition layer forming step is exposed to light through a mask so that only the irradiated portion of the coating film is cured.

The exposure is preferably performed by irradiation with a radiation. In particular, ultraviolet rays such as g-line or i-line is preferably used as the radiation for exposure, and high-pressure mercury lamps are more preferred. The irradiation intensity is preferably from 5 $mJ/cm^2$ to 1500 $mJ/cm^2$, more preferably from 10 $mJ/cm^2$ to 1000 $mJ/cm^2$, most preferably from 10 $mJ/cm^2$ to 800 $mJ/cm^2$.

Development Step

After the exposure step, alkali development (the development step) may be performed so that the unexposed portion resulting from the exposure step can be dissolved in an aqueous alkali solution. In this step, only the photocured portion is left.

The developing solution is preferably an organic alkali developer that does not damage underlying circuits or the like. The development is generally performed at a temperature of 20˚C to 30˚C for a time period of 20 to 90 seconds.

Examples of the alkali for use in the developing solution include ammonia water and organic alkaline compounds such as ethylamine, diethylamine, dimethylethanolamine, tetramethylammonium hydroxide, tetraethylammonium hydroxide, choline, pyrrole, piperidine, and 1,8-diazabicyclo-[5,4,0]-7-undecene. An aqueous alkaline solution prepared by diluting the alkali with pure water to a concentration of 0.001 to 10% by mass, preferably of 0.01 to 1% by mass may be used. When a developing solution comprising such an aqueous alkaline solution is used, washing (rinsing) with pure water is generally performed after the development.

If necessary, the method of the invention for producing a color filter may further include the step of curing the colored pattern by heating and/or exposure to light after the photopolymerizable composition layer-forming step, the exposure step and the development step have been conducted.

The colored photopolymerizable composition layer-forming step, the exposure step and the development step (and optionally the curing step) may be repeated for the number of times corresponding to the number of the desired hues, whereby a color filter of the desired hues may be produced.

<Solid-state imaging device>

The solid-state imaging device of the invention includes the color filter of the invention.
Because the color filter of the invention uses the photopolymerizable composition for color filter of the invention, a colored pattern formed therefrom exhibits high adhesiveness to a support (substrate), and the cured composition is excellent in resistance to development and is thus excellent in exposure sensitivity, is superior in adhesiveness to a substrate in exposed portions and may form a pattern of high resolution to give a desired sectional shape. Accordingly, the color filter of the invention may be used in a liquid crystal display device and a solid state imaging device such as CCD, and is particularly suitable for CCD devices and CMOS of high resolution of one million or more pixels. That is, the color filter of the invention is preferably applied to a solid imaging device.
The color filter of the invention may be used as a color filter arranged between a light-receiving part of each pixel and a light-collecting micro-lens, which constitutes CCD.

(Planographic Printing Plate precursor)

A description is given below of the planographic printing plate precursor of the invention.
The planographic printing plate precursor includes a support and a photosensitive layer that is placed on the support and contains the photopolymerizable composition of the invention.
If necessary, the planographic printing plate precursor may further include any other layer such as a protective layer and an intermediate layer. Since the planographic printing plate precursor contains the photopolymerizable composition of the invention in the photosensitive layer and thus have a high sensitivity, stability over time and printing durability.
Each element of the planographic printing plate precursor of the invention is described below.

Photosensitive Layer

The photosensitive layer is a layer containing the photopolymerizable composition of the invention. Specifically, the photosensitive layer may be formed by a process that includes using the photopolymerizable composition (2) (a preferred example of the photopolymerizable composition of the invention) as a composition for forming a photosensitive layer (hereinafter also referred to as "the photosensitive layer-forming composition"), applying a coating liquid containing the composition to a support and dying the coating to form a photosensitive layer.
For the application of the photosensitive layer-forming composition to the support, the respective components to be contained in the composition may be used in the form of solutions in various organic solvents. Examples of solvents that may be used in this process include acetone, methyl ethyl ketone, cyclohexane, ethyl acetate, ethylene dichloride, tetrahydrofuran, toluene, ethylene glycol monomethyl ether, ethylene glycol monoethyl ether, ethylene glycol dimethyl ether, propylene glycol monomethyl ether, propylene glycol monoethyl ether, acetyl acetone, cyclohexanone, diacetone alcohol, ethylene glycol monomethyl ether acetate, ethylene glycol ethyl ether acetate, ethylene glycol monoisopropyl ether, ethylene glycol monobutyl ether acetate, 3-methoxypropanol, methoxymethoxyethanol, diethylene glycol monomethyl ether, diethylene glycol monoethyl ether, diethylene glycol dimethyl ether, diethylene glycol diethyl ether, propylene glycol monomethyl ether acetate, propylene glycol monoethyl ether acetate, 3-methoxypropyl acetate, N,N-dimethylformamide, dimethylsulfoxide, γ-butyrolactone, methyl lactate, and ethyl lactate. Only a single solvent may be used, or a mixture of two or more solvents may be used. The coating solution appropriately has a solids content of 2 to 50% by mass.
The coating amount of the photosensitive layer on the support is preferably selected as appropriate depending on applications, because it may have effects on the sensitivity and/or developability of the photosensitive layer and on the strength and/or printing durability of the exposed film. If the coating amount is too small, the printing durability may be insufficient. If the coating amount is too large, the sensitivity may be reduced so that the necessary exposure time may increase, and the development time may also undesirably increase. For a main object of the invention, namely for a planographic printing plate for scanning exposure, the coating amount in terms of the mass after drying may be appropriately from 0.1 $g/m^2$ to 10 $g/m^2$, more preferably from 0.5 $g/m^2$ to 5 $g/m^2$.

Support

In the planographic printing plate precursor of the invention, the support preferably has a hydrophilic surface. The hydrophilic support may be selected from known conventional hydrophilic supports used for planographic printing

plates, without particular limitations.
The support is preferably a dimensionally stable plate-shaped material, examples of which include paper, paper laminated with plastic (such as polyethylene, polypropylene, or polystyrene), a metal plate (such as of an aluminum, zinc or cupper plate), a plastic film (such as a film of cellulose diacetate, cellulose triacetate, cellulose propionate, cellulose butyrate, cellulose acetate butyrate, cellulose nitrate, polyethylene terephthalate, polyethylene, polystyrene, polypropylene, polycarbonate, or polyvinyl acetal), and paper or a plastic film on which metal such as those listed above is laminated or vapor-deposited. If necessary, the surface of these materials may be subjected to an appropriate known physical and/or chemical treatment for the purpose of imparting hydrophilicity, improving strength or the like.

Paper, a polyester film or an aluminum plate is preferred as the support. The aluminum plate is particularly preferred, because it has excellent dimensional stability, is relatively inexpensive and can provide a highly hydrophilic or strong surface by being subjected to surface treatment that may be conducted as necessary. A composite sheet comprising a polyethylene terephthalate film and an aluminum sheet bonded thereto as described in JP-B No. 48-18327 is also preferable.

Preferred examples of the aluminum plate include a pure aluminum plate and a plate of an aluminum alloy comprising aluminum as the main component and a small amount of other elements. A plastic film on which aluminum is laminated or vapor-deposited may also be used. Examples of such other elements to be contained in aluminum alloys include silicon, iron, manganese, copper, magnesium, chromium, zinc, bismuth, nickel, and titanium. The content of foreign elements in the alloy is at most 10% by mass or less. In the invention, pure aluminum is particularly preferred. However, it is difficult to produce completely pure aluminum in terms of refinement technology, and therefore, trace amounts of other elements may be contained. The aluminum plate for use in the invention is not limited to have a particular composition, and may be appropriately selected from aluminum plates of any known conventional material. The aluminum plate for use in the invention preferably may have a thickness of about 0.1 mm to about 0.6 mm, preferably of 0.15 mm to 0.4 mm, particularly preferably of 0.2 mm to 0.3mm.

When the support has a metallic surface, specifically an aluminum surface, it preferably has undergone surface treatment such as surface roughening (graining), immersion into an aqueous solution of sodium silicate, potassium fluorozirconate, phosphate, or the like, or anodic oxidation.

Surface roughening of the aluminum plate may be performed by various methods such as methods of mechanically roughening the surface, methods of electrochemically dissolving and roughening the surface, and methods of chemically and selectively dissolving the surface. The mechanical roughening may be a known mechanical method such as ball polishing, brush polishing, blast polishing, and buff polishing. Electrochemical surface roughening methods may be performed in an electrolytic solution of hydrochloric acid, nitric acid or the like under alternating or direct current. A combination of both methods may also be used in combination as disclose in JP-A No. 54-63902. Prior to roughening of the surface of the aluminum plate, if necessary, degreasing with a surfactant, an organic solvent or an aqueous alkaline solution may be performed to remove rolling oil from the surface.

An aluminum plate that has undergone immersion in an aqueous sodium silicate solution after surface roughening is preferably used. An aluminum plate that has undergone the processes of anodic oxidation of the aluminum plate and immersion in an aqueous alkali metal silicate solution as described in JP-B No. 47-5125 may also preferably be used. For example, the anodic oxidation may be performed by allowing a current to flow through the aluminum plate serving as an anode in an electrolyte solution containing one or more of aqueous or nonaqueous solutions of inorganic acids such as phosphoric acid, chromic acid, sulfuric acid, and boric acid, organic acids such as oxalic acid and sulfamic acid, or salts thereof

The silicate electrodeposition described in U.S. No. 3,658,662 is also effective.

The surface treatment disclosed in JP-B No. 46-27481 and JP-ANos. 52-58602 and 52-30503 is also useful which uses an electrolytically grained support in combination with the anodic oxidation and sodium silicate treatment.

In a preferred treatment, mechanical surface roughening, chemical etching, electrolytic graining, anodic oxidation, and sodium silicate treatment are sequentially performed as described in JP-ANo. 56-28893.

After these treatments, undercoating may be preferably performed. The undercoating may include a water-soluble resin such as polyvinyl phosphonic acid, a polymer or copolymer having a sulfonic acid group in a side chain, or polyacrylic acid; a water-soluble metal salt (such as zinc borate); a yellow dye; or an amine salt.

A sol-gel-treated substrate is also preferably used in which a functional group capable of undergoing a radical addition reaction is covalently bonded as disclosed in JP-A No. 07-154983.

In another preferred example, a water-resistant hydrophilic layer may be formed as a surface layer on an arbitrary support. Examples of such a surface layer include a layer comprising an inorganic pigment and a binding agent as described in U.S. Patent No. 3,055,295 and JP-ANo. 56-13168, a hydrophilic swelling layer as described in JP-A No. 09-80744, and a sol-gel film of titanium oxide, polyvinyl alcohol or silicates as described in Japanese Patent Application National Publication (Laid-Open) No. 08-507727.

These hydrophilizing treatments may be performed not only to hydrophilize the surface of the support but also to prevent an undesirable reaction of the photopolymerizable composition to be provided thereon and to improve adhesion

of the photosensitive layer.

Protective Layer

The planographic printing plate precursor of the invention preferably further includes a protective layer on the photosensitive layer. The protective layer can prevent low-molecular compounds such as basic substances or oxygen in the air, which inhibits image-forming reaction in the photosensitive layer caused by exposure to light, from entering the photosensitive layer, and enables exposure in the air. Therefore, the protective layer preferably has such characteristics that the protective layer is less permeable to low-molecular compounds such as oxygen, does not substantially inhibit transmission of light for exposure, is excellent in adhesion property to the photosensitive layer, and is easily removable by a development process after exposure.

Improvements of the protective layer have been made as described in detail in U.S. Patent No. 3,458,311 and JP-A No. 55-49729. For example, water-soluble polymer compounds having relatively high crystallinity may be preferably used for the protective layer. Examples of such materials include water-soluble polymers such as polyvinyl alcohol, polyvinylpyrrolidone, acidic celluloses, gelatin, gum arabic, and polyacrylic acid. In particular, the best result in terms of basic characteristics such as oxygen barrier characteristics and removability by development can be obtained using polyvinyl alcohol as a main component.

Polyvinyl alcohol for use in the protective layer may be partially substituted by at least one of an ester, an ether and an acetal, as long as it has an unsubstituted vinyl alcohol unit for imparting necessary oxygen barrier characteristics and water solubility. In the same way, the polyvinyl alcohol may partially have at least one other copolymerization component. Specifically, the polyvinyl alcohol may be hydrolyzed to a degree of 71 to 100 mol% and may have a mass average molecular weight in the range of 300 to 2400. Examples of the polyvinyl alcohol include PVA-105, PVA-110, PVA-117, PVA-117H, PVA-120, PVA-124, PVA-124H, PVA-CS, PVA-CST, PVA-HC, PVA-203, PVA-204, PVA-205, PVA-210, PVA-217, PVA-220, PVA-224, PVA-217EE, PVA-217E, PVA-220E, PVA-224E, PVA-405, PVA-420, PVA-613, and L-8 manufactured by Kuraray Co., Ltd.

The components (choice of PVAs, use of additives and the like), the coating amount and the like of the protective layer may be selected in consideration of on oxygen barrier characteristics, removability by development, fogging, adhesion property, or scratch resistance. In general, a higher rate of hydrolysis of the PVA to be used (a higher content of unsubstituted vinyl alcohol units in the protective layer) or a larger thickness of the film leads to a higher level of oxygen barrier characteristics, which is advantageous in terms of sensitivity. When oxygen barrier characteristics are increased excessively, problems may occur such as unnecessary polymerization reactions during manufacture or storage and unnecessary fogging or thickening of image lines during imagewise exposure. The adhesion to the image portion and the scratch resistance are also very important for the handleability of the printing plate. Methods for applying the protective layer are described in detail in, for example, U.S. Patent No. 3,458,313 and JP-A No. 55-49729.

The protective layer may also have another function. For example, a colorant (such as a water-soluble dye) capable of efficiently transmitting light in the range of 350 nm to 450 nm to be used for exposure and efficiently absorbing light at 500 nm or more may be added to the protective layer so that safe light suitability can be further improved without a reduction in sensitivity.

Other Layers

Other layers such as layers for improving adhesion between the photosensitive layer and the support and layers for improving the removability of the unexposed photosensitive layer by development may also be provided. For example, the adhesion or the printing durability may be improved by adding a diazonium structure-containing compound or a compound capable of relatively strongly interacting with the substrate, such as phosphonic compounds or by providing an undercoat layer of such a compound. On the other hand, the developability of the non-image portion or the antifouling properties may be improved by adding a hydrophilic polymer such as polyacrylic acid or polysulfonic acid or by providing an undercoat layer of such a polymer.

Plate Making

In general, the planographic printing plate precursor is imagewise exposed to light and then the unexposed portion is removed by using a developing solution to form an image.

Any known light exposure method may be applied to the planographic printing plate precursor of the invention, without limitations. The wavelength of the light source is preferably from 350 nm to 450 nm, and specifically InGaN semiconductor lasers are preferred. The exposure mechanism may be of any system such as an internal drum system, an external drum system or a flat bed system. A highly water-soluble component may be used for the photosensitive layer so that the layer can dissolved in neutral water or weakly alkaline water. The planographic printing plate having

such a constitution may be subjected to on-press exposure and development after mounted on a printing machine.

Available laser light sources in the range of 350 nm to 450 nm include the lasers described below.

Gas lasers include Ar ion lasers (364 nm, 351 nm, 10 mW to 1 W), Kr ion lasers (356 nm, 351 nm, 10 mW to 1 W) and He-Cd lasers (441 nm, 325 nm, 1 to 100 mW); Solid-state lasers include a combination of Nd:YAG ($YVO_4$) and SHG crystal (twice) (355 nm, 5 mW to 1 W) and a combination of Cr:LiSAF and SHG crystal (430 nm, 10 mW); Semiconductor lasers include $KNbO_3$ ring resonators (430 nm, 30 mW), a combination of a waveguide type wavelength converting device and an AlGaAs or InGaAs semiconductor (380 to 450 nm, 5 to 100 mW), a combination of a waveguide type wavelength converting device and an AlGaInP or AlGaAs semiconductor (300 to 350 nm, 5 to 100 mW), and AlGaInN lasers (350 to 450 nm, 5 to 30 mW); Other lasers include pulsed lasers such as $N_2$ lasers (337 nm, 0.1 to 10 mJ pulse) and XeF lasers (351 nm, 10 to 250 mJ pulse).

Above all, AlGaInN semiconductor lasers (commercially available InGaN semiconductor lasers (400 to 410 nm, 5 to 30 mW)) are particularly preferred in view of wavelength characteristics and cost.

For scanning exposure type planographic printing plate exposure systems, internal drum, external drum, or flat bed systems may be used as exposure mechanisms, and all of the light sources describe above, except for pulsed lasers, may be used as light sources. Practically, the exposure systems described below are particularly preferred in view of the relationship between the sensitivity of the material and plate making time.

A single-beam exposure system of an internal drum system using a single gas or solid-state laser source;
A multi-beam exposure system of a flat bed system using a number of (ten or more) semiconductor lasers; and
A multi-beam exposure system of an external drum system using a number of (ten or more) semiconductor lasers.

For laser direct drawing type planographic printing plates as described above, the following equation (eq1) is generally established, wherein X ($J/cm^2$) is the sensitivity of the photosensitive material, S ($cm^2$) is the exposed area of the photosensitive material, q (W) is the power of one laser light source, n is the number of the laser light sources, and t (s) is the total exposure time.

$$(eq1): X \cdot S = n \cdot q \cdot t$$

In the case of (i) the internal drum system (single beam), the following equation (eq2) is generally established, wherein f (radian/s) is the rotational frequency of the laser, Lx (cm) is the sub-scanning length of the photosensitive material, Z (dots/cm) is the resolution, and t (s) is the total exposure time.

$$(eq2): f \cdot Z \cdot t = Lx$$

In the case of (ii) the external drum system (multi-beam), the following equation (eq3) is generally established, wherein F (radian/s) is the rotational frequency of the drum, Lx (cm) is the sub-scanning length of the photosensitive material, Z (dots/cm) is the resolution, t (s) is the total exposure time, and n is the number of the beams.

$$(eq3): F \cdot Z \cdot n \cdot t = Lx$$

In the case of (iii) the flat bed system (multi-beam), the following equation (eq4) is generally established, wherein H (radian/s) is the rotational frequency of the polygon mirror, Lx (cm) is the sub-scanning length of the photosensitive material, Z (dots/cm) is the resolution, t (s) is the total exposure time, and n is the number of the beams.

$$(eq4): H \cdot Z \cdot n \cdot t = Lx$$

Substitution of the resolution (2560 dpi) required of a practical printing plate, the size of the plate (A1/B1, 42 inches in sub-scanning length), the exposure condition of about 20 sheets per hour, and the photosensitive characteristics

of the photopolymerizable composition of the invention (the photosensitive wavelength, and a sensitivity of about 0.1 $mJ/cm^2$) into the equations suggests that the photosensitive material of the invention should preferably be used in combination with a semiconductor laser of a multi-beam exposure type. Also taking handleability and cost into account, it is suggested that the photosensitive material of the invention is most preferably used in combination with a semiconductor laser, multi-beam exposure system of an external drum system.

Other examples of light sources that may be used for exposure include any of ultra-high pressure, high pressure, medium pressure, and low pressure mercury lamps, chemical lamps, carbon arc lamps, xenon lamps, metal halide lamps, a variety of visible and ultraviolet laser lamps, fluorescent lamps, tungsten lamps, and sunlight.

Developing solutions suitable for the planographic printing plate precursor of the invention include the developers described in JP-B No. 57-7427. Appropriate developers include aqueous solutions of inorganic alkaline agents such as sodium silicate, potassium silicate, sodium hydroxide, potassium hydroxide, lithium hydroxide, sodium tertiary phosphate, sodium secondary phosphate, ammonium tertiary phosphate, ammonium secondary phosphate, sodium metasilicate, sodium bicarbonate, and ammonia water, and aqueous solutions of organic alkaline agents such as monoethanolamine and diethanolamine. The alkaline agent may be added such that the alkaline solution may have a concentration of 0.1 to 10% by mass, preferably of 0.5 to 5% by mass.

If necessary, the alkaline aqueous solution may contain a small amount of a surfactant or an organic solvent such as benzyl alcohol, 2-phenoxyethanol, or 2-butoxyethanol. Examples of such additives include those described in U.S. Patent Nos. 3,375,171 and 3,615,480.

The developing solutions described in JP-ANos. 50-26601 and 58-54341 and JP-B Nos. 56-39464 and 56-42860 are also excellent.

The developing solution described in JP-A No. 2002-202616 is particularly preferred which contains a nonionic compound represented by Formula ($\alpha$) and has a pH of 11.5 to 12.8 and a conductivity of 3 to 30 mS/cm.

Formula ($\alpha$): A-W

In Formula ($\alpha$), A represents a hydrophobic organic group wherein log P of A-H is 1.5 or more, and W represents a nonionic hydrophilic organic group wherein log P of W-H is less than 1.0.

The log P is used generally as a hydrophobic parameter described in C. Hansch, A. Leo, "Substituent Constants for Correlation Analysis in Chemistry and Biology", J. Wile & Sons, 1979, and is defined as logarithm of equilibrium concentration ratio P calculated from proportion distributed in each layer in a 2-layer system of octanol/water of an objective molecule (A-H and W-H). The log P is used as an index for specifying groups A and W in the formula ($\alpha$) and determined by calculation from known data based on a method described in A. K. Ghose, et al. J. Comput. Chem. 9, 80 (1988) with respect to A-H and W-H structures where a hydrogen atom is conveniently bound to each organic group of A and W.

Components in this developing solution are described in detail in paragraphs (0024) to (0067) in JP-A No. 2002-202616.

It is effective to add the nonionic compound represented by Formula ($\alpha$) such that its concentration in the developing solution becomes 0.1 to 15% by mass, preferably of 1.0 to 8.0% by mass.

If necessary, the process of making a printing plate from the planographic printing plate precursor may further include heating the whole surface before or during exposure or between exposure and development. Such heating can facilitate the image forming reaction in the photosensitive layer so that advantages such as an increase in sensitivity or printing durability and stabilization of sensitivity can be achieved. In addition, whole surface exposure or whole surface post heating may be effectively performed on the image after the development in order to improve the strength or printing durability of the image. In general, heating before the development is preferably performed under mild conditions at 150˚C or lower. At 150˚C or lower, the non-image portion can be free of the problem of fogging. Very strong conditions should be used for heating after the development. Such conditions are generally in the range of 200 to 500˚C. At 200˚C or higher, the image strengthening effect can be sufficiently obtained. At 500˚C or lower, problems such as deterioration of the support and thermal decomposition of the image portion do not occur.

The inventors made extensive study, and as a result, they found that an oxime compound having a novel structure may be used to attain excellent absorbance to light having a wavelength of 365 nm or 405 nm and excellent temporal stability. Specific means for solving the problems described above is shown below:

<1> A compound represented by the following Formula (1):

Formula (1)

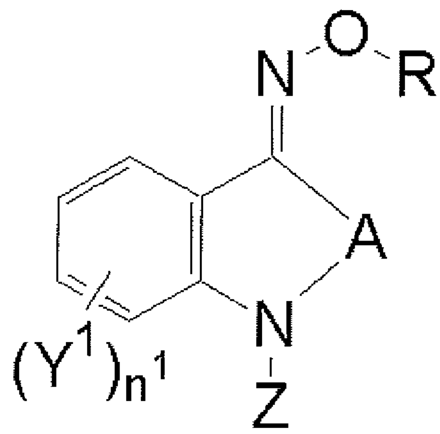

wherein R, $Y^1$ and Z each independently represent a monovalent substituent, $n^1$ represents an integer of 0 to 4, $Y^1$ and Z may be bound to each other to form a ring, and A represents a divalent linking group.

<2> The compound of the above-mentioned <1>, which is represented by the following Formula (2):

Formula (2)

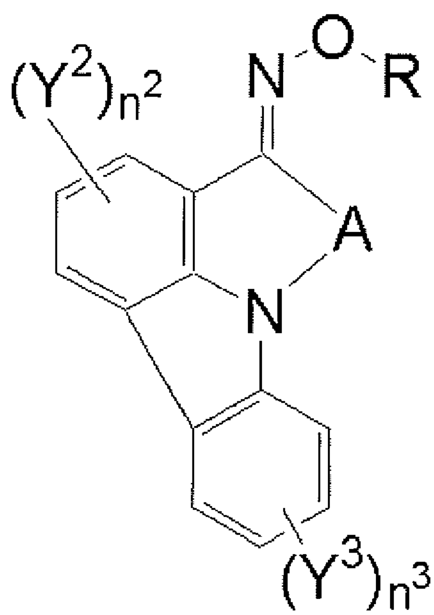

wherein R, $Y^2$ and $Y^3$ each independently represent a monovalent substituent, $n^2$ and $n^3$ independently represent an integer of 0 to 4, and A represents a divalent linking group.

<3> The compound of the above-mentioned <1> or <2>, which is represented by the following Formula (3):

Formula (3)

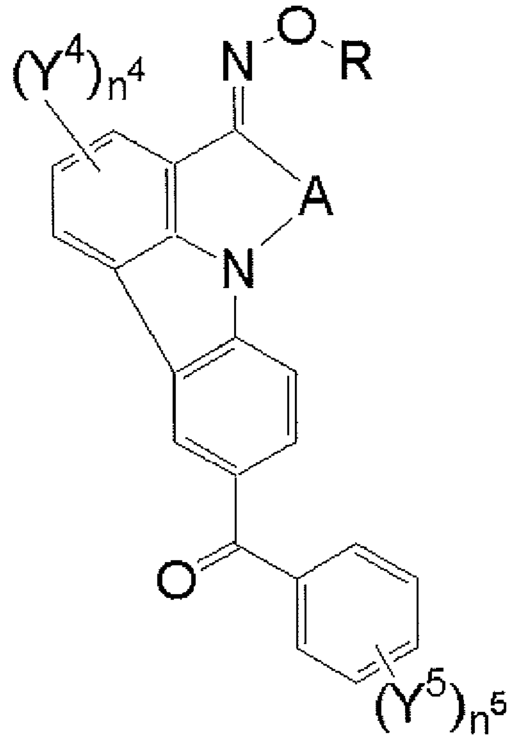

wherein R, $Y^4$ and $Y^5$ each independently represent a monovalent substituent, $n^4$ and $n^5$ independently represent an integer of 0 to 4, and A represents a divalent linking group.

<4> A photopolymerizable composition containing (A) the compound of any of the above-mentioned <1> to <3> and (B) a polymerizable compound.

<5> The photopolymerizable composition of the above-mentioned <4>, which further contains (C) a colorant.

<6> The photopolymerizable composition of the above-mentioned <5>, wherein the colorant (C) is a pigment, further containing (D) a pigment dispersant.

<7> The photopolymerizable composition of the above-mentioned <5> or <6>, wherein the colorant (C) is a black colorant.

<8> A photopolymerizable composition for color filter, which contains the photopolymerizable composition of any of the above-mentioned <5> to <7>.

<9> A color filter having a colored pattern using the photopolymerizable composition for color filter of the above-mentioned <8> on a support.

<10> A method for producing a color filter, including:

applying the photopolymerizable composition for color filter of the above-mentioned <8> on a support to form a photopolymerizable composition layer,
exposing the photopolymerizable composition layer via a mask to light, and
developing the photopolymerizable composition layer after exposure to form a colored pattern.

<11> A solid-state imaging device including the color filter of the above-mentioned <9>.

<12> A planographic printing plate precursor having a photosensitive layer containing the photopolymerizable composition of the above-mentioned <4> on a support.

EXAMPLES

Hereinafter, the invention will be described in more detail with reference to the Examples, but the invention is not limited to such examples unless the gist of the invention is exceeded. Unless otherwise specified, "part" is based on mass, and "%" and "wt%" refer to "% by mass".

Details of the specific oxime compounds (Specific Compounds 1 to 9) and comparative compounds (Comparative Compounds 1 to 3) used in the Examples and Comparative Examples are shown below.

| Compound No. | Structure |
|---|---|
| Specific Compound 1 | 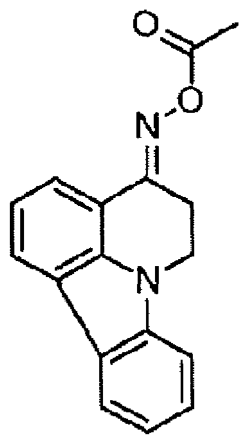 |
| Specific Compound 2 | 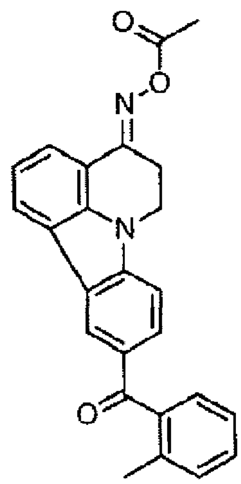 |
| Specific Compound 3 | 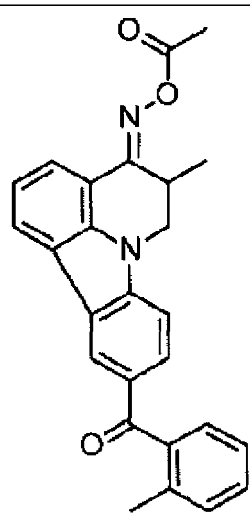 |

(continued)

| Compound No. | Structure |
|---|---|
| Specific Compound 4 | 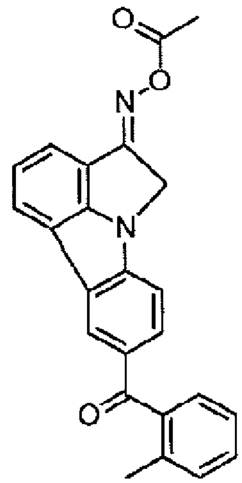 |
| Specific Compound 5 | 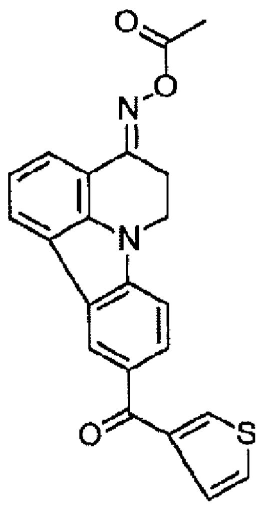 |
| Specific Compound 6 | 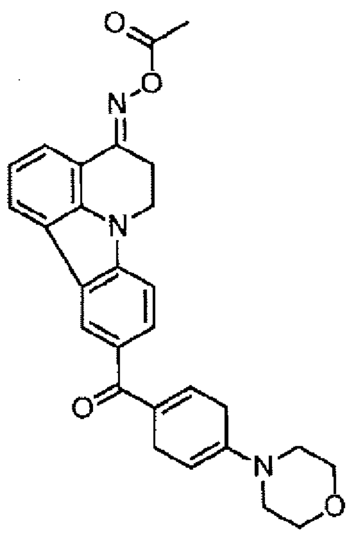 |

(continued)

| Compound No. | Structure |
|---|---|
| Specific Compound 7 | 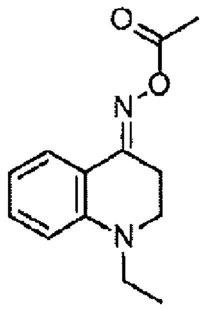 |
| Specific Compound 8 | 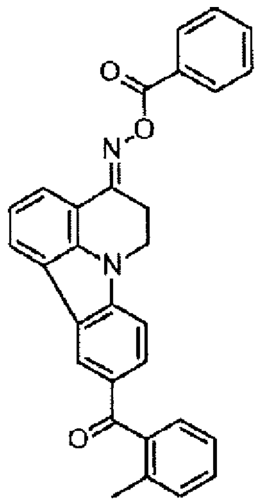 |
| Specific Compound 9 | 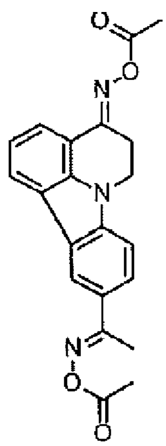 |
| Comparative Compound 1 | IRGACURE OXE01 (manufactured by Ciba Specialty Chemicals) |
| Comparative Compound 2 | IRGACURE OXE02 (manufactured by Ciba Specialty Chemicals) |
| Comparative Compound 3 | 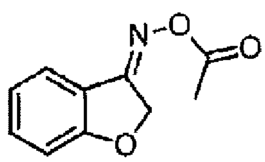 |

Specific Compounds 1 to 9 that are the specific oxime compounds listed above were synthesized by a method shown below.

Synthesis Example 1: Synthesis of Specific Compound 1 that is a specific oxime compound

Compound A below (18.0 g, 76.2 mmol) was dissolved in 100 ml tetrahydrofuran, and triethylamine (9.25 g, 91.4 mmol) was added thereto. Then, the mixture was cooled to 0˚C, and acetyl chloride (7.18 g, 91.4 mmol) was added dropwise over 20 minutes to the mixture which was then warmed to room temperature and stirred for 2 hours. The reaction solution was dropped into 150 ml distilled water cooled to 0˚C, an organic layer was extracted with ethyl acetate and dried with magnesium sulfate, and the solvent was distilled away under reduced pressure. Precipitated crystals were washed with methanol to give Specific Compound 1 (recovery 19.5 g, yield 92%) having the following structure:
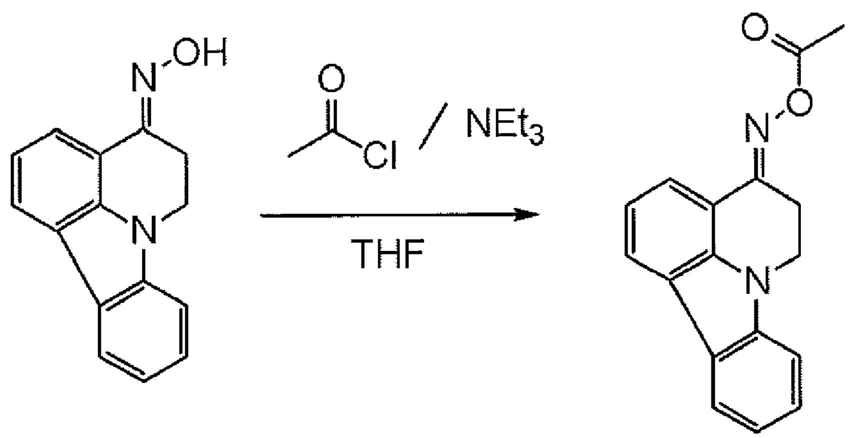


Compound A Specific Compound 1

The structure of the resulting specific compound 1 was identified by NMR.
(1H-NMR 400 MHz heavy acetone): 8.22 (d, 1H, J=7.8 Hz), 8.20 (d, 1H, J=7.8 Hz), 7.95 (d, 1H, J=7.6 Hz), 7.60 (d, 1H, J=8.0 Hz), 7.54-7.50 (m, 1H), 7.27 (t, 2H, 7.3 Hz), 4.45 (t, 2H, 6.8 Hz), 3.50 (t, 2H, 6.8 Hz), 2.29 (s, 3H).

Synthesis Example 2: Synthesis of Compound 2 that is a specific oxime compound

Specific Compound B below (20.0 g, 56.4 mmol) was dissolved in 100 ml tetrahydrofuran, and triethylamine (6.85 g, 67.7 mmol) was added thereto. Then, the mixture was cooled to 0˚C, and acetyl chloride (5.32 g, 67.7 mmol) was added dropwise over 20 minutes to the mixture which was then warmed to room temperature and stirred for 2 hours. The reaction solution was dropped into 150 ml distilled water cooled to 0˚C, an organic layer was extracted with ethyl acetate and dried with magnesium sulfate, and the solvent was distilled away under reduced pressure. Precipitated crystals were washed with methanol to give Specific Compound 2 (recovery 19.0 g, yield 85%) having the following structure:
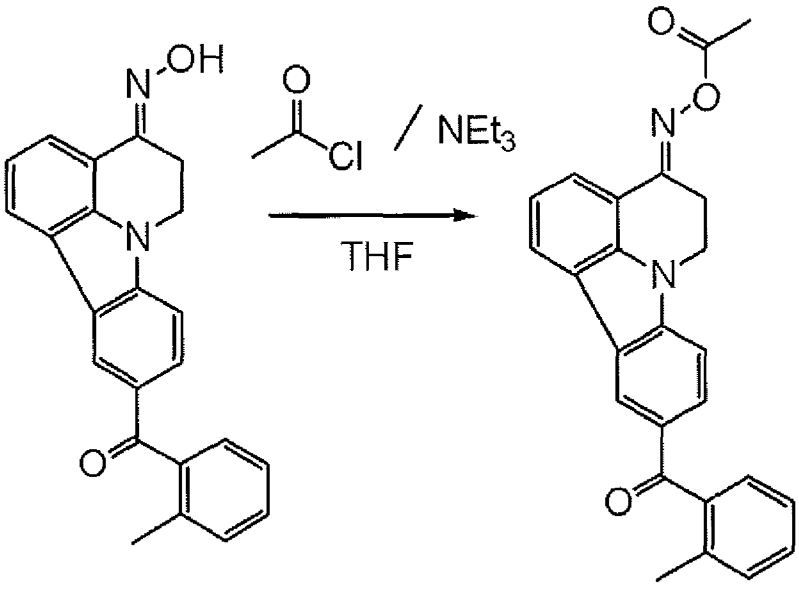


Compound B Specific Compound 2

The structure of the resulting specific compound 2 was identified by NMR. (1H-NMR 400 MHz heavy acetone): 8.62 (s, 1H), 8.29 (d, 1H, J=7.8 Hz), 8.02-7.98 (m, 2H), 7.71 (d, 1H, J=8.7 Hz), 7.49-7.32 (m, 1H), 7.40-7.32 (m, 4H), 4.57 (t, 2H, J=6.8 Hz), 3.53 (t, 2H, J=6.8 Hz), 2.30 (s, 3H), 2.29 (s, 3H).

Specific Compounds 3 to 9 that are the specific oxime compounds listed above were synthesized in the same manner as in Synthesis Examples 1 to 2.

The structures of Comparative Compound 1 (IRGACURE OXE 01) and Comparative Compound 2 (IRGACURE OXE 02) shown in the above compound list are as follows:

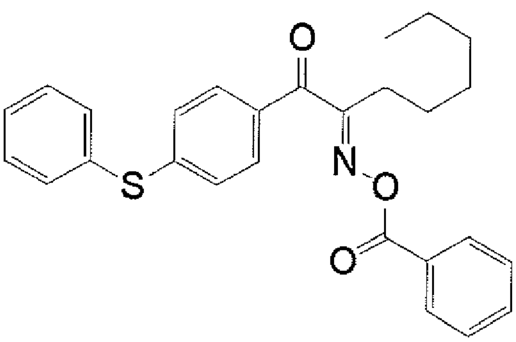

IRGACURE OXE 01

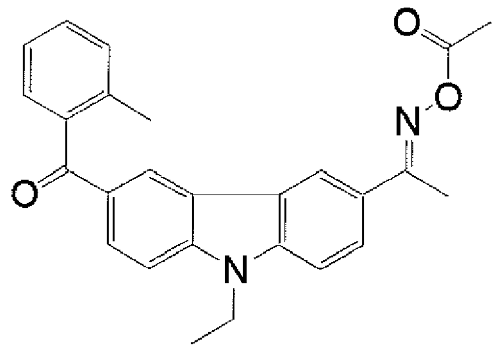

IRGACURE OXE 02

Example 1-1

<Preparation and Evaluation of Photopolymerizable Composition 1>

Photopolymerizable Composition 1 was prepared in the following manner and evaluated for its sensitivity. A uniform composition containing 0.08 mmol of Specific Compound 1 above as the specific oxime compound, 1 g of pentaerythritol tetraacrylate as a radical-polymerizable compound, 1 g ofpolymethyl methacrylate (molecular weight of ca. 996000, manufactured by Aldrich) as a binder resin, and 16 g of cyclohexanone as solvent was prepared. The resulting composition was used as a coating solution, applied to a glass plate by a spin coater, and dried at 40˚C for 10 minutes to prepare a coating film of 1.5 μm in thickness. A 21√(2-step tablet (gray-scale film manufactured by Dainippon Screen Mfg. Co., Ltd.) was placed on this coating film which was then exposed via a heat-ray cut filter to light from a 500 mW high-pressure mercury lamp (manufactured by Ushio, Inc.) for 30 seconds, followed by development treatment by dipping it in toluene for 60 seconds. When the number of stages at which it was completely cured and insolubilized, corresponding to the step tablet, was evaluated as sensitivity, the sensitivity was 8 stages.
The greater the number of sensitivity stages, the higher the sensitivity.

Example 1-2 to Example 1-9, and Comparative Example 1-1 to Comparative

Example 1-3

Photopolymerizable Compositions 2 to 12 were prepared in the same manner as in Example 1-1 except that 0.08 mmol of the compounds listed above (Specific Compounds 2 to 9 and Comparative Compounds 1 to 3) were used respectively in place of Specific Compound 1 (0.08 mmol) used as the specific oxime compound in Example 1-1, and the respective compositions were evaluated for the number of sensitivity stages in the same manner as in Example 1-1.
The evaluation results of Examples 1-1 to 1-9 and Comparative Examples 1-1 to 1-3 are shown in Table 1 below.
Table 1

| | Compound | Photopolymerizable Composition | Number of Sensitivity Stages |
|---|---|---|---|
| Example 1-1 | Specific Compound 1 | 1 | 8 |
| Example 1-2 | Specific Compound 2 | 2 | 9 |
| Example 1-3 | Specific Compound 3 | 3 | 9 |
| Example 1-4 | Specific Compound 4 | | 9 |
| Example 1-5 | Specific Compound 5 | 5 | 9 |
| Example 1-6 | Specific Compound 6 | 6 | 9 |
| Example 1-7 | Specific Compound 7 | 7 | 7 |
| Example 1-8 | Specific Compound 8 | 8 | 9 |
| Example 1-9 | Specific Compound 9 | 9 | 8 |

(continued)

| | Compound | Photopolymerizable Composition | Number of Sensitivity Stages |
|---|---|---|---|
| Comparative example 1-1 | Comparative Compound 1 | 10 | 5 |
| Comparative example 1-2 | Comparative Compound 2 | 11 | 5 |
| Comparative example 1-3 | Comparative Compound 3 | 12 | 5 |

Example 2-1

1. Preparation of Colored Photopolymerizable Composition A-1

As a color filter-forming photopolymerizable composition, negative-type Colored Photopolymerizable Composition A-1 containing a colorant (pigment) was prepared and used to prepare a color filter.

1-1. Preparation of Pigment Dispersion (P1)

40 Parts by mass of a 30/70 (mass ratio) mixture of C.I. Pigment Green 36 and C.I. Pigment Yellow 219 as pigments, 10 parts by mass (about 4.51 parts by mass in terms of solid content) of BYK2001 (Disperbyk, solid content 45.1% by mass, manufactured by BYK Chemie) as a dispersant, and 150 parts by mass of ethyl 3-ethoxypropionate as a solvent were mixed and dispersed for 15 hours with a beads mill to prepare a pigment dispersion (P1).

The average particle size of the pigment in the resulting pigment dispersion (P1), as determined by dynamic light scattering method, was 200 nm.

1-2. Preparation of Colored Photopolymerizable Composition A-1 (coating solution)

Components in Composition A-1 below were mixed and dissolved to prepare Colored Photopolymerizable Composition A-1.

<Composition A-1>

- Pigment dispersion (P1) 600 parts by mass
- Alkali-soluble resin 200 parts by mass
  (Benzyl methacrylate/methacrylic acid/hydroxyethyl methacrylate copolymer, molar ratio: 80/10/10, Mw: 10,000)
- Multifunctional monomer: dipentaerythritol hexaacrylate 60 parts by mass
- Specific oxime compound: Specific Compound 1 60 parts by mass
- Solvent: propylene glycol monomethyl ether acetate 1,000 parts by mass
- Surfactant (trade name: Tetranik 150R1, manufactured by BASF) 1 part by mass
- γ-Methacryloxypropyltriethoxysilane 5 parts by mass

2. Preparation of a color filter

2-1. Formation of a photopolymerizable composition layer

A 550 mmx650 mm glass substrate was slit-coated with the thus obtained pigment-containing Colored Photopolymerizable Composition A-1 as a resist solution under the following conditions and then left for 10 minutes as it was, followed by vacuum drying and pre-baking (100°C, 80 seconds) to form a photopolymerizable composition coating film (photopolymerizable composition layer) thereon.

(Slit-coating conditions)

Space of an opening at the top of a coating head: 50 μm

Coating speed: 100 mm/sec
Clearance between the substrate and the coating head: 150 μm
Coating thickness (thickness after drying): 2 μm
Coating temperature: 23˚C

2-2. Exposure & Development

Thereafter, the photopolymerizable composition layer was subjected to pattern exposure with a 2.5-kw ultrahigh pressure mercury lamp. After exposure, the whole area of the photopolymerizable composition layer was covered with 10% aqueous solution of an organic developing solution (trade name: CD, manufactured by Fuji Film Electronics Materials Co., Ltd) and left for 60 seconds as it was.

2-3. Heat Treatment

Then, the photopolymerizable composition layer was sprayed with a shower of purified water to wash the developing solution away and then heated in an oven at 220˚C for 1 hour (post-baking). In this manner, a color filter having a colored pattern on the glass substrate was obtained.

3. Evaluation of Performance

The storage stability and exposure sensitivity of the colored photopolymerizable composition, the developability of the colored photopolymerizable composition upon formation of a colored pattern on a glass substrate, the coloration of the resulting colored pattern with time under heating, the adhesiveness of the pattern to the substrate, and the sectional shape of the pattern were evaluated in the following manner. The evaluation results were collectively shown in Table 2.

3-1. Storage stability of the colored photopolymerizable composition

The colored photopolymerizable composition was stored at room temperature for 1 month, and the degree of precipitation of foreign substances was evaluated visually under the following judgment criteria:

- Judgment Criteria -

A: No precipitation was recognized.
B: Slight precipitation was recognized.
C: Precipitation was recognized.

3-2. Exposure sensitivity of the colored photopolymerizable composition

The colored photopolymerizable composition was spin-coated on a glass substrate and dried to form a coating film of 1.0 μm in thickness. Spin coating was conducted at 300 rpm for 5 seconds and then at 800 rpm for 20 seconds, and drying was conducted at 100˚C for 80 seconds. Then, the resulting coating film was exposed via a testing photomask with a line width of 2.0 μm to various amounts of light in the range of 10 to 1600 mJ/$cm^2$ from a proximity-type exposure device provided with an ultrahigh pressure mercury lamp (manufactured by Hitachi High-Tech Electronics Engineering Co., Ltd.). Then, the coating film after exposure was developed under the condition of 25˚C for 60 seconds with 60% CD-2000 developing solution (manufactured by Fuji Film Electronics Materials Co., Ltd). Thereafter, the coating film was rinsed with running water for 20 seconds and then spray-dried to complete patterning.
In evaluation of exposure sensitivity, the minimum light exposure at which the thickness of the coating film in the region exposed to light in the exposure step reached 95% or more based to the thickness (=100%) of the coating film before exposure was evaluated as exposure sensitivity. A smaller value of exposure sensitivity is indicative of higher sensitivity.

3-3. Developability, pattern sectional shape, coloring change with time under forced heating, and substrate adhesiveness

Developability, substrate adhesiveness, coloring change with time under forced heating, and pattern sectional shape were evaluated by confirming the substrate surface and sectional shape after post-baking in "2-3. Heat Treatment" by observation in a usual manner under an optical microscope and with SEM photographs. The detailed evaluation method is as follows.

<Developability>

In the exposure step, the presence or absence of residues in the region not irradiated with light (unexposed region) was observed to evaluate developability. The evaluation criteria are as follows.

- Evaluation Criteria -

A: No residue was observed in the unexposed region.
B: Slight residues were observed in the unexposed region but at a practically unproblematic level.
C: Significant residues were confirmed in the unexposed region.

<Evaluation of coloring with time under forced heating>

The photopolymerizable composition layer (colored pattern) after exposure and after development was heated on a hot plate at 200˚C for 1 hour and then evaluated with MCPD-3000 (manufactured by Otsuka Electronic Co.) for its color difference ∆Eab* before and after heating, under the following criteria:

- Evaluation Criteria -

A: ∆rab*≤5

B: 5 < µEab* < 8

C: µEabx≥8

<Substrate adhesiveness>

Substrate adhesiveness was evaluated by observing whether pattern defects had been generated or not under the following criteria:

- Evaluation Criteria -

A: No pattern defect was observed.
B: Pattern defects were seldom observed, but partial defects were observed.
C: Quite a lot of pattern defects were observed.

<Pattern Sectional Shape>

The sectional shape of the formed pattern was evaluated by observation. The sectional shape of the pattern is preferably a forward tapered shape, most preferably a rectangular shape. An inverse tapered shape is not preferable.

<Post-Heating Pattern Sectional Shape>

The sectional shape of the pattern formed after post-baking in "2-3. Heat Treatment" was evaluated by observation. The sectional shape of the pattern is preferably a forward tapered shape, most preferably a rectangular shape. An inverse tapered shape is not preferable.

Examples 2-2 to 2-17, Comparative Examples 2-1 to 2-3

Colored Photopolymerizable Compositions A-2 to A-17 and A'-1 to A'-3 were prepared in the same manner as in Example 2-1 except that the amounts of the respective compounds shown in Table 2 below were used in place of 60 parts by mass of Specific Compound 1 (specific oxime compound) in the composition A-1 used in preparing Colored

Photopolymerizable Composition A-1 in Example 2-1, and the types and the amounts of the sensitizers and the co-sensitizers shown in Table 2 below were used in Examples 2-10 to 2-17, in order to obtain color filters. The same evaluation as in Example 2-1 was carried out. The results are shown in Table 2.

Table 2

| | Compound | | | Sensitizer | | Co-sensitizer | | Storage stability | Exposure sensitivity (mJ/cm$^2$) | Developability | Coloring change with time under forced heating | Substrate adhesiveness | Pattern sectional shape | Post-heating pattern sectional shape |
|---|---|---|---|---|---|---|---|---|---|---|---|---|---|---|
| | specific | comparative | content | type | content | type | content | | | | | | | |
| Example 2-1 | 1 | - | 60 | - | - | - | - | A | 100 | A | A | A | rectangular | rectangular |
| Example 2-2 | 2 | - | 60 | - | - | - | - | A | 90 | A | A | A | rectangular | rectangular |
| Example 2-3 | 3 | - | 60 | - | - | - | - | A | 90 | A | A | A | rectangular | rectangular |
| Example 2-4 | 4 | - | 60 | - | - | - | - | A | 90 | A | A | A | rectangular | rectangular |
| Example 2-5 | 5 | - | 60 | - | - | - | - | A | 90 | A | A | A | rectangular | rectangular |
| Example 2-6 | 6 | - | 60 | - | - | - | - | A | 90 | A | A | A | rectangular | rectangular |
| Example 2-7 | 7 | - | 60 | - | - | - | - | A | 120 | A | A | A | rectangular | rectangular |
| Example 2-8 | 8 | - | 60 | - | - | - | - | A | 90 | A | A | A | rectangular | rectangular |
| Example 2-9 | 9 | - | 60 | - | - | - | - | A | 100 | A | A | A | rectangular | rectangular |
| Example 2-10 | 2 | - | 50 | A1 | 10 | - | - | A | 90 | A | A | A | rectangular | rectangular |
| Example 2-11 | 2 | - | 50 | A2 | 10 | - | - | A | 90 | A | A | A | rectangular | rectangular |
| Example 2-12 | 2 | - | 50 | A3 | 10 | - | - | A | 80 | A | A | A | rectangular | rectangular |
| Example 2-13 | 2 | - | 50 | - | - | F1 | 10 | A | 90 | A | A | A | rectangular | rectangular |
| Example 2-14 | 2 | - | 40 | A3 | 10 | F1 | 10 | A | 80 | A | A | A | rectangular | rectangular |
| Example 2-15 | 2 | - | 40 | A3 | 10 | F2 | 10 | A | 80 | A | A | A | rectangular | rectangular |
| Example 2-16 | 2 | - | 40 | A3 | 10 | F3 | 10 | A | 70 | A | A | A | rectangular | rectangular |
| Example 2-17 | 2 | - | 40 | A3 | 10 | F3 | 5 | A | 70 | A | A | A | rectangular | rectangular |
| | | | | | | LD-5 | 5 | | | | | | | |

(continued)

| | Compound | | | Sensitizer | | Co-sensitizer | | Storage stability | Exposure sensitivity ($mJ/cm^2$) | Developability | Coloring change with time under forced heating | Substrate adhesiveness | Pattern sectional shape | Post-heating pattern sectional shape |
|---|---|---|---|---|---|---|---|---|---|---|---|---|---|---|
| | specific | comparative | content | type | content | type | content | | | | | | | |
| Comparative example 2-1 | - | 1 | 60 | - | - | - | - | A | 150 | A | B | B | inverse taper | forward taper |
| Comparative example 2-2 | | 2 | 60 | - | - | - | - | A | 140 | A | C | B | inverse taper | forward taper |
| Comparative example 2-3 | - | 3 | 60 | - | - | - | - | A | 140 | A | B | B | inverse taper | forward taper |

In Table 2 above, numerical 1 to 9 in column "specific" in column "Compound" refer to Specific Compounds 1 to 9 respectively, and numerical 1 to 3 in column "comparative" refer to Comparative Compounds 1 to 3 respectively. The "content" is expressed in parts by mass.
In Table 2 below, the types A1 to A3 of the sensitizer and the types F1 to F3 and LD-5 of the co-sensitizer refer to the following compounds:
A1: 4,4-Bisdiethylaminobenzophenone
A2: Diethylthioxanthone
A3:

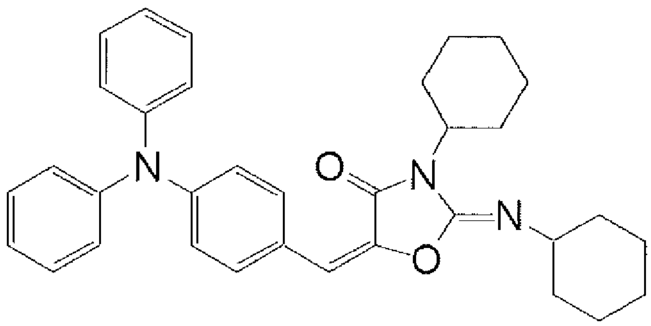

F1: 2-Mercaptobenzimidazole
F2: 2-Mercaptobenzothiazole
F3: N-Phenyl-2-mercaptobenzimidazole
LD-5: 2,2'-Bis(o-chlorophenyl)-4,4',5,5'-tetraphenylbiimidazole

From the results in Table 2, it may be seen that the colored photopolymerizable compositions in the Examples containing the specific oxime compounds (compounds 1 to 9) are excellent in storage stability (temporal stability). It may also be seen that these colored photopolymerizable compositions have high exposure sensitivity, are excellent in developability upon formation of colored patterns on color filters, are free of coloring in the resulting colored patterns with time under heating, and are excellent in both substrate adhesiveness and pattern sectional shape.

Example 3-1

1. Preparation of Resist solution

Components in the following composition were mixed and dissolved to prepare a resist solution.

- Composition of the resist solution -

- Propylene glyol monoethyl ether acetate (PGMEA) 19.20 parts by mass
- Ethyl lactate 36.67 parts by mass
- Resin 30.51 parts by mass
  (40% solution of a benzyl methacrylate/methacrylic acid/2-hydroxyethyl methacrylate copolymer (molar ratio = 60/22/18) in PGMEA)
- Dipentaerythritol hexaacrylate (polymerizable compound) 12.20 parts by mass
- Polymerization inhibitor (p-methoxyphenol) 0.0061 part by mass
- Fluorochemical surfactant 0.83 part by mass
  (trade name: F-475, manufactured by Dainippon Ink And Chemicals, Incorporated)
- Photopolymerization initiator 0.586 part by mass
  (trade name: TAZ-107, that is, a trihalomethyl triazine-based photopolymerization initiator, manufactured by Midori Kagaku Co., Ltd.)

2. Preparation of a silicon wafer substrate with an undercoat layer

A 6-inch silicon wafer was treated by heating in an oven at 200˚C for 30 minutes. Then, the resist solution was applied onto this silicon wafer so as for its film thickness after drying to reach 2 μm, and then dried by heating in an oven at 220˚C for 1 hour to form an undercoat layer to give a silicon wafer substrate with an undercoat layer.

3. Preparation of Colored Photopolymerizable Composition B-1

Components in Composition B-1 below were mixed and dissolved to prepare colorant (dye)-containing Colored Photopolymerizable Composition B-1.

<Composition B-1>

- Cyclohexanone 80 parts by mass
- Colorant C.I. Acid Blue 108 7.5 parts by mass
- Colorant C.I. Solvent Yellow 162 2.5 parts by mass
- Radical-polymerizable monomer (polymerizable compound) 7.0 parts by mass
  (a mixture of pentaerythritol triacrylate and dipentaerythritol hexaacrylate in a ratio of 3:7)
- Specific Compound 1 (specific oxime compound) 2.5 parts by mass
- Glycerol propoxylate 0.5 part by mass
  (number-average molecular weight Mn: 1,500, molar extinction coefficient $\varepsilon = 0$)

4. Evaluation of storage stability of Colored Photopolymerizable Composition B-1 (coating solution)

Colored Photopolymerizable Composition B-1 was stored at room temperature for 1 month, and the degree of precipitation of foreign substances was evaluated visually under the following judgment criteria. The results are shown in Table 3 below.

- Judgment Criteria -

A: No precipitation was recognized.
B: Slight precipitation was recognized.
C: Precipitation was recognized.

5. Preparation and evaluation of a color filter with Colored Photopolymerizable Composition B-1

Colored Polymerizable Composition B-1 prepared in item 3. above was applied onto the undercoat layer of the silicon wafer substrate with an undercoat layer obtained in item 2. above to form a photocurable coating film thereon. Then, the coating film was heat-treated (pre-baked) on a hot plate at 100˚C for 120 seconds such that the coating film after drying became 0.9 μm in thickness.
Then, the coating film was exposed, via a 2-μm-square island pattern mask, to light with a wavelength of 365 nm at an exposure amount of 10 to 1600 mJ/cm$^2$ from an i-line stepper exposure device FPA-3000i5+ (manufactured by Canon Co., Ltd.).
Thereafter, the silicon wafer substrate on which the irradiated coating film had been formed was placed on a horizontal rotating table of a spin shower developer (trade name: DW-30, manufactured by Chemitronics Co., Ltd.) and subjected to paddle development at 23˚C for 60 seconds with CD-2000 (manufactured by Fuji Film Electronics Materials Co., Ltd) to form a colored pattern on the silicon wafer substrate.
The silicon wafer substrate having a colored pattern formed thereon was fixed to the horizontal rotating table by a vacuum chuck system, and the silicon wafer substrate was rotated at 50 rpm with a rotating device and simultaneously rinsed with a shower of purified water supplied via a spraying nozzle downwards to the rotating center of the substrate and then spray-dried.
A color filter having a colored pattern formed on a substrate was obtained in this manner.

<Exposure sensitivity and pattern size>

The minimum light exposure at which the thickness of the coating film in the region exposed to light in the exposure step was 95% or more relative to the thickness (=100%) of the coating film before exposure was evaluated as exposure sensitivity. A smaller value of exposure sensitivity is indicative of higher sensitivity.
The size of the colored pattern was measured with a length measuring SEM (trade name: S-9260A, manufactured by Hitachi High-Technologies Corp.). A pattern size nearer 2 μm is indicative of higher sensitivity with sufficient curability. The results are shown in Table 3 below.

<Developability, coloring with time under heating, substrate adhesiveness, pattern sectional shape, and post-heating pattern sectional shape>

Developability, coloring with time under heating, substrate adhesiveness, pattern sectional shape, and post-heating pattern sectional shape were evaluated by the evaluation method and evaluation criteria shown in Example 2-1. The results are shown in Table 3 below.

Examples 3-2 to 3-17, Comparative Examples 3-1 to 3-3

Colored Photopolymerizable Compositions B-2 to B-17 and B'-1 to B'-3 were prepared in the same manner as in Example 3-1 except that the amounts of the respective compounds shown in Table 3 below were used in place of 2.5 parts by mass of Specific Compound 1 (specific oxime compound) in Composition B-1 used in preparing Colored Photopolymerizable Composition B-1 in Example 3-1, and the types and the amounts of the sensitizers and co-sensitizers shown in Table 3 below were used in Examples 3-10 to 3-17 respectively, in order to obtain color filters. Then, the same evaluation as in Example 3-1 was carried out. The results are shown in Table 3.

Table 3

| | Composition | Compound | | | Sensitizer | | Co-sensitizer | | Storage stability | Exposure sensitivity ($mJ/cm^2$) | Pattern size (μm) | Developability | Coloring change with time under forced heating | Substrate adhesiveness | Pattern sectional shape | Post-heating pattern sectional shape |
|---|---|---|---|---|---|---|---|---|---|---|---|---|---|---|---|---|
| | | specific | Comparative | content | type | content | type | content | | | | | | | | |
| Example 3-1 | B-1 | 1 | - | 2.5 | - | - | - | - | A | 1000 | 1.96 | A | A | A | rectangular | rectangular |
| Example 3-2 | B-2 | 2 | - | 2.5 | - | - | - | - | A | 900 | 1.97 | A | A | A | rectangular | rectangular |
| Example 3-3 | B-3 | 3 | - | 2.5 | - | - | - | - | A | 900 | 1.97 | A | A | A | rectangular | rectangular |
| Example 3-4 | B-4 | 4 | - | 2.5 | - | - | - | - | A | 900 | 1.97 | A | A | A | rectangular | rectangular |
| Example 3-5 | B-5 | 5 | - | 2.5 | - | - | - | - | A | 900 | 1.97 | A | A | A | rectangular | rectangular |
| Example 3-6 | B-6 | 6 | - | 2.5 | - | - | - | - | A | 900 | 1.97 | A | A | A | rectangular | rectangular |
| Example 3-7 | B-7 | 7 | - | 2.5 | - | - | - | - | A | 1200 | 1.94 | A | A | A | rectangular | rectangular |
| Example 3-8 | B-8 | 8 | - | 2.5 | - | - | - | - | A | 900 | 1.97 | A | A | A | rectangular | rectangular |
| Example 3-9 | B-9 | 9 | - | 2.5 | - | - | - | - | A | 900 | 1.97 | A | A | A | rectangular | rectangular |
| Example 3-10 | B-10 | 2 | - | 2.0 | A1 | 0.5 | - | - | A | 850 | 1.97 | A | A | A | rectangular | rectangular |
| Example 3-11 | B-11 | 2 | - | 2.0 | A2 | 0.5 | - | - | A | 850 | 1.97 | A | A | A | rectangular | rectangular |
| Example 3-12 | B-12 | 2 | - | 2.0 | A3 | 0.5 | - | - | A | 800 | 1.98 | A | A | A | rectangular | rectangular |
| Example 3-13 | B-13 | 2 | - | 2.0 | - | - | F1 | 0.5 | A | 850 | 1.98 | A | A | A | rectangular | rectangular |
| Example 3-14 | B-14 | 2 | - | 1.5 | A3 | 0.5 | F1 | 0.5 | A | 800 | 1.98 | A | A | A | rectangular | rectangular |
| Example 3-15 | B-15 | 2 | - | 1.5 | A3 | 0.5 | F2 | 0.5 | A | 800 | 1.98 | A | A | A | rectangular | rectangular |

| | | | | | | | | | | | | | | | | |
|---|---|---|---|---|---|---|---|---|---|---|---|---|---|---|---|---|
| Example 3-16 | B-16 | 2 | - | 1.5 | A3 | 0.5 | F3 | 0.5 | A | 750 | 1.98 | A | A | A | rectangular | rectangular |
| Example 3-17 | B-17 | 2 | - | 1.5 | A3 | 0.5 | F3<br>LD-5 | 0.25<br>0.25 | A | 750 | 1.98 | A | A | A | rectangular | rectangular |
| Comparative example 3-1 | B'-1 | - | 1 | 2.5 | - | - | - | - | A | 1400 | 1.92 | A | B | B | inverse taper | forward taper |
| Comparative example 3-2 | B'-2 | - | 2 | 2.5 | - | - | - | - | A | 1300 | 1.92 | A | C | B | inverse taper | forward taper |
| Comparative example 3-3 | B'-3 | - | 3 | 2.5 | - | - | - | - | A | 1300 | 1.92 | A | B | B | inverse taper | forward taper |

In Table 3 above, numerical 1 to 9 in column "specific" in column "Compound" refer to Specific Compounds 1 to 9 respectively, and numerical 1 to 3 in column "comparative" refer to Comparative Compounds 1 to 3 respectively. The "content" is expressed in parts by mass.
In Table 3 above, the sensitizers A1 to A3 and the co-sensitizers F1 to F3 and LD-5 refer respectively to the compounds mentioned above.

Example 3-18

Components in Composition C-1 below were mixed and dissolved to prepare colorant (pigment)-containing Colored Photopolymerizable Composition C-1.

<Composition C-1>

- Ethyl 3-ethoxypropionate (solvent) 17.9 parts by mass
- Dispersion of Colorant C.I. Pigment Red 254 26.7 parts by mass
  (solid content: 15% by mass, the solid content of the pigment: 60%)
- Dispersion of Colorant C.I. Pigment Yellow 139 17.8 parts by mass
  (solid content: 15% by mass, the solid content of the pigment: 60%)
- Radical-polymerizable monomer (polymerizable compound) 3.5 parts by mass
  (a mixture of pentaerythritol triacrylate and dipentaerythritol hexaacrylate in a ratio of 3:7)
- Specific Compound 1 (specific oxime compound) 0.5 part by mass
- Benzyl methacrylate/methacrylic acid copolymer 2.0 parts by mass
  (molar ratio = 70/30)

Examples 3-19 to 3-34, Comparative Examples 3-4 to 3-6

Colored Photopolymerizable Compositions C-2 to C-17 and C'-1 to C'-3 were prepared in the same manner as in Example 3-18 except that the amounts of the respective compounds shown in Table 4 below were used in place of 0.5 part by mass of Specific Compound 1 (specific oxime compound) in Composition C-1 used in preparing Colored Photopolymerizable Composition C-1 in Example 3-18, and the types and the amounts of the sensitizers and co-sensitizers shown in Table 4 below were used in Examples 3-27 to 3-34 respectively.
The resulting colored photopolymerizable compositions were evaluated in the same manner as in Example 3-1. The results are shown in Table 4.
Table 4

| | Composition | Compound | | | Sensitizer | | Co-sensitizer | | Storage stability | Exposure sensitivity ($mJ/cm^2$) | Pattern size (μm) | Developability | Coloring change with time under forced heating | Substrate adhesiveness | Pattern sectional shape | Post-heating pattern sectional shape |
|---|---|---|---|---|---|---|---|---|---|---|---|---|---|---|---|---|
| | | specific | comparative | content | type | content | type | content | | | | | | | | |
| Example 3-18 | C-1 | 1 | - | 0.5 | - | - | - | - | A | 800 | 1.97 | A | A | A | rectangular | rectangular |
| Example 3-19 | C-2 | 2 | - | 0.5 | - | - | - | - | A | 700 | 1.98 | A | A | A | rectangular | rectangular |
| Example 3-20 | C-3 | 3 | - | 0.5 | - | - | - | - | A | 700 | 1.98 | A | A | A | rectangular | rectangular |
| Example 3-21 | C-4 | 4 | - | 0.5 | - | - | - | - | A | 700 | 1.98 | A | A | A | rectangular | rectangular |
| Example 3-22 | C-5 | 5 | - | 0.5 | - | - | - | - | A | 700 | 1.98 | A | A | A | rectangular | rectangular |
| Example 3-23 | C-6 | 6 | - | 0.5 | - | - | - | - | A | 700 | 1.98 | A | A | A | rectangular | rectangular |
| Example 3-24 | C-7 | 7 | - | 0.5 | - | - | - | - | A | 1000 | 1.96 | A | A | A | rectangular | rectangular |
| Example 3-25 | C-8 | 8 | - | 0.5 | - | - | - | - | A | 700 | 1.98 | A | A | A | rectangular | rectangular |
| Example 3-26 | C-9 | 9 | - | 0.5 | - | - | - | - | A | 700 | 1.98 | A | A | A | rectangular | rectangular |
| Example 3-27 | C-10 | 2 | - | 0.4 | A1 | 0.1 | - | - | A | 650 | 1.98 | A | A | A | rectangular | rectangular |
| Example 3-28 | C-11 | 2 | - | 0.4 | A2 | 0.1 | - | - | A | 650 | 1.98 | A | A | A | rectangular | rectangular |
| Example 3-29 | C-12 | 2 | - | 0.4 | A3 | 0.1 | - | - | A | 650 | 1.98 | A | A | A | rectangular | rectangular |
| Example 3-30 | C-13 | 2 | - | 0.4 | - | - | F1 | 0.1 | A | 650 | 1.98 | A | A | A | rectangular | rectangular |
| Example 3-31 | C-14 | 2 | - | 0.3 | A3 | 0.1 | F1 | 0.1 | A | 650 | 1.98 | A | A | A | rectangular | rectangular |
| Example | C-15 | 2 | - | 0.3 | A3 | 0.1 | F2 | 0.1 | A | 650 | 1.98 | A | A | A | rectan | rectang |

| | | | | | | | | | | | | | | | | |
|---|---|---|---|---|---|---|---|---|---|---|---|---|---|---|---|---|
| 3-32 | | | | | | | | | | | | | | | gular | ular |
| Example 3-33 | C-16 | 2 | - | 0.3 | A3 | 0.1 | F3 | 0.1 | A | 600 | 1.98 | A | A | A | rectangular | rectangular |
| Example 3-34 | C-17 | 2 | - | 0.3 | A3 | 0.5 | F3<br>LD-5 | 0.05<br>0.05 | A | 600 | 1.98 | A | A | A | rectangular | rectangular |
| Comparative example 3-4 | C'-1 | - | 1 | 0.5 | - | - | - | - | A | 1200 | 1.92 | A | B | B | inverse taper | forward taper |
| Comparative example 3-5 | C'-2 | - | 2 | 0.5 | - | - | - | - | A | 1100 | 1.92 | A | C | B | inverse taper | forward taper |
| Comparative example 3-6 | C'-3 | - | 3 | 0.5 | - | - | - | - | A | 1200 | 1.92 | A | A | B | inverse taper | forward taper |

In Table 4 above, numerical 1 to 9 in column "specific" in column "Compound" refer to Specific Compounds 1 to 9 respectively, and numerical 1 to 3 in column "comparative" refer to Comparative Compounds 1 to 3 respectively. The "content" is expressed in parts by mass.
In Table 4 above, the sensitizers A1 to A3 and the co-sensitizers F1 to F3 and LD-5 refer respectively to the compounds mentioned above.

Example 3-35

Components in Composition D-1 below were mixed and dissolved to prepare colorant (pigment)-containing Colored Photopolymerizable Composition D-1.

<Composition D-1>

- Ethyl 3-ethoxypropionate (solvent) 17.9 parts by mass
- Dispersion of Colorant C.I. Pigment Red 254 33.34 parts by mass
  (solid content: 15% by mass, the solid content of the pigment: 60%)
- Dispersion of Colorant C.I. Pigment Yellow 139 22.23 parts by mass
  (solid content: 15% by mass, the solid content of the pigment: 60%)
- Radical-polymerizable monomer (polymerizable compound) 2.5 parts by mass
  (a mixture of pentaerythritol triacrylate and dipentaerythritol hexaacrylate in a ratio of 3:7)
- Specific Compound 1 (specific oxime compound) 0.5 part by mass
- Benzyl methacrylate/methacrylic acid copolymer 2.0 parts by mass
  (molar ratio = 70/30)

Examples 3-36 to 3-51, Comparative Examples 3-7 to 3-9

Colored Photopolymerizable Compositions D-2 to D-17 and D'-1 to D'-3 were prepared in the same manner as in Example 3-35 except that 0.5 part by mass of the respective compounds shown in Table 5 below were used respectively in place of 0.5 part by mass of Specific Compound 1 (specific oxime compound) in Composition D-1 used in preparing Colored Photopolymerizable Composition D-1 in Example 3-35.
The resulting colored photopolymerizable compositions were evaluated in the same manner as in Example 3-1. The results are shown in Table 5.
Table 5

| | Composition | Compound | | | Sensitizer | | Co-sensitizer | | Storage stability | Exposure sensitivity ($mJ/cm^2$) | Pattern size (μm) | Developability | Coloring change with time under forced heating | Substrate adhesiveness | Pattern sectional shape | Post-heating pattern sectional shape |
|---|---|---|---|---|---|---|---|---|---|---|---|---|---|---|---|---|
| | | Specific | comparative | content | type | content | type | content | | | | | | | | |
| Example 3-35 | D-1 | 1 | - | 0.5 | - | - | - | - | A | 1500 | 1.96 | A | A | A | rectangular | rectangular |
| Example 3-36 | D-2 | 2 | - | 0.5 | - | - | - | - | A | 1400 | 1.96 | A | A | A | rectangular | rectangular |
| Example 3-37 | D-3 | 3 | - | 0.5 | - | - | - | - | A | 1400 | 1.96 | A | A | A | rectangular | rectangular |
| Example 3-38 | D-4 | 4 | - | 0.5 | - | - | - | - | A | 1400 | 1.96 | A | A | A | rectangular | rectangular |
| Example 3-39 | D-5 | 5 | - | 0.5 | - | - | - | - | A | 1400 | 1.96 | A | A | A | rectangular | rectangular |
| Example 3-40 | D-6 | 6 | - | 0.5 | - | - | - | - | A | 1400 | 1.96 | A | A | A | rectangular | rectangular |
| Example 3-41 | D-7 | 7 | - | 0.5 | - | - | - | - | A | 1600 | 1.94 | A | A | A | rectangular | rectangular |
| Example 3-42 | D-8 | 8 | - | 0.5 | - | - | - | - | A | 1400 | 1.96 | A | A | A | rectangular | rectangular |
| Example 3-43 | D-9 | 9 | - | 0.5 | - | - | - | - | A | 1400 | 1.96 | A | A | A | rectangular | rectangular |
| Example 3-44 | D-10 | 2 | - | 0.4 | A1 | 0.1 | - | - | A | 1300 | 1.96 | A | A | A | rectangular | rectangular |
| Example 3-45 | D-11 | 2 | - | 0.4 | A2 | 0.1 | - | - | A | 1300 | 1.96 | A | A | A | rectangular | rectangular |
| Example 3-46 | D-12 | 2 | - | 0.4 | A3 | 0.1 | - | - | A | 1300 | 1.96 | A | A | A | rectangular | rectangular |
| Example 3-47 | D-13 | 2 | - | 0.4 | - | - | F1 | 0.1 | A | 1300 | 1.96 | A | A | A | rectangular | rectangular |
| Example 3-48 | D-14 | 2 | - | 0.3 | A3 | 0.1 | F1 | 0.1 | A | 1300 | 1.96 | A | A | A | rectangular | rectangular |

| | | | | | | | | | | | | | | | | |
|---|---|---|---|---|---|---|---|---|---|---|---|---|---|---|---|---|
| Example 3-49 | D-15 | 2 | - | 0.3 | A3 | 0.1 | F2 | 0.1 | A | 1300 | 1.96 | A | A | A | rectangular | rectangular |
| Example 3-50 | D-16 | 2 | - | 0.3 | A3 | 0.1 | F3 | 0.1 | A | 1200 | 1.96 | A | A | A | rectangular | rectangular |
| Example 3-51 | D-17 | 2 | - | 0.3 | A3 | 0.1 | F3<br>LD-5 | 0.05<br>0.05 | A | 1200 | 1.96 | A | A | A | rectangular | rectangular |
| Comparative example 3-7 | D'-1 | - | 1 | 0.5 | - | - | - | - | A | 3000 | 1.92 | A | B | B | inverse taper | forward taper |
| Comparative example 3-8 | D'-2 | - | 2 | 0.5 | - | - | - | - | A | 2800 | 1.92 | A | C | B | inverse taper | forward taper |
| Comparative example 3-9 | D'-3 | - | 3 | 0.5 | - | - | - | - | A | 2800 | 1.92 | A | A | B | inverse taper | forward taper |

In Table 5 above, numerical 1 to 9 in column "specific" in column "Compound" refer to Specific Compounds 1 to 9 respectively, and numerical 1 to 3 in column "comparative" refer to Comparative Compounds 1 to 3 respectively. The "content" is expressed in parts by mass.
In Table 5 below, the sensitizers A1 to A3 and the co-sensitizers F1 to F3 and LD-5 refer respectively to the compounds mentioned above.

From the results in Tables 3 to 5, it may be seen that the colored photopolymerizable compositions in the Examples containing the specific oxime compounds (Compounds 1 to 9) are excellent in storage stability (temporal stability). It may also be seen that these colored photopolymerizable compositions have high exposure sensitivity, are excellent in developability upon formation of colored patterns on color filters, are free of coloring in the resulting colored patterns with time under heating, and are excellent in both substrate adhesiveness and pattern sectional shape.
As is also evident from Table 5, it may be seen that even if the content of the pigment is high, the compositions have excellent exposure sensitivity.

Examples 4-1 to 4-38, Comparative Examples 4-1 to 4-12 Preparation of a black photopolymerizable composition

<Preparation of Black Carbon Dispersion A>

Components in Composition 1 below were dispersed with a twin roll to yield a dispersion having high viscosity. The viscosity of this dispersion was 70,000 mPa·s.
Thereafter, components in Composition 2 below were added to this dispersion and then stirred with a homogenizer under the condition of 3,000 rpm for 3 hours. The resulting mixed solution was finely dispersed for 4 hours with a dispersing machine (DISPER MAT, manufactured by GETZMANN) using 0.3 mm zirconia beads to prepare Carbon Black Dispersion A (referred to hereinafter as CB Dispersion A). The viscosity of this mixed solution was 37 mPa·s.

Composition 1

- Carbon black (Pigment Black 7) having an average primary particle size of 15 nm 23 parts
- 45% Solution of benzyl methacrylate/methacrylic acid copolymer in propylene glycol monomethyl ether acetate 22 parts
  (BzMA/MAA= 70/30, Mw: 30,000)
- Solsperse 5000 (manufactured by Zeneca) 1.2 parts

Composition 2

- 45% Solution of benzyl methacrylate/methacrylic acid copolymer in propylene glycol monomethyl ether acetate 22 parts
  (BzMA/MAA= 70/30, Mw: 30,000)
- Propylene glycol monomethyl ether acetate 200 parts

<Preparation of Titan Black Dispersion A>

Components in Composition 3 below were dispersed with a twin roll to yield a dispersion having high viscosity. The viscosity of this dispersion was 40,000 mPa·s.
Before this dispersion treatment for yielding a highly viscous dispersion, the components may be kneaded with a kneader for 30 minutes.

Composition 3

- Titan Black 13M-C having an average primary particle size of 75 nm 39 parts
  (Pigment Black 35 manufactured by Mitsubishi Materials Corporation)
- Solution of benzyl (meth)acrylate/(meth)acrylic acid copolymer in propylene glycol monomethyl ether acetate 8 parts

(BzMA/MAA= 70/30, Mw: 30,000, solid content: 40% by mass)
- Solsperse 5000 (manufactured by Zeneca) 1 part

Components in Composition 4 below were added to the resulting dispersion and then stirred with a homogenizer under the condition of 3,000 rpm for 3 hours. The resulting mixed solution was finely dispersed for 4 hours with a dispersing machine (DISPER MAT, manufactured by GETZMANN) using 0.3 mm zirconia beads to prepare Titan Black Dispersion A (referred to hereinafter as TB Dispersion A). The viscosity of this mixed solution was 7.0 mPa·s.

Composition 4

- Solution of benzyl (meth)acrylate/(meth)acrylic acid copolymer in propylene glycol monomethyl ether acetate 8 parts
  (BzMA/MAA = 70/30, Mw 30,000, solid content 40% by mass)
- Propylene glycol monomethyl ether acetate 200 parts

<Preparation of Black Copolymerizable Compositions E-1 to E-38 and E'-1 to E'-12>

Components in Composition E-a below were mixed by means of a stirrer to prepare Black Copolymerizable Compositions E-1 to E-18 and E'-1 to E'-6.

Composition E-a

- Methacrylatelacrylic acid copolymer (alkali-soluble resin) 1.6 parts by mass
- Dipentaerythritol hexaacrylate 2.3 parts by mass
- Ethoxylated pentaerythritol tetraacrylate 0.8 part by mass
- CB Dispersion A or TB Dispersion A obtained above 24 parts by mass
- Propylene glycol monomethyl ether acetate 10 parts by mass
- Ethyl-3-ethoxy propionate 8 parts by mass
- Compound shown in Table 6 below: specific oxime compound, comparative compound or LD-5 the amount shown in Table 6
- Co-sensitizer: the above-mentioned F3 not added or 0.1 part by mass

Components in Composition E-b below were mixed by means of a stirrer to prepare Black Copolymerizable Compositions E-19 to E-38 and E'-7 to E'-12.

Composition E-b

- Dipentaerythritol hexaacrylate 2.3 parts by mass
- CB Dispersion A or TB Dispersion A obtained above 24 parts by mass
- Propylene glycol monoethyl ether acetate 10 parts by mass
- Ethyl-3-ethoxy propionate 8 parts by mass
- Compound shown in Table 7 below: specific oxime compound, comparative compound or LD-5 the amount shown in Table 7
- Co-sensitizer: the above-mentioned F3 not added or 0.1 part by mass

Evaluation

Black Copolymerizable Compositions E-1 to E-38 and E'-1 to E'-12 obtained as described above were evaluated in the following manner. The results are shown collectively in Tables 6 and 7.

- Evaluation of exposure sensitivity -

The exposure sensitivity of each of Black Copolymerizable Compositions E-1 to E-38 and E'-1 to E'-12 obtained as described above was determined and evaluated by the following method.

Each of Black Photopolymerizable Compositions E-1 to E-38 and E'-1 to E'-12 was applied uniformly onto a silicon wafer substrate at a number of spin-coating revolutions regulated such that a coating film after drying on a hot plate at 120˚C for 120 seconds became 1.0 μm in thickness, thereby giving a 1.0 μm coating film.
Then, the coating film was exposed, via a pattern mask with 10-nm L&S (line and space), to light in the range of 100 to 5,100 mJ/cm$^2$ varying in 100 mJ/cm$^2$ increments from an i-line stepper FPA-3000iS+ (manufactured by Canon Co., Ltd.). After irradiation, the coating film was subjected to paddle development at 23˚C for 60 seconds with 0.3% aqueous solution of tetramethyl ammonium hydroxide (TMAH), then rinsed with a spin shower of purified water for 20 seconds and washed again with purified water. Thereafter, adhered water droplets were removed with a high-degree air, and the substrate was naturally dried, to give a black image pattern thereon.
Each colored image pattern thus obtained was evaluated with an optical microscope under the following criteria.

The minimum light exposure at which the thickness of the coating film in the region exposed to light in the exposure step was 95% or more relative to the thickness (=100%) of the coating film before exposure was evaluated as exposure sensitivity.
A smaller value of exposure sensitivity is indicative of higher sensitivity.

- Evaluation of storage stability (temporal stability) -

Black Photopolymerizable Compositions E-1 to E-38 and E'-1 to E'-12 obtained as described above were evaluated for their storage stability (temporal stability) by the following method.
That is, Black Photopolymerizable Compositions E-1 to E-38 and E'-1 to E'-12 were stored at room temperature for 1 month, and the degree of precipitation of foreign substances was evaluated visually under the following judgment criteria:

- Judgment Criteria -

A: No precipitation was recognized.
B: Slight precipitation was recognized.
C: Precipitation was recognized.

- Evaluation of developability -

The developability of each of Black Copolymerizable Compositions E-1 to E-38 and E'-1 to E'-12 obtained as described above was evaluated by the following method.
That is, it was observed whether residues had occurred in the region not irradiated with light (unexposed region) in the exposure step for evaluation of sensitivity, thereby evaluating developability. The evaluation criteria are as follows.

- Evaluation Criteria -

A: No residue was observed in the unexposed region.
B: Slight residues were observed in the unexposed region but at a practically unproblematic level.
C: Significant residues were confirmed in the unexposed region.

Table 6

| | Composition | Formulation | Dispersion | Compound | | | Co-sensitizer | | Exposure sensitivity (mJ/cm$^2$) | Storage stability | Developability |
|---|---|---|---|---|---|---|---|---|---|---|---|
| | | | | specif ic | comparative | content | type | content | | | |
| Example 4-1 | E-1 | E-a | CB Dispersion A | 1 | - | 0.8 | - | - | 100 | A | A |
| Example 4-2 | E-2 | E-a | CB Dispersion A | 2 | - | 0.8 | - | - | 100 | A | A |
| Example 4-3 | E-3 | E-a | CB Dispersion A | 3 | - | 0.8 | - | - | 100 | A | A |
| Example 4-4 | E-4 | E-a | CB Dispersion A | 4 | - | 0.8 | - | - | 100 | A | A |
| Example 4-5 | E-5 | E-a | CB Dispersion A | 5 | - | 0.8 | - | - | 100 | A | A |
| Example 4-6 | E-6 | E-a | CB Dispersion A | 6 | - | 0.8 | - | - | 100 | A | A |
| Example 4-7 | E-7 | E-a | CB Dispersion A | 7 | - | 0.8 | - | - | 300 | A | A |
| Example 4-8 | E-8 | E-a | CB Dispersion A | 8 | - | 0.7 | - | - | 100 | A | A |
| Example 4-9 | E-9 | E-a | CB Dispersion A | 9 | - | 0.7 | - | - | 100 | A | A |
| Example 4-10 | E-10 | E-a | CB Dispersion A | 1 | - | 0.1 | F3 | 0.1 | 80 | A | A |
| Example 4-11 | E-11 | E-a | TB Dispersion A | 1 | - | 0.8 | - | - | 200 | A | A |
| Example 4-12 | E-12 | E-a | TB Dispersion A | 2 | - | 0.8 | - | - | 200 | A | A |
| Example 4-13 | E-13 | E-a | TB Dispersion A | 3 | - | 0.8 | - | - | 200 | A | A |
| Example 4-14 | E-14 | E-a | TB Dispersion A | 4 | - | 0.8 | - | - | 200 | A | A |

(continued)

| | Composition | Formulation | Dispersion | Compound | | | Co-sensitizer | | Exposure sensitivity (mJ/$cm^2$) | Storage stability | Developability |
|---|---|---|---|---|---|---|---|---|---|---|---|
| | | | | specif ic | comparative | content | type | content | | | |
| Example 4-15 | E-15 | E-a | TB Dispersion A | 7 | - | 0.8 | - | - | 400 | A | A |
| Example 4-16 | E-16 | E-a | TB Dispersion A | 8 | - | 0.8 | - | - | 200 | A | A |
| Example 4-17 | E-17 | E-a | TB Dispersion A | 9 | - | 0.8 | - | - | 200 | A | A |
| Example 4-18 | E-18 | E-a | TB Dispersion A | 1 | - | 0.7 | F3 | 0.1 | 150 | A | A |
| Comparative example 4-1 | E'-1 | E-a | CB Dispersion A | - | 1 | 0.8 | - | - | 600 | A | A |
| Comparative example 4-2 | E'-2 | E-a | CB Dispersion A | - | 2 | 0.8 | - | - | 500 | A | A |
| Comparative example 4-3 | E'-3 | E-a | CB Dispersion A | - | 3 | 0,8 | - | - | 500 | A | A |
| Comparative example 4-4 A | E'-4 | E-a | TB Dispersion | - | 1 | 0.8 | - | - | 800 | A | A |
| Comparative example 4-5 | E'-5 | E-a | TB Dispersion A | - | 2 | 0.8 | - | - | 700 | A | A |
| Comparative example 4-6 | E'-6 | E-a | TB Dispersion A | - | 3 | 0.8 | - | - | 700 | A | A |

Table 7

| | Composition | Formulation | Dispersion | Compound | | | Co-sensitizer | | Exposure sensitivity ($mJ/cm^2$) | Storage stability | Developability |
|---|---|---|---|---|---|---|---|---|---|---|---|
| | | | | specific | comparative | content | type | content | | | |
| Example 4-19 | E-19 | E-b | CB Dispersion A | 1 | - | 0.8 | - | - | 500 | A | A |
| Example 4-20 | E-20 | E-b | CB Dispersion A | 2 | - | 0.8 | - | - | 400 | A | A |
| Example 4-21 | E-21 | E-b | CB Dispersion A | 3 | - | 0.8 | - | - | 400 | A | A |
| Example 4-22 | E-22 | E-b | CB Dispersion A | 4 | - | 0.8 | - | - | 400 | A | A |
| Example 4-23 | E-23 | E-b | CB Dispersion A | 5 | - | 0.8 | - | - | 400 | A | A |
| Example 4-24 | E-24 | E-b | CB Dispersion A | 6 | - | 0.8 | - | - | 400 | A | A |
| Example 4-25 | E-25 | E-b | CB Dispersion A | 7 | - | 0.8 | - | - | 700 | A | A |
| Example 4-26 | E-26 | E-b | CB Dispersion A | 8 | - | 0.8 | - | - | 400 | A | A |
| Example 4-27 | E-27 | E-b | CB Dispersion A | 9 | - | 0.8 | - | - | 400 | A | A |
| Example 4-28 | E-28 | E-b | CB Dispersion A | 2 | - | 0.7 | F3 | 0.1 | 300 | A | A |
| Example 4-29 | E-29 | E-b | TB Dispersion A | 1 | - | 0.8 | - | - | 300 | A | A |
| Example 4-30 | E-30 | E-b | TB Dispersion A | 2 | - | 0.8 | - | - | 200 | A | A |
| Example 4-31 | E-31 | E-b | TB Dispersion A | 3 | - | 0.8 | - | - | 200 | A | A |
| Example 4-32 | E-32 | E-b | TB Dispersion A | 4 | - | 0.8 | - | - | 200 | A | A |

(continued)

| | Composition | Formulation | Dispersion | Compound | | | Co-sensitizer | | Exposure sensitivity ($mJ/cm^2$) | Storage stability | Developability |
|---|---|---|---|---|---|---|---|---|---|---|---|
| | | | | specific | comparative | content | type | content | | | |
| Example 4-33 | E-33 | E-b | TB Dispersion A | 5 | - | 0.8 | - | - | 200 | A | A |
| Example 4-34 | E-34 | E-b | TB Dispersion A | 6 | - | 0.8 | - | - | 200 | A | A |
| Example 4-35 | E-35 | E-b | TB Dispersion A | 7 | - | 0.8 | - | - | 400 | A | A |
| Example 4-36 | E-36 | E-b | TB Dispersion A | 8 | - | 0.8 | - | - | 200 | A | A |
| Example 4-37 | E-37 | E-b | TB Dispersion A | 9 | - | 0.8 | - | - | 200 | A | A |
| Example 4-38 | E-38 | E-b | TB Dispersion A | 2 | - | 0.7 | F3 | 0.1 | 150 | A | A |
| Comparative example 4-7 | E'-7 | E-b | CB Dispersion A | - | 1 | 0.8 | - | - | 900 | A | B |
| Comparative example 4-8 | E'-8 | E-b | CB Dispersion A | - | 2 | 0.8 | - | - | 800 | A | B |
| Comparative example 4-9 | E'-9 | E-b | CB Dispersion A | - | 3 | 0.8 | - | - | 800 | A | A |
| Comparative example 4-10 | E'-10 | E-b | TB Dispersion A | - | 1 | 0.8 | - | - | 600 | A | B |
| Comparative example 4-11 | E'-11 | E-b | TB Dispersion A | - | 2 | 0.8 | - | - | 600 | A | B |
| Comparative example 4-12 | E'-12 | E-b | TB Dispersion A | - | 3 | 0.8 | - | - | 600 | A | A |

In Tables 6 and 7 above, numerical 1 to 9 in column "specific" in column "Compound" refer to Specific Compounds 1 to 9 respectively, and numerical 1 to 3 in column "comparative" refer to Comparative Compounds 1 to 3 respectively. The "content" is expressed in parts by mass.

As is evident from Tables 6 and 7, it may be seen that the black copolymerizable compositions in the Examples containing the specific oxime compounds are excellent in storage stability (temporal stability). It may also be seen that because these black copolymerizable compositions are superior to those in the Comparative Examples in respect of exposure sensitivity and developability in the unexposed region, they are capable of forming a black pattern (colored pattern) with a lower amount of exposure light.

Example 5

<Preparation of Full-Color Color Filters>

The black image pattern prepared in Example 4-1 was used as a black matrix, and on the black matrix, a 1.6×1.6 μm green (G) colored pattern was prepared in the same manner as described in Example 2-1 by using Colored Photopolymerizable Composition A-1. Blue (B) and red (R) colored photopolymerizable compositions were prepared respectively in the same manner as in preparation of Colored Photopolymerizable Composition A-1 except that only the pigment (a mixture of C.I. Pigment Green 36 and C.I. Pigment Yellow 219 in a mass ratio of 30/70) was changed to a blue pigment (a mixture of C.I. Pigment Blue 15:6 and C.I. Pigment Violet 23 in a mass ratio of 30/70) and a red pigment (C.I. Pigment Red 254) respectively.

In the same manner as in formation of the green (G) pattern on the substrate by Green (G) Photopolymerizable Composition A-1, 1.6×1.6 μm blue (B) and red (R) patterns were formed successively on the substrate to prepare a color filter for solid-state imaging device.

When the resulting full-color color filter was evaluated for the black image pattern, the R, G and B colored patterns and the sectional shape and substrate adhesiveness of each pattern in the same manner as in Example 2-1, it was found that any patterns were rectangular, free of pattern defects and excellent in substrate adhesiveness.

Example 6

<Preparation of a solid-state imaging device>

When the full-color color filter obtained in Example 5 was integrated in a solid-state imaging device, the solid-state imaging device was confirmed to be excellent in color separation with high resolution.

Examples 7-1 to 7-15, Comparative Examples 7-1 to 7-4

<Preparation of a support>

After an aluminum plate of quality 1S having a thickness of 0.30 mm was surface-ground with No. 8 nylon brush and an aqueous suspension of 800 mesh pumice stone, the plate was sufficiently washed with water. Then, the aluminum plate was etched by immersing it in 10% sodium hydroxide at 70˚C for 60 seconds, washed with running water, neutralized and washed with 20% $HNO_3$ and washed with water. The aluminum plate was subjected to an electrolytic surface roughening treatment at the condition of electricity at the anode time of 300 coulomb/dm$^2$ in 1% aqueous nitric acid solution at VA=12.7 V using a sinusoidal alternating waved current. The surface roughness of the plate was determined and it was found to be 0 to 45 μm (as expressed in terms ofRa unit). Subsequently, the aluminum plate was desmutted by immersing it in 30% $H_2SO_4$ aqueous solution at 55˚C for 2 minutes, and then anodized at a current density of 5 A/dm$^2$ in 20% $H_2SO_4$ aqueous solution at 33˚C for 50 seconds with a cathode placed on the side of the grained surface of the aluminum plate. As a result, the thickness of the resulting anodized film was found to be 2.7 g/m$^2$.

In this manner, Support A-1 for planographic printing plate precursor was obtained.

<Formation of a photosensitive layer>

The resulting support was coated with a photosensitive layer coating solution with the following composition such that the amount of the coating solution after drying reached 1.4 g/m$^2$, followed by drying at 95˚C to form a photosensitive layer.

- Composition for Photosensitive Layer Coating Solution -

- Addition-polymerizable compound (M, N or O in Table 8) 0.80 part by mass
- Binder polymer (B1, B2 or B3 in Table 8) 0.90 part by mass
- Sensitizer (A1, A2 or A3 in Table 8) not added or 0.10 part by mass
- Compound shown in Table 8 below: specific oxime compound, comparative compound or LD-5 0.05 part by mass
- Co-sensitizer (the above-mentioned F2 or F3 in Table 8) not added or 0.25 part by mass
- Fluorochemical surfactant 0.02 part by mass
  (MEGAFAC F-177, manufactured by Dainippon Ink And Chemicals, Inc.)
- Heat polymerization inhibitor 0.03 part by mass
  (N-nitrosohydroxylamine aluminum salt)
- ε-Type copper phthalocyanine dispersion 0.2 part by mass
- Methyl ethyl ketone 16.0 parts by mass
- Propylene glycol monomethyl ether 16.0 parts by mass

<Formation of a protective layer>

The resulting photosensitive layer was coated with 3 % by mass aqueous solution of polyvinyl alcohol (saponification degree 98 mol%, polymerization degree 550) such that the mass of the coating solution after drying reached 2 g/m$^2$, followed by drying at 100˚C for 2 minutes to form a protective layer.
The planographic printing plate precursors in the Examples and the planographic printing plate precursors in the Comparative Examples were obtained in the manner described above.

<Plate making>

Each of the planographic printing plate precursors was subjected to the following exposure/development treatment.

Exposure

The planographic printing plate precursor was subjected to scanning exposure to 50 μJ/cm$^2$ light from a violet LD with a wavelength of 405 nm (trade name: Violet Boxer, manufactured by FFEI) under the conditions of 175 lines/inch at 4,000 dpi to form a solid image and a 1 to 99% halftone dot image (in 1% increments) thereon.

Development

The exposed plate was subjected to standard treatment in an automatic developer (trade name: LP-850P2, manufactured by Fuji Photo Film Co., Ltd.) charged with the following developing solution 1 and a finishing gum liquid (trade name: FP-2W, manufactured by Fuji Photo Film Co., Ltd.) The preheat conditions were as follows: the temperature of the planographic printing plate precursor was 100˚C, the developing solution temperature was 30˚C and the dipping period in the developing solution was about 15 seconds.
The developing solution 1 was composed of the following composition with pH 11.5 at 25˚C and an electric conductivity of 5 mS/cm.

- Composition of Developing Solution 1 -

- Potassium hydroxide 0.15 g
- Polyoxyethylene phenyl ether (n = 13) 5.0 g
- Chelest 400 (chelating agent) 0.1 g
- Water 94.75 g

Evaluation

The sensitivity and storage stability of the planographic printing plate precursor were evaluated by the following methods. The results are collectively shown in Table 8.

1. Evaluation of Sensitivity

The planographic printing plate precursor was exposed to light under the above conditions, and immediately developed under the above conditions to form an image, and 50% halftone dot area (%) was measured by a halftone dot area measuring instrument (Gretag Macbeth). The greater the number, the better the sensitivity.

2. Test of Printing Durability of Image Portion

The planographic printing plate precursor was used in printing with an ink (GEOS-G (N), manufactured by Dainippon Ink and Chemicals, Inc.) in a printing machine (R201, manufactured by Roland Company). Solid image portions on printed matters were observed, and printing durability was examined by determining the number of printed matters until blurring of images thereon was initiated. The greater the number of printed matters, the better the printing durability.

3. Evaluation of Storage Stability (amount of forced change with time)

Each of the planographic printing plate precursors was measured for its halftone dot area in the same manner as described above in evaluation of sensitivity except that together with an interleaving paper, the precursor was sealed with an aluminum craft paper and left in such a state at 60˚C for 4 days. Then, the difference between the halftone dot area of the precursor thus left at 60˚C for 4 days and the halftone dot area of the precursor not left at 60˚C for 4 days was determined to evaluate forced halftone change with time (Δ%). A smaller absolute value of this forced change with time is indicative of less influence of the forced change with time, that is, indicative of higher temporal stability.

Table 8

| | Support | Photosensitive layer | | | | | | | Sensitivity (%)(50% halftone dot area) | Amount (%) of forced change with time | Test of printing durability of image portion |
|---|---|---|---|---|---|---|---|---|---|---|---|
| | | Compound | | Addition-polymerizable compound | Binder polymer | Sensitizer | Co-sensitizer | Coating amount ($g/m^2$) | | | |
| | | specific | comparative | | | | | | | | |
| Example 7-1 | A-1 | 1 | - | M | B1 | - | - | 1.4 | 47 | 2 | 60,000 |
| Example 7-2 | A-1 | 2 | - | M | B1 | - | - | 1.4 | 48 | 2 | 70,000 |
| Example 7-3 | A-1 | 3 | - | M | B1 | - | - | 1.4 | 48 | 2 | 70,000 |
| Example 7-4 | A-1 | 4 | - | M | B1 | - | - | 1.4 | 48 | 2 | 70,000 |
| Example 7-5 | A-1 | 5 | - | M | B1 | - | - | 1.4 | 48 | 2 | 70,000 |
| Example 7-6 | A-1 | 6 | - | M | B1 | - | - | 1.4 | 48 | 2 | 70,000 |
| Example 7-7 | A-1 | 7 | - | M | B1 | - | - | 1.4 | 46 | 2 | 55,000 |
| Example 7-8 | A-1 | 8 | - | M | B1 | - | - | 1.4 | 47 | 2 | 60,000 |
| Example 7-9 | A-1 | 9 | - | M | B1 | - | - | 1.4 | 47 | 2 | 60,000 |
| Example 7-10 | A-1 | 2 | - | M | B1 | A1 | F2 | 1.4 | 47 | 2 | 70,000 |
| Example 7-11 | A-1 | 2 | - | N | B2 | A1 | F2 | 1.4 | 47 | 2 | 70,000 |
| Example 7-12 | A-1 | 2 | - | O | B3 | A1 | F2 | 1.4 | 47 | 2 | 100,000 |
| Example 7-13 | A-1 | 2 | - | O | B3 | A2 | F2 | 1.4 | 49 | 2 | 100,000 |
| Example 7-14 | A-1 | 2 | - | O | B3 | A3 | F3 | 1.4 | 50 | 2 | 100,000 |
| Example 7-15 | A-1 | 2 | - | O | B3 | A3 | F3 | 1.4 | 51 | 2 | 100,000 |
| Comparative example 7-1 | A-1 | - | 1 | M | B1 | - | - | 1.4 | 42 | 2 | 40,000 |

(continued)

| | Support | Photosensitive layer | | | | | | | Sensitivity (%)(50% halftone dot area) | Amount (%) of forced change with time | Test of printing durability of image portion |
|---|---|---|---|---|---|---|---|---|---|---|---|
| | | Compound | | Addition-polymerizable compound | Binder polymer | Sensitizer | Co-sensitizer | Coating amount ($g/m^2$) | | | |
| | | specific | comparative | | | | | | | | |
| Comparative example 7-2 | A-1 | - | 2 | M | B1 | - | - | 1.4 | 43 | 2 | 40,000 |
| Comparative example 7-3 | A-1 | - | 3 | M | B1 | - | - | 1.4 | 43 | 2 | 40,000 |
| Comparative example 7-4 | A-1 | - | LD-5 | M | B1 | A1 | F2 | 1.4 | 45 | 2 | 50,000 |

In Table 8, details of M, N and O in the column "Addition-polymerizable compound" and B1, B2 and B3 in the column "Binder polymer" are as follows. B3 is a mixture (mixing ratio 50/50 (molar ratio)) of a MDI/HMDI copolymer (molar ratio 80/20) and a copolymer of DMPA/PPG (m = 3)/TEG (molar ratio 52/22/26) each having the following structure.

M 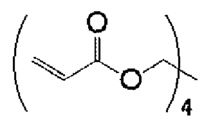

N 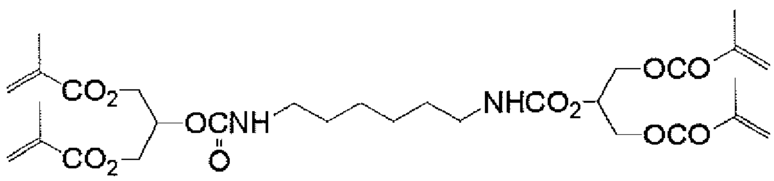

O 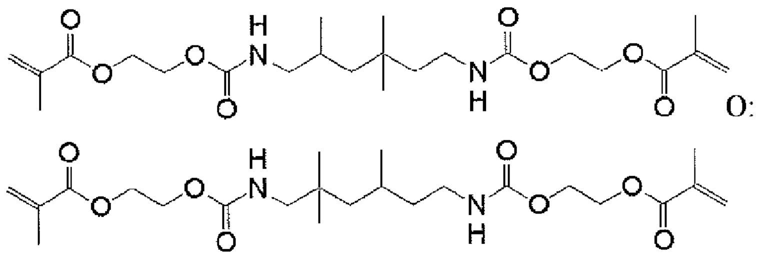

O: Mixture of the two isomers

B1 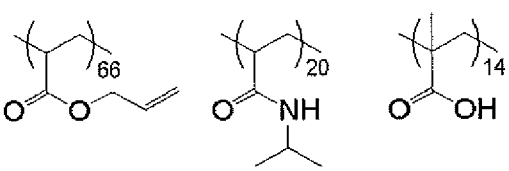

B2 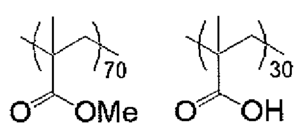

B3
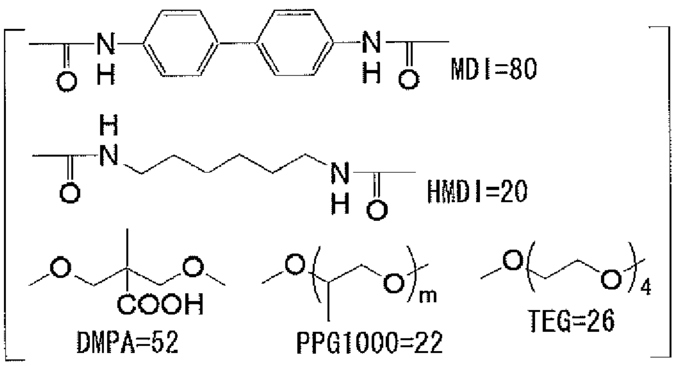


As is evident from Table 8, it may be seen that the planographic printing plate precursors in Examples 7-1 to 7-15, containing the specific oxime compound of the invention in the photosensitive layer, have high sensitivity and are excellent in temporal stability and printing durability.
On the other hand, the planographic printing plate precursors in Comparative Examples 7-1 to 7-4 were inferior to those of the Examples in all items of sensitivity, temporal stability, and printing durability.
A first object of the invention is to provide a photopolymerizable composition which is highly sensitive to light with a wavelength of 365 nm or 405 nm, is excellent in temporal stability, and may form a cured film capable of preventing film physical properties from deteriorating under heating with time, as well as an oxime compound used preferably in the photopolymerizable composition.
A second object of the invention is to provide a photopolymerizable composition for color filter, which is excellent in temporal stability, is cured with high sensitivity, has excellent pattern formability, forms a colored pattern excellent in adhesiveness to a support, and has a pattern shape excellent in post-heating after development.
A third object of the invention is to provide a color filter manufactured using the photopolymerizable composition for color filter, which has an excellent pattern shape and is excellent in adhesiveness to a support, a production method capable of forming the color filter with high productivity, as well as a solid-state imaging device including the color filter.
Still another object of the invention is to provide a planographic printing plate precursor using the photopolymerizable composition.
According to the invention, there may be provided a photopolymerizable composition which is highly sensitive to light with a wavelength of 365 nm or 405 nm, is excellent in temporal stability, and may form a cured film capable of preventing film physical properties from deteriorating under heating with time, as well as an oxime compound used preferably in the photopolymerizable composition. There may also be provided a photopolymerizable composition for color filter, which is excellent in temporal stability, is cured with high sensitivity, has excellent pattern formability, forms a colored pattern excellent in adhesiveness to a support, and has a pattern shape excellent in post-heating after development. Further, there may be provided a color filter manufactured using the photopolymerizable composition for color filter, which has an excellent pattern shape and is excellent in adhesiveness to a support, a production method capable of forming the color filter with high productivity, as well as a solid-state imaging device including the color filter. Moreover, there may be provided a planographic printing plate precursor using the photopolymerizable composition.

## Claims

1. A compound represented by the following Formula (1):

Formula (1)

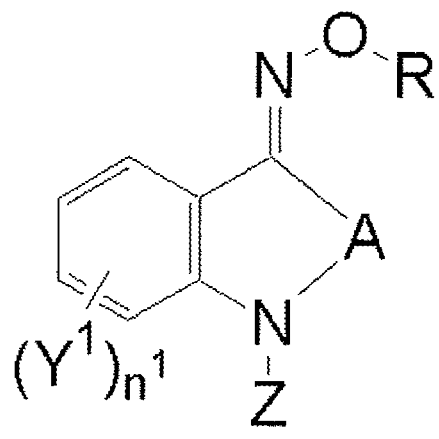

wherein R, $Y^1$ and Z each independently represent a monovalent substituent, $n^1$ represents an integer of 0 to 4, $Y^1$ and Z may be bound to each other to form a ring, and A represents a divalent linking group.

**2.** The compound of claim 1, which is represented by the following Formula (2):

Formula (2)

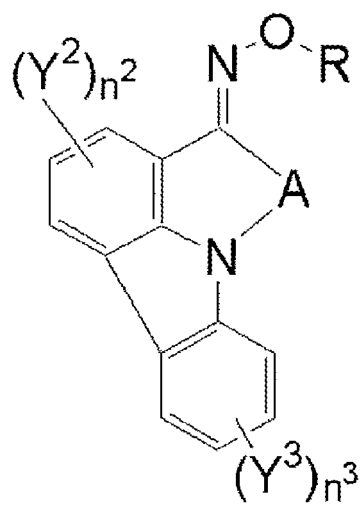

wherein R, $Y^2$ and $Y^3$ each independently represent a monovalent substituent, $n^2$ and $n^3$ independently represent an integer of 0 to 4, and A represents a divalent linking group.

**3.** The compound of claim 1 or 2, which is represented by the following Formula (3):

Formula (3)

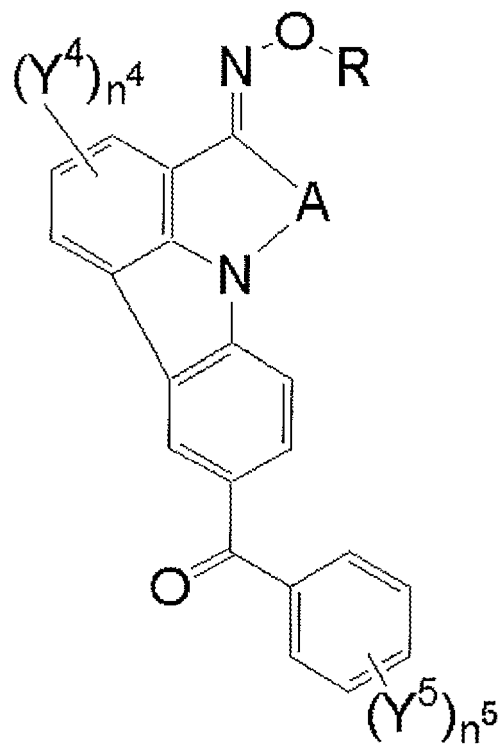

wherein R, $Y^4$ and $Y^5$ each independently represent a monovalent substituent, $n^4$ and $n^5$ independently represent an integer of 0 to 4, and A represents a divalent linking group.

4. A photopolymerizable composition comprising (A) the compound of any of claims 1 to 3 and (B) a polymerizable compound.

5. The photopolymerizable composition of claim 4, which further comprises (C) a colorant.

6. A photopolymerizable composition for color filter, which comprises the photopolymerizable composition of claim 5.

7. A color filter having a colored pattern using the photopolymerizable composition for color filter of claim 6 on a support.

8. A method for producing a color filter, comprising:

   applying the photopolymerizable composition for color filter of claim 6 on a support to form a photopolymerizable composition layer,
   exposing the photopolymerizable composition layer via a mask to light, and
   developing the photopolymerizable composition layer after exposure to form a colored pattern.

9. A solid-state imaging device including the color filter of claim 7.

10. A planographic printing plate precursor having a photosensitive layer containing the photopolymerizable composition of claim 4 on a support.

Europäisches Patentamt
European Patent Office
Office européen des brevets

## PARTIAL EUROPEAN SEARCH REPORT

which under Rule 63 of the European Patent Convention shall be considered, for the purposes of subsequent proceedings, as the European search report

Application Number
EP 09 15 4902

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GERALD N. EVENSON ETAL.: "O-carbamoyl oximes as potential analgesics" JOURNAL OF HETEROCYCLIC CHEMISTRY, vol. 17, March 1980 (1980-03), pages 351-352, XP002537904 USPROVO, UT * table I * ----- | 1 | INV. C07D471/16 C07D215/42 G03F7/031 |
| X | P. CATSOULACOS ET AL.: "On the synthesis of 3-amino-2,3-dihydro-1H-pyrido(3,2,1-kl)-phenothiazine 5,5-dioxide and...." JOURNAL OF HETEROCYCLIC CHEMISTRY., vol. 28, 1991, pages 1437-1440, XP002537905 USHETERO CORP., TAMPA, FL. * page 1437; figures I,II,III * ----- | 1 | |
| | -/-- | | |

TECHNICAL FIELDS SEARCHED (IPC)

C07D
G03F

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Berlin | 21 July 2009 | Rufet, Jacques |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 09 15 4902

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | NISHIJIMA K ET AL: "Synthesis and diuretic activity of 4,5-dihydro-6H-imidazo[4,5,1-ij]quinoline-6-one 6-oxime-O-sulfonic acid derivatives" EUROPEAN JOURNAL OF MEDICINAL CHEMISTRY, EDITIONS SCIENTIFIQUE ELSEVIER, PARIS, FR, vol. 33, no. 10, 1 October 1998 (1998-10-01), pages 763-774, XP004160038 ISSN: 0223-5234 * the whole document * ----- | 1 | |
| X | NISHIJIMA K ET AL: "Synthesis and diuretic..." EUROPEAN JOURNAL OF MEDICINAL CHEMISTRY, EDITIONS SCIENTIFIQUE ELSEVIER, PARIS, FR, vol. 33, 1 October 1998 (1998-10-01), pages 267-277, XP002537906 EDITIONS SCIENTIFIQUE ELSEVIER, PARIS, FR ISSN: 0223-5234 * the whole document * ----- | 1 | TECHNICAL FIELDS SEARCHED (IPC) |
| X | A. P. STANKEVECIUS ET AL.: "Synthesis of 3-aryl(hetaryl)-4-(2-cyanophenyl)-1,2,4-triazolin-5-ones" Chemistry of Heterocyclic Compounds, vol. 39, no. 11, 2003, pages 1525-1526, XP002537907 Plenum Publishing Corporation * page 1525 * ----- | 1 | |
| X | DATABASE BEILSTEIN BEILSTEIN INSTITUTE FOR ORGANIC CHEMISTRY, FRANKFURT-MAIN, DE; Database-Accession N. 11302845, 11302846 (BRN) XP002537909 * abstract * -/-- | 1 | |

3

EPO FORM 1503 03.82 (P04C10)

Europäisches Patentamt
European Patent Office
Office européen des brevets

# PARTIAL EUROPEAN SEARCH REPORT

Application Number
EP 09 15 4902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & ANGEWANDTE CHEMIE, INTERNATIONAL EDITION, vol. 46, no. 33, 2007, pages 6325-6328, ----- | | |
| X | DATABASE BEILSTEIN BEILSTEIN INSTITUTE FOR ORGANIC CHEMISTRY, FRANKFURT-MAIN, DE; Database-Accession N. 15433 (BRN) XP002537910 * abstract * & MONATSHEFTE FUR CHEMIE., 1913, page 1743, ATSPRINGER VERLAG. WIEN. ----- | 1 | |
| X | DATABASE BEILSTEIN BEILSTEIN INSTITUTE FOR ORGANIC CHEMISTRY, FRANKFURT-MAIN, DE; Database-Accession N. 48045, 368280 (BRN) XP002537911 * abstract * & JOURNAL OF THE CHEMICAL SOCIETY, 1952, pages 3046-3049, ----- | 1 | TECHNICAL FIELDS SEARCHED (IPC) |
| X | DATABASE BEILSTEIN BEILSTEIN INSTITUTE FOR ORGANIC CHEMISTRY, FRANKFURT-MAIN, DE; Database-Accession N. 1498152 (BRN) XP002537912 * abstract * & CHEMICAL AND PHARMACEUTICAL BULLETIN., vol. 17, no. 7, 1969, pages 1346-1350, JPPHARMACEUTICAL SOCIETY OF JAPAN. TOKYO. ----- | 1 | |
| X | DATABASE BEILSTEIN BEILSTEIN INSTITUTE FOR ORGANIC CHEMISTRY, FRANKFURT-MAIN, DE; Database-Accession N. 1475336 (BRN) XP002537913 * abstract * -/-- | 1 | |

3

EPO FORM 1503 03.82 (P04C10)

Europäisches Patentamt
European Patent Office
Office européen des brevets

# PARTIAL EUROPEAN SEARCH REPORT

Application Number
EP 09 15 4902

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| | & FARMACO.,<br>vol. 28, 1973, pages 157-158,<br>ITSOCIETA CHIMICA ITALIANA, PAVIA.<br>----- | | |
| X | DATABASE BEILSTEIN<br>BEILSTEIN INSTITUTE FOR ORGANIC CHEMISTRY, FRANKFURT-MAIN, DE; Database-Accession N. 8927120 (BRN)<br>XP002537914<br>* abstract *<br>& PHOSPHORUS, SULFUR AND SILICON AND THE RELATED ELEMENTS,<br>vol. 166, 2000, pages 303-314,<br>----- | 1 | |
| X | HIROAKI OHNO ET AL.: "Heck-type cyclization of oxime ethers:"<br>ANGEWANDTE CHEMIE. INTERNATIONAL EDITION., vol. 46, no. 33, 2007, pages 6325-6328, XP002537908<br>DEVCH VERLAG, WEINHEIM.<br>* table 1 *<br>----- | 1 | TECHNICAL FIELDS SEARCHED (IPC) |
| X | WO 00/33834 A (NEUROSEARCH AS [DK]; JENSEN BO SKAANING [DK]; JOERGENSEN TINO DYHRING) 15 June 2000 (2000-06-15)<br>* claim 1 *<br>----- | 1 | |
| X | EP 1 582 520 A (MOCHIDA PHARM CO LTD [JP]) 5 October 2005 (2005-10-05)<br>page 40, compounds 64 to 69, 71<br>----- | 1 | |
| X | US 3 813 396 A (YALE H ET AL)<br>28 May 1974 (1974-05-28)<br>* claim 1; examples 1-19; tables I,II *<br>----- | 1 | |
| X | US 3 838 135 A (MAGNIEN E ET AL)<br>24 September 1974 (1974-09-24)<br>* claim 1; table I *<br>-----<br>-/-- | 1 | |

3

EPO FORM 1503 03.82 (P04C10)

Europäisches Patentamt
European Patent Office
Office européen des brevets

# PARTIAL EUROPEAN SEARCH REPORT

Application Number
EP 09 15 4902

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | US 2006/128689 A1 (GOMTSYAN ARTHUR [US] ET AL) 15 June 2006 (2006-06-15)<br>* examples 1,26B,98B,102F,103 *<br>----- | 1 | |
| A,D | JP 2007 231000 A (FUJIFILM CORP)<br>13 September 2007 (2007-09-13)<br>* the whole document *<br>----- | 1-10 | |
| A | WO 2006/018405 A (CIBA SC HOLDING AG [CH]; TANABE JUNICHI [JP]; KUNIMOTO KAZUHIKO [JP];) 23 February 2006 (2006-02-23)<br>* claims 1-15 *<br>----- | 1-10 | |
| A | WO 2007/062963 A (CIBA SC HOLDING AG [CH]; MATSUMOTO AKIRA [JP]; TANABE JUNICHI [JP]; KU) 7 June 2007 (2007-06-07)<br>* claims 1-15 *<br>----- | 1-10 | TECHNICAL FIELDS SEARCHED (IPC) |
| A | JP 2005 070767 A (FUJI PHOTO FILM CO LTD)<br>17 March 2005 (2005-03-17)<br>* paragraph 3 - page 27, paragraph 1; claim 1 *<br>----- | 1-10 | |

3

EPO FORM 1503 03.82 (P04C10)

Europäisches Patentamt
European Patent Office
Office européen des brevets

**INCOMPLETE SEARCH SHEET C**

Application Number
EP 09 15 4902

Claim(s) searched completely:
2-10

Claim(s) searched incompletely:
1

Reason for the limitation of the search:

The initial phase of the search revealed a very large number of documents relevant to the issue of novelty. So many documents were retrieved that it is impossible to determine which parts of the claim 1 may be said to define subject-matter for which protection might legitimately be sought (Article 84 EPC). For these reasons, a meaningful search of the whole claimed subject matter of claim 1 could not be carried out (Rule 63 EPC). The extent of the search was consequently limited.

The search of claim 1 was restricted to the use of compounds according to present formula (1) wherein A is an C1-C4 alkylene group as photopolymerizable initiators

## ANNEX TO THE EUROPEAN SEARCH REPORT ON EUROPEAN PATENT APPLICATION NO. EP 09 15 4902

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2009

| Patent document cited in search report | Publication date | Patent family member(s) | Publication date |
|---|---|---|---|
| WO 0033834 A | 15-06-2000 | AT 285769 T | 15-01-2005 |
| | | AU 1648600 A | 26-06-2000 |
| | | DE 69922997 D1 | 03-02-2005 |
| | | DE 69922997 T2 | 15-12-2005 |
| | | EP 1135123 A1 | 26-09-2001 |
| | | JP 2002531498 T | 24-09-2002 |
| | | US 2002016354 A1 | 07-02-2002 |
| EP 1582520 A | 05-10-2005 | AU 2003277674 A1 | 03-06-2004 |
| | | CA 2505876 A1 | 27-05-2004 |
| | | WO 2004043959 A1 | 27-05-2004 |
| | | US 2006004028 A1 | 05-01-2006 |
| US 3813396 A | 28-05-1974 | NONE | |
| US 3838135 A | 24-09-1974 | NONE | |
| US 2006128689 A1 | 15-06-2006 | NONE | |
| JP 2007231000 A | 13-09-2007 | NONE | |
| WO 2006018405 A | 23-02-2006 | AT 381540 T | 15-01-2008 |
| | | CA 2575046 A1 | 23-02-2006 |
| | | CN 101014569 A | 08-08-2007 |
| | | DE 602005003960 T2 | 16-10-2008 |
| | | JP 2008509967 T | 03-04-2008 |
| | | KR 20070044062 A | 26-04-2007 |
| | | US 2008096115 A1 | 24-04-2008 |
| WO 2007062963 A | 07-06-2007 | CN 101321727 A | 10-12-2008 |
| | | EP 1957457 A1 | 20-08-2008 |
| | | JP 2009519904 T | 21-05-2009 |
| | | KR 20080083650 A | 18-09-2008 |
| | | US 2009087759 A1 | 02-04-2009 |
| JP 2005070767 A | 17-03-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4255513 A
- US 4590145 A
- JP 2000080068 A
- JP 2001233842 A
- JP 2006342166 A
- JP 2007231000 A
- JP 2005202252 A
- US 4575330 A
- DE 19700064
- EP 678534 A
- JP 51047334 B
- JP 57196231 A
- JP 59005240 A
- JP 59005241 A
- JP 2226149 A
- JP 1165613 A
- JP 54021726 B
- JP 48041708 B
- JP 51037193 A
- JP 2032293 B
- JP 2016765 B
- JP 58049860 B
- JP 56017654 B
- JP 62039417 B
- JP 62039418 B
- JP 63277653 A
- JP 63260909 A
- JP 1105238 A
- JP 48064183 A
- JP 49043191 B
- JP 52030490 B
- JP 46043946 B
- JP 1040337 B
- JP 1040336 B
- JP 2025493 A
- JP 61022048 A
- JP 6490403 A
- JP 6491102 A
- JP 1094301 A
- JP 6011614 A
- JP 2592207 B
- US 4808501 A
- US 5667920 A
- US 5059500 A
- JP 5333207 A
- JP 6035183 A
- JP 6051115 A
- JP 6194828 A
- JP 8211599 A
- JP 4249549 A
- JP 10123316 A
- JP 11302283 A
- JP 7286107 A
- JP 2001004823 A
- JP 8015522 A
- JP 8029771 A
- JP 8146215 A
- JP 11343437 A
- JP 8062416 A
- JP 2002014220 A
- JP 2002014221 A
- JP 2002014222 A
- JP 2002014223 A
- JP 8302224 A
- JP 8073758 A
- JP 8179120 A
- JP 8151531 A
- JP 4420189 B
- JP 51082102 A
- JP 52134692 A
- JP 59138205 A
- JP 60084305 A
- JP 62018537 A
- JP 6433104 A
- JP 53000702 A
- JP 55500806 B
- JP 5142772 A
- JP 56075643 A
- JP 48042965 B
- JP 55034414 B
- JP 6308727 A
- JP 59044615 A
- JP 54034327 B
- JP 58012577 B
- JP 54025957 B
- JP 54092723 A
- JP 59053836 A
- JP 59071048 A
- JP 2001115595 A
- JP 2001115598 A
- JP 2000187322 A
- JP 2002062698 A
- JP 2001242612 A
- JP 7012004 B
- JP 7120041 B
- JP 7120042 B
- JP 8012424 B
- JP 63287944 A

- JP 63287947 A
- JP 1271741 A
- JP 10116232 A
- JP 2002107918 A
- EP 993966 A
- EP 1204000 A
- JP 2001318463 A
- JP 2006078749 A
- JP 11171907 A
- JP 7120040 B
- JP 6250387 A
- JP 6191605 A
- JP 48018327 B
- JP 54063902 A
- JP 47005125 B
- US 3658662 A
- JP 46027481 B
- JP 52058602 A
- JP 52030503 A
- JP 56028893 A
- JP 7154983 A
- US 3055295 A
- JP 56013168 A
- JP 9080744 A
- JP 8507727 A
- US 3458311 A
- JP 55049729 A
- US 3458313 A
- JP 57007427 B
- US 3375171 A
- US 3615480 A
- JP 50026601 A
- JP 58054341 A
- JP 56039464 B
- JP 56042860 B
- JP 2002202616 A

**Non-patent literature cited in the description**

- *Journal of the Adhesion Society of Japan,* 1984, vol. 20 (7), 300-308
- **M. R. Sander et al.** *Journal of Polymer Society,* 1972, vol. 10, 3173
- *J. Appl. Chem.,* 1961, vol. 34, 2203-2207
- **C. Hansch ; A. Leo.** Substituent Constants for Correlation Analysis in Chemistry and Biology. J. Wile & Sons, 1979
- **A. K. Ghose et al.** *J. Comput. Chem.,* 1988, vol. 9, 80